# EUROPEAN PATENT APPLICATION

(11) **EP 4 467 541 A1**
(43) Date of publication of application: **27.11.2024**
(21) Application number: 23742827.1
(22) Date of filing: 13.01.2023
(51) Int. Cl.: C07D 471/04, C07D 217/24, C07D 217/26, C07D 217/16, C07D 217/18, A61K 31/498, A61K 35/00

(54) **SALT CRYSTAL FORM AND FREE BASE CRYSTAL FORM OF KINASE INHIBITOR**

(30) Priority: 21.01.2022 CN 202210070772
(71) Applicant: Cytosinlab Therapeutics Co., Ltd., Shanghai 201203 (CN)
(72) Inventor: WU, Haiping, Shanghai 201203 (CN); WANG, Meng, Shanghai 201203 (CN); MI, Yuan, Shanghai 201203 (CN); FU, Xingnian, Shanghai 201203 (CN)
(74) Representative: Dehns
(86) International application number: PCT/CN2023/072174
(87) International publication number: WO 2023/138512

(57) **Abstract**

A salt crystal form and a free base crystal form of a kinase inhibitor. Specifically, disclosed are a salt crystal form formed by a compound as shown in formula I and different acids and a free base crystal form thereof.

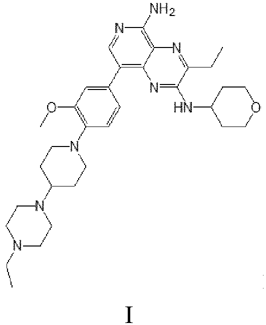

## Description

### Technical field

The present invention relates to the field of pharmaceutical chemistry, particularly to the salt crystal form and free base crystal form of compound and preparation methods therefor.

### Background

The occurrence and development of malignant tumors is a complex process. The traditional therapeutic drugs include the following main classes: alkylating agents, antimetabolites, natural products and antibiotics. The disadvantages of of these classes of drugs are poor efficancy and serious side effects. Small molecule kinase inhibitors are currently a field of interesting as potential cancer therapy. Protein kinases are catalysts that play a key role in almost every aspect of cell biology and biochemistry. These enzymes generate signaling modules that regulate cell cycle progression, proliferation, programmed cell death (apoptosis), cytoskeletal function, motility, differentiation, development, transcription, and translation. Protein kinases play several roles, and careful regulation of them is crucial, since abnormal activities of them may lead to cancer, cardiovascular disease, inflammation, and neurological disorders. Dysregulation, overexpression, and mutation of protein kinases are reasons for the pathogenesis of human diseases, which makes these enzymes attractive drug targets. Growth factor receptors with the activity of protein tyrosine kinases (PTKs) are called receptor tyrosine kinases. Protein receptor tyrosine kinases are a class of tightly regulated enzymes, and the abnormal activation of different members in the family is one of the markers of cancer. FLT3, along with receptors such as KIT, FMS, and platelet-derived growth factor receptor (PDGFR), belongs to the family of receptor tyrosine kinases, and plays important roles in the regulation of hematopoiesis.

FLT3 (FMS-like tyrosine kinase-3) is a specific cytokine receptor expressed on hematopoietic stem cells, which regulates the survival and growth of hematopoietic stem and progenitor cells, the maturation of dendritic cells, and the maintenance of regulatory T cell homeostasis. The high incidence of FLT3 mutation and poor prognosis suggest that it may be an important target for the treatment of AML.

In summary, there is an urgent need to develop new FLT3 kinase inhibitors in this field.

### Summary of invention

The purpose of the present invention is to provide salt crystal forms and free base crystal forms of the compound of formula I to meet the needs of drug research and development.

In the first aspect of the present invention, provided is a crystal form of a compound of formula I, 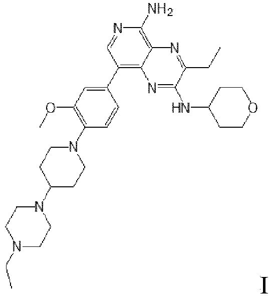 the crystal form is a free base crystal form Form T, and the free base crystal form Form T has the following X-ray powder diffraction characteristic peaks: 20.644 ° ± 0.2 °, 17.233 ° ± 0.2 °, 23.479 ° ± 0.2 °, 15.867 ° ± 0.2 °, 20.091 ° ± 0.2 °.

In another preferred embodiment, the free base crystal form Form T further optionally has one or more (such as 2, 3, 5, 8, or 10) X-ray powder diffraction characteristic peaks selected from the group consisting of: 15.505°±0.2°, 26.036°±0.2°, 19.457°±0.2°, 14.223°±0.2°, 25.043°±0.2°, 16.205°±0.2°, 23.143°±0.2°, 11.843°±0.2°, 9.973°±0.2°, 14.944°±0.2°, 18.921°±0.2°, 26.36°±0.2°, 8.858°±0.2°, 17.69°±0.2°, 22.069°±0.2°, 12.867°±0.2°, 18.428°±0.2°, 5.403°±0.2°, 26.675°±0.2°, 10.788°±0.2°, 22.412°±0.2°, 27.533°±0.2°, 24.211°±0.2°, 32.856°±0.2°, 12.351°±0.2°, 27.321°±0.2°, 40.066°±0.2°, 35.91°±0.2°, 29.079°±0.2°, 31.878°±0.2°, 36.087°±0.2°, 37.559°±0.2°, 21.598°±0.2°, 28.468°±0.2°, 11.168°±0.2°, 34.799°±0.2°, 40.437°±0.2°, 30.249°±0.2°, 33.551°±0.2°, 34.06°±0.2°, 7.753°±0.2°, 30.809°±0.2°, 35.509°±0.2°, 38.988°±0.2°, 29.487°±0.2°, 38.282°±0.2°.

In another preferred embodiment, the DSC spectrum of the free base crystal form Form T has three endothermic peaks at 90-95 ° C, 115-120 ° C, and 220-225 ° C.

In another preferred embodiment, the enthalpy value of the first endothermic peak in the DSC spectrum of the free base crystal form Form T is 50.223 J/g; the enthalpy value of the second endothermic peak in the DSC spectrum of the free base crystal form Form T is 51.492 J/g; and the enthalpy value of the third endothermic peak in the DSC spectrum of the crystal form is 80.538 J/g.

In the second aspect of the present invention, provided is a crystal form of a compound of formula I, 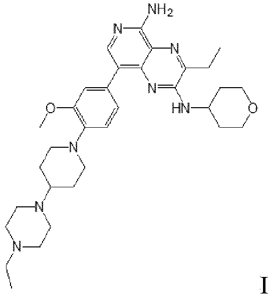 the crystal form is a free base crystal form Form B, and the free base crystal form Form B has the following X-ray powder diffraction characteristic peaks: 19.31 ° ± 0.2 °, 23.059 ° ± 0.2 °, 25.888 ° ± 0.2 °, 12.805 ° ± 0.2 °, and 17.188 ° ± 0.2 °.

In another preferred embodiment, the crystal form is a free base crystal form Form B, and the free base crystal form Form B further optionally has one or more (such as 2, 3, 5, 8, or 10) X-ray powder diffraction characteristic peaks selected from the group consisting of: 14.464°±0.2°, 20.6°±0.2°, 18.446°±0.2°, 6.337°±0.2°, 29.3°±0.2°, 16.155°±0.2°, 8.937°±0.2°, 9.29°±0.2°, 23.545°±0.2°, 32.361°±0.2°, 23.932°±0.2°, 10.227°±0.2°, 39.324°±0.2°, 15.042°±0.2°, 26.791°±0.2°, 30.37°±0.2°, 21.132°±0.2°, 24.448°±0.2°, 27.831°±0.2°, 36.599°±0.2°.

In another preferred embodiment, the DSC spectrum of the free base crystal form Form B has two endothermic peaks at 115-120 ° C and 225-230 ° C.

In another preferred embodiment, the enthalpy value of the first endothermic peak in the DSC spectrum of the free base crystal form Form B is 63.092 J/g; and the enthalpy value of the second endothermic peak in the DSC spectrum of the crystal form is 76.592 J/g.

In the third aspect of the present invention, provided is a crystal form of a compound of formula I: 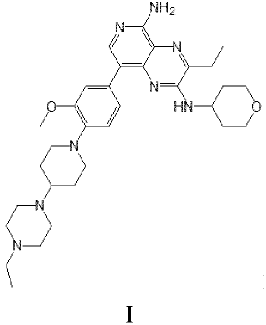 the crystal form is selected from the group consisting of:
salt crystal forms I-A, I-B, and I-C formed by the compound of formula I and p-benzenesulfonic acid;
salt crystal forms II-A, II-B, and II-C formed by the compound of formula I and fumaric acid;
salt crystal forms III-A, III-B, III-C formed by the compound of formula I and methanesulfonic acid;
a salt crystal form IV-A formed by the compound of formula I and phosphoric acid;
salt crystal forms V-A and V-B formed by the compounds of formula of formula I and maleic acid;
a salt crystal form VI-A formed by the compound of formula I and citric acid;
a salt crystal form VII-A formed by the compound of formula I and malic acid;
salt crystal forms VIII-A, VIII-B, and VIII-C formed by the compound of formula I and salicylic acid;
salt crystal forms IX-A, IX-B, IX-C, IX-D, IX-E, and IX-F formed by the compound of formula I and hydrochloric acid;
alternatively, the crystal form is a free base crystal form selected from the group consisting of: Form A, Form C, Form D, Form E, Form F, Form G, Form H, Form I, Form J, Form K, Form L, Form M, Form N, Form O, Form P, Form Q, Form R, and Form S.

In another preferred embodiment, the crystal form is a crystal form selected from the group consisting of:
the crystal form is a salt crystal form I-A, and the X-ray powder diffraction pattern of the salt crystal form I-A includes 2 or more (3, 4, or 5) X-ray powder diffraction characteristic peaks selected from the group consisting of: 22.558°±0.2°, 6.153°±0.2°, 6.365°±0.2°, 11.62°±0.2°, 23.392°±0.2°;
the crystal form is a salt crystal form I-B, and the X-ray powder diffraction pattern of the salt crystal form I-B includes 2 or more (3, 4, or 5) X-ray powder diffraction characteristic peaks selected from the group consisting of: 6.349°±0.2°, 18.611°±0.2°, 20.111°±0.2°, 19.354°±0.2°, 19.659°±0.2°;
the crystal form is a salt crystal form I-C, and the X-ray powder diffraction pattern of the salt crystal form I-C includes 2 or more (3, 4, or 5) X-ray powder diffraction characteristic peaks selected from the group consisting of: 6.156°±0.2°, 19.228°±0.2°, 6.348°±0.2°, 18.607°±0.2°, 24.203°±0.2°;
the crystal form is a salt crystal form II-A, and the X-ray powder diffraction pattern of the salt crystal form II-A includes 2 or more (3, 4, or 5) X-ray powder diffraction characteristic peaks selected from the group consisting of: 7.068°±0.2°, 22.629°±0.2°, 21.298°±0.2°, 18.108°±0.2°, 19.312°±0.2°;
the crystal form is a salt crystal form II-B, and the X-ray powder diffraction pattern of the salt crystal form II-B includes 2 or more (3, 4, or 5) X-ray powder diffraction characteristic peaks selected from the group consisting of: 22.639°±0.2°, 8.057°±0.2°, 12.303°±0.2°, 5.666°±0.2°, 18.977°±0.2°;
the crystal form is a salt crystal form II-C, and the X-ray powder diffraction pattern of the salt crystal form II-C includes 2 or more (3, 4, or 5) X-ray powder diffraction characteristic peaks selected from the group consisting of: 22.482°±0.2°, 9.03°±0.2°, 7.668°±0.2°, 18.149°±0.2°, 7.162°±0.2°;
the crystal form is a salt crystal form III-A, and the X-ray powder diffraction pattern of the salt crystal form III-A includes 2 or more (3, 4, or 5) X-ray powder diffraction characteristic peaks selected from the group consisting of: 9.199°±0.2°, 4.609°±0.2°, 23.106°±0.2°, 18.031°±0.2°, 13.798°±0.2°;
the crystal form is salt crystal form III-B, and the X-ray powder diffraction pattern of the salt crystal form III-B includes 2 or more (3, 4, or 5) X-ray powder diffraction characteristic peaks selected from the group consisting of: 16.693°±0.2°, 22.437°±0.2°, 22.187°±0.2°, 14.045°±0.2°, 8.936°±0.2°;
the crystal form is a salt crystal form III-C, and the X-ray powder diffraction pattern of the salt crystal form III-C includes 2 or more (3, 4, or 5) X-ray powder diffraction characteristic peaks selected from the group consisting of: 19.533°±0.2°, 24.465°±0.2°, 17.496°±0.2°, 9.057°±0.2°, 9.74°±0.2°;
the crystal form is a salt crystal form IV-A, and the X-ray powder diffraction pattern of the salt crystal form IV-A includes 2 or more (3, 4, or 5) X-ray powder diffraction characteristic peaks selected from the group consisting of: 21.594°±0.2°, 12.749°±0.2°, 23.759°±0.2°, 19.944°±0.2°, 27.501°±0.2°;
the crystal form is a salt crystal form V-A, and the X-ray powder diffraction pattern of the salt crystal form V-A includes 2 or more (3, 4, or 5) X-ray powder diffraction characteristic peaks selected from the group consisting of: 19.511°±0.2°, 8.538°±0.2°, 26.084°±0.2°, 12.999°±0.2°, 23.284°±0.2°;
the crystal form is a salt crystal form V-B, and the X-ray powder diffraction pattern of the salt crystal form V-B includes 2 or more (3, 4, or 5) X-ray powder diffraction characteristic peaks selected from the group consisting of: 22.48°±0.2°, 18.654°±0.2°, 17.31°±0.2°, 10.05°±0.2°, 19.992°±0.2°;
the crystal form is a salt crystal form VI-A, and the X-ray powder diffraction pattern of the salt crystal form VI-A includes 2 or more (3, 4, or 5) X-ray powder diffraction characteristic peaks selected from the group consisting of: 17.502°±0.2°, 8.712°±0.2°, 17.012°±0.2°, 7.854°±0.2°, 20.072°±0.2°;
the crystal form is a salt crystal form VII-A, and the X-ray powder diffraction pattern of the salt crystal form VII-A includes 2 or more (3, 4, or 5) X-ray powder diffraction characteristic peaks selected from the group consisting of: 22.102°±0.2°, 17.149°±0.2°, 14.246°±0.2°, 19.119°±0.2°, 22.613°±0.2°;
the crystal form is a salt crystal form VIII-A, and the X-ray powder diffraction pattern of the salt crystal form VIII-A includes 2 or more (3, 4, or 5) X-ray powder diffraction characteristic peaks selected from the group consisting of: 3.834°±0.2°, 4.282°±0.2°, 7.593°±0.2°, 21.198°±0.2°, 16.946°±0.2°;
the crystal form is a salt crystal form VIII-B, and the X-ray powder diffraction pattern of the salt crystal form VIII-B includes 2 or more (3, 4, or 5) X-ray powder diffraction characteristic peaks selected from the group consisting of: 23.438°±0.2°, 14.857°±0.2°, 15.021°±0.2°, 24.138°±0.2°, 20.337°±0.2°;
the crystal form is a salt crystal form VIII-C, and the X-ray powder diffraction pattern of the salt crystal form VIII-C includes 2 or more (3, 4, or 5) X-ray powder diffraction characteristic peaks selected from the group consisting of: 8.752°±0.2°, 19.298°±0.2°, 16.631°±0.2°, 17.601°±0.2°, 25.289°±0.2°;
the crystal form is a salt crystal form IX-A, and the X-ray powder diffraction pattern of the salt crystal form IX-A includes 2 or more (3, 4, or 5) X-ray powder diffraction characteristic peaks selected from the group consisting of: 17.395°±0.2°, 21.697°±0.2°, 14.43°±0.2°, 13.765°±0.2°, 22.166°±0.2°;
the crystal form is a salt crystal form IX-B, and the X-ray powder diffraction pattern of the salt crystal form IX-B includes 2 or more (3, 4, or 5) X-ray powder diffraction characteristic peaks selected from the group consisting of: 20.835°±0.2°, 13.293°±0.2°, 17.98°±0.2°, 23.877°±0.2°, 21.622°±0.2°;
the crystal form is a salt crystal form IX-C, and the X-ray powder diffraction pattern of the salt crystal form IX-C includes 2 or more (3, 4, or 5) X-ray powder diffraction characteristic peaks selected from the group consisting of: 20.232°±0.2°, 18.948°±0.2°, 23.486°±0.2°, 15.549°±0.2°, 19.362°±0.2°;
the crystal form is salt crystal form IX-D, and the X-ray powder diffraction pattern of the salt crystal form IX-D includes 2 or more (3, 4, or 5) X-ray powder diffraction characteristic peaks selected from the group consisting of: 18.88°±0.2°, 22.356°±0.2°, 15.398°±0.2°, 21.954°±0.2°, 9.411°±0.2°;
the crystal form is a salt crystal form IX-E, and the X-ray powder diffraction pattern of the salt crystal form IX-E includes 2 or more (3, 4, or 5) X-ray powder diffraction characteristic peaks selected from the group consisting of: 22.072°±0.2°, 17.415°±0.2°, 18.438°±0.2°, 18.002°±0.2°, 11.596°±0.2°;
the crystal form is a salt crystal form IX-F, and the X-ray powder diffraction pattern of the salt crystal form IX-F includes 2 or more (3, 4, or 5) X-ray powder diffraction characteristic peaks selected from the group consisting of: 19.477°±0.2°, 23.233°±0.2°, 16.372°±0.2°, 26.063°±0.2°, 21.02°±0.2°;
the crystal form is a free base crystal form Form A, and the X-ray powder diffraction pattern of the free base crystal form Form A includes 2 or more (3, 4, or 5) X-ray powder diffraction characteristic peaks selected from the group consisting of: 20.536°±0.2°, 16.957°±0.2°, 15.689°±0.2°, 19.34°±0.2°, 9.858°±0.2°;
the crystal form is a free base crystal form Form C, and the X-ray powder diffraction pattern of the free base crystal form Form C includes 2 or more (3, 4, or 5) X-ray powder diffraction characteristic peaks selected from the group consisting of: 20.503 ° ± 0.2 °, 18.54 ° ± 0.2 °, 17.404 ° ± 0.2 °, 16.871 ° ± 0.2 °, 16.214 ° ± 0.2 °;
the crystal form is a free base crystal form Form D, and the X-ray powder diffraction pattern of the free base crystal form Form D includes 2 or more (3, 4, or 5) X-ray powder diffraction characteristic peaks selected from the group consisting of: 20.722 ° ± 0.2 °, 14.963 ° ± 0.2 °, 6.586 ° ± 0.2 °, 12.967 ° ± 0.2 °, 17.286 ° ± 0.2 °;
the crystal form is a free base crystal form Form E, and the X-ray powder diffraction pattern of the free base crystal form Form E includes 2 or more (3, 4, or 5) X-ray powder diffraction characteristic peaks selected from the group consisting of: 8.559 ° ± 0.2 °, 13.753 ° ± 0.2 °, 20.533 ° ± 0.2 °, 12.913 ° ± 0.2 °, 6.776 ° ± 0.2 °;
the crystal form is a free base crystal form Form F, and the X-ray powder diffraction pattern of the free base crystal form Form F includes 2 or more (3, 4, or 5) X-ray powder diffraction characteristic peaks selected from the group consisting of: 19.324 ° ± 0.2 °, 25.949 ° ± 0.2 °, 12.82 ° ± 0.2 °, 8.952 ° ± 0.2 °, 6.36 ° ± 0.2 °;
the crystal form is a free base crystal form Form G, and the X-ray powder diffraction pattern of the free base crystal form Form G includes 2 or more (3, 4, or 5) X-ray powder diffraction characteristic peaks selected from the group consisting of: 8.228 ° ± 0.2 °, 19.529 ° ± 0.2 °, 16.987 ° ± 0.2 °, 5.253 ° ± 0.2 °, 24.365 ° ± 0.2 °;
the crystal form is a free base crystal form Form H, and the X-ray powder diffraction pattern of the free base crystal form Form H includes 2 or more (3, 4, or 5) X-ray powder diffraction characteristic peaks selected from the group consisting of: 19.327 ° ± 0.2 °, 23.084 ° ± 0.2 °, 25.903 ° ± 0.2 °, 12.823 ° ± 0.2 °, 19.18 ° ± 0.2 °;
the crystal form is a free base crystal form Form I, and the X-ray powder diffraction pattern of the free base crystal form Form I includes 2 or more (3, 4, or 5) X-ray powder diffraction characteristic peaks selected from the group consisting of: 22.011 ° ± 0.2 °, 20.226 ° ± 0.2 °, 20.986 ° ± 0.2 °, 13.416 ° ± 0.2 °, 13.033 ° ± 0.2 °;
the crystal form is a free base crystal form Form J, and the X-ray powder diffraction pattern of the free base crystal form Form J includes 2 or more (3, 4, or 5) X-ray powder diffraction characteristic peaks selected from the group consisting of: 6.403 ° ± 0.2 °, 21.406 ° ± 0.2 °, 11.755 ° ± 0.2 °, 21.045 ° ± 0.2 °, 17.314 ° ± 0.2 °;
the crystal form is a free base crystal form Form K, and the X-ray powder diffraction pattern of the free base crystal form Form K includes 2 or more (3, 4, or 5) X-ray powder diffraction characteristic peaks selected from the group consisting of: 19.314 ° ± 0.2 °, 23.064 ° ± 0.2 °, 5.637 ° ± 0.2 °, 11.396 ° ± 0.2 °, 17.197 ° ± 0.2 °;
the crystal form is a free base crystal form Form L, and the X-ray powder diffraction pattern of the free base crystal form Form L includes 2 or more (3, 4, or 5) X-ray powder diffraction characteristic peaks selected from the group consisting of: 21.963°±0.2°, 22.518°±0.2°, 20.943°±0.2°, 19.12°±0.2°, 16.382°±0.2°;
the crystal form is a free base crystal form Form M, and the X-ray powder diffraction pattern of the free base crystal form Form M includes 2 or more (3, 4, or 5) X-ray powder diffraction characteristic peaks selected from the group consisting of: 20.09°±0.2°, 15.616°±0.2°, 20.566°±0.2°, 9.241°±0.2°, 16.989°±0.2°;
the crystal form is a free base crystal form Form N, and the X-ray powder diffraction pattern of the free base crystal form Form N includes 2 or more (3, 4, or 5) X-ray powder diffraction characteristic peaks selected from the group consisting of: 20.569 ° ± 0.2 °, 16.952 ° ± 0.2 °, 19.507 ° ± 0.2 °, 16.069 ° ± 0.2 °, 7.91 ° ± 0.2 °;
the crystal form is a free base crystal form Form O, and the X-ray powder diffraction pattern of the free base crystal form Form O includes 2 or more (3, 4, or 5) X-ray powder diffraction characteristic peaks selected from the group consisting of: 21.72 ° ± 0.2 °, 18.877 ° ± 0.2 °, 17.769 ° ± 0.2 °, 16.511 ° ± 0.2 °, 20.049 ° ± 0.2 °;
the crystal form is a free base crystal form Form P, and the X-ray powder diffraction pattern of the free base crystal form Form P includes 2 or more (3, 4, or 5) X-ray powder diffraction characteristic peaks selected from the group consisting of: 8.249 ° ± 0.2 °, 16.791 ° ± 0.2 °, 15.796 ° ± 0.2 °, 19.798 ° ± 0.2 °, 22.14 ° ± 0.2 °;
the crystal form is a free base crystal form Form Q, and the X-ray powder diffraction pattern of the free base crystal form Form Q includes 2 or more (3, 4, or 5) X-ray powder diffraction characteristic peaks selected from the group consisting of: 8.114 ° ± 0.2 °, 19.767 ° ± 0.2 °, 22.837 ° ± 0.2 °, 12.949 ° ± 0.2 °, 21.397 ° ± 0.2 °;
the crystal form is a free base crystal form Form R, and the X-ray powder diffraction pattern of the free base crystal form Form R includes 2 or more (3, 4, or 5) X-ray powder diffraction characteristic peaks selected from the group consisting of: 22.248 ° ± 0.2 °, 12.339 ° ± 0.2 °, 19.597 ° ± 0.2 °, 22.748 ° ± 0.2 °, 17.034 ° ± 0.2 °;
the crystal form is a free base crystal form Form S, and the X-ray powder diffraction pattern of the free base crystal form Form S includes 2 or more (3, 4, or 5) X-ray powder diffraction characteristic peaks selected from the group consisting of: 5.388 ° ± 0.2 °, 18.299 ° ± 0.2 °, 10.892 ° ± 0.2 °, 16.589 ° ± 0.2 °, 20.774 ° ± 0.2 °;

In another preferred embodiment, the crystal form is a crystal form selected from the group consisting of (wherein "has one or more X-ray powder diffraction characteristic peaks selected from the group consisting of" refers to that the crystal form has 5 or more, such as 6, 7, 8, 9, 10, 12, 15 or 20, X-ray powder diffraction characteristic peaks selected from the group consisting of):
the crystal form is the salt crystal form I-A, and the salt crystal form I-A further has one or more X-ray powder diffraction characteristic peaks selected from the group consisting of: 24.186°±0.2°, 19.235°±0.2°, 17.135°±0.2°, 16.848°±0.2°, 12.786°±0.2°, 24.743°±0.2°, 17.992°±0.2°, 18.56°±0.2°, 12.375°±0.2°, 5.584°±0.2°, 10.978°±0.2°, 21.829°±0.2°, 28.451°±0.2°, 21.034°±0.2°, 19.832°±0.2°;
the crystal form is the salt crystal form I-B, and the salt crystal form I-B further has one or more X-ray powder diffraction characteristic peaks selected from the group consisting of: 24.741°±0.2°, 13.702°±0.2°, 23.493°±0.2°, 12.931°±0.2°, 18.012°±0.2°, 23.206°±0.2°, 12.482°±0.2°, 27.48°±0.2°, 23.009°±0.2°, 24.5°±0.2°, 14.085°±0.2°, 12.002°±0.2°, 26.106°±0.2°, 20.379°±0.2°, 10.064°±0.2°, 22.71°±0.2°, 25.653°±0.2°, 24.151°±0.2°, 8.967°±0.2°, 11.06°±0.2°, 6.56°±0.2°, 17.675°±0.2°, 20.886°±0.2°, 15.52°±0.2°, 9.39°±0.2°, 30.381°±0.2°, 28.579°±0.2°, 5.902°±0.2°, 29.863°±0.2°, 22.001°±0.2°, 21.463°±0.2°, 16.3°±0.2°, 26.746°±0.2°, 32.347°±0.2°, 35.995°±0.2°;
the crystal form is the salt crystal form I-C, and the salt crystal form I-C further has one or more X-ray powder diffraction characteristic peaks selected from the group consisting of: 12.443°±0.2°, 22.553°±0.2°, 11.588°±0.2°, 12.764°±0.2°, 23.357°±0.2°, 10.93°±0.2°, 14.126°±0.2°, 16.8°±0.2°, 20.219°±0.2°, 18.114°±0.2°, 9.22°±0.2°, 17.146°±0.2°, 10.033°±0.2°, 21.797°±0.2°, 26.531°±0.2°, 20.676°±0.2°, 16.128°±0.2°, 21.078°±0.2°, 15.292°±0.2°, 37.716°±0.2°;
the crystal form is the salt crystal form II-A, and the salt crystal form II-A further has one or more X-ray powder diffraction characteristic peaks selected from the group consisting of: 20.504°±0.2°, 6.404°±0.2°, 22.168°±0.2°, 24.809°±0.2°, 8.079°±0.2°, 12.049°±0.2°, 7.599°±0.2°, 14.156°±0.2°, 28.701°±0.2°, 19.947°±0.2°, 25.28°±0.2°, 8.973°±0.2°, 16.494°±0.2°, 12.411°±0.2°, 11.643°±0.2°, 27.741°±0.2°, 12.804°±0.2°, 32.311°±0.2°, 26.183°±0.2°;
the crystal form is the salt crystal form II-B, and the salt crystal form II-B further has one or more X-ray powder diffraction characteristic peaks selected from the group consisting of: 20.904 ° ± 0.2 °, 9.02 ° ± 0.2 °, 14.963 ° ± 0.2 °, 16.106 ° ± 0.2 °, 18.159 ° ± 0.2 °, 4.093 ° ± 0.2 °;
the crystal form is the salt crystal form II-C, and the salt crystal form II-C further has one or more X-ray powder diffraction characteristic peaks selected from the group consisting of: 11.704°±0.2°, 16.564°±0.2°, 8.665°±0.2°, 19.939°±0.2°, 27.791°±0.2°, 20.587°±0.2°, 12.603°±0.2°, 15.285°±0.2°, 16.911°±0.2°, 19.336°±0.2°, 18.563°±0.2°, 26.158°±0.2°, 9.711°±0.2°, 25.703°±0.2°, 23.442°±0.2°, 14.203°±0.2°, 24.9°±0.2°, 28.92°±0.2°, 31.336°±0.2°, 13.62°±0.2°;
the crystal form is the salt crystal form III-A, and the salt crystal form III-A further has one or more X-ray powder diffraction characteristic peaks selected from the group consisting of: 16.97°±0.2°, 18.427°±0.2°, 24.243°±0.2°, 20.545°±0.2°, 20.124°±0.2°, 21.289°±0.2°, 14.368°±0.2°, 25.954°±0.2°, 21.976°±0.2°, 30.329°±0.2°, 28.621°±0.2°, 10.253°±0.2°, 27.03°±0.2°, 32.555°±0.2°, 16.261°±0.2°, 30.07°±0.2°, 28.953°±0.2°, 19.523°±0.2°, 15.063°±0.2°, 27.784°±0.2°, 31.627°±0.2°, 41.318°±0.2°;
the crystal form is the salt crystal form III-B, and the salt crystal form III-B further has one or more X-ray powder diffraction characteristic peaks selected from the group consisting of: 20.243°±0.2°, 23.628°±0.2°, 24.494°±0.2°, 19.827°±0.2°, 18.705°±0.2°, 23.096°±0.2°, 21.151°±0.2°, 29.191°±0.2°, 11.507°±0.2°, 10.076°±0.2°, 7.053°±0.2°, 30.258°±0.2°, 17.86°±0.2°, 25.239°±0.2°, 4.442°±0.2°, 21.562°±0.2°, 15.847°±0.2°, 7.435°±0.2°, 13.474°±0.2°, 26.342°±0.2°, 19.309°±0.2°, 28.778°±0.2°, 27.626°±0.2°, 38.331°±0.2°;
the crystal form is the salt crystal form III-C, and the salt crystal form III-C further has one or more X-ray powder diffraction characteristic peaks selected from the group consisting of: 21.36°±0.2°, 22.78°±0.2°, 15.371°±0.2°, 28.275°±0.2°, 26.74°±0.2°, 31.615°±0.2°, 29.332°±0.2°, 4.507°±0.2°, 22.485°±0.2°, 17.935°±0.2°, 16.759°±0.2°, 18.25°±0.2°, 18.746°±0.2°, 25.926°±0.2°, 12.932°±0.2°, 10.125°±0.2°, 15.807°±0.2°, 23.727°±0.2°, 34.512°±0.2°, 4.866°±0.2°, 20.415°±0.2°, 14.639°±0.2°, 39.286°±0.2°, 11.223°±0.2°, 30.33°±0.2°, 35.619°±0.2°;
the crystal form is the salt crystal form IV-A, and the salt crystal form IV-A further has one or more X-ray powder diffraction characteristic peaks selected from the group consisting of: 22.271°±0.2°, 24.681°±0.2°, 20.389°±0.2°, 7.866°±0.2°, 18.339°±0.2°, 25.842°±0.2°, 9.546°±0.2°, 19.184°±0.2°, 16.168°±0.2°, 32.892°±0.2°, 17.538°±0.2°, 18.625°±0.2°, 21.014°±0.2°, 30.816°±0.2°, 17.09°±0.2°, 23.137°±0.2°, 30.574°±0.2°;
the crystal form is the salt crystal form V-A, and the salt crystal form V-A further has one or more X-ray powder diffraction characteristic peaks selected from the group consisting of: 17.104°±0.2°, 4.31°±0.2°, 17.374°±0.2°, 14.939°±0.2°, 21.4°±0.2°, 29.51°±0.2°, 6.319°±0.2°, 10.728°±0.2°, 6.524°±0.2°, 30.089°±0.2°, 27.924°±0.2°, 18.693°±0.2°, 7.084°±0.2°, 32.535°±0.2°, 25.051°±0.2°, 14.103°±0.2°, 20.847°±0.2°, 20.158°±0.2°, 39.538°±0.2°, 30.432°±0.2°;
the crystal form is the salt crystal form V-B, and the salt crystal form V-B further has one or more X-ray powder diffraction characteristic peaks selected from the group consisting of: 11.711°±0.2°, 18.356°±0.2°, 15.916°±0.2°, 9.279°±0.2°, 26.763°±0.2°, 8.67°±0.2°, 13.883°±0.2°, 8.292°±0.2°, 12.729°±0.2°, 26.159°±0.2°, 20.643°±0.2°, 14.695°±0.2°, 21.578°±0.2°, 23.761°±0.2°, 32.047°±0.2°, 25.1°±0.2°, 4.659°±0.2°, 15.563°±0.2°, 29.532°±0.2°;
the crystal form is the salt crystal form VI-A, and the salt crystal form VI-A further has one or more X-ray powder diffraction characteristic peaks selected from the group consisting of: 8.39°±0.2°, 14.974°±0.2°, 16.728°±0.2°, 4.667°±0.2°, 4.884°±0.2°, 8.937°±0.2°, 25.416°±0.2°, 14.393°±0.2°, 10.9°±0.2°, 22.593°±0.2°, 18.275°±0.2°, 9.346°±0.2°, 26.405°±0.2°, 18.928°±0.2°, 26.006°±0.2°, 22.013°±0.2°;
the crystal form is the salt crystal form VII-A, and the salt crystal form VII-A further has one or more X-ray powder diffraction characteristic peaks selected from the group consisting of: 11.58°±0.2°, 14.468°±0.2°, 20.398°±0.2°, 13.667°±0.2°, 25.16°±0.2°, 18.032°±0.2°, 21.293°±0.2°, 20.145°±0.2°, 14.952°±0.2°, 12.439°±0.2°, 13.387°±0.2°, 23.299°±0.2°, 25.584°±0.2°, 9.577°±0.2°, 21.067°±0.2°, 26.73°±0.2°, 27.7°±0.2°, 18.511°±0.2°, 7.514°±0.2°, 28.217°±0.2°, 16.773°±0.2°, 19.824°±0.2°, 33.282°±0.2°, 39.237°±0.2°, 15.864°±0.2°, 34.927°±0.2°, 37.048°±0.2°, 30.018°±0.2°, 29.501°±0.2°;
the crystal form is the salt crystal form VIII-A, and the salt crystal form VIII-A further has one or more X-ray powder diffraction characteristic peaks selected from the group consisting of: 3.834 ° ± 0.2 °, 4.282 ° ± 0.2 °, 7.593 ° ± 0.2 °, 21.198 ° ± 0.2 °, 16.946 ° ± 0.2 °, 25.302 ° ± 0.2 °, 8.451 ° ± 0.2 °, 11.334 ° ± 0.2 °, 17.526 ° ± 0.2 °, 19.847 ° ± 0.2 °, 15.123 ° ± 0.2 °, 23.412 ° ± 0.2 °, 24.234 ° ± 0.2 °, 12.73 ° ± 0.2 °, 19.367 ° ± 0.2 °, 27.174 ° ± 0.2 °, 10.128 ° ± 0.2 °, 13.942 ° ± 0.2 °, 28.858 ° ± 0.2 °, 14.369 ° ± 0.2 °;
the crystal form is the salt crystal form VIII-B, and the salt crystal form VIII-B further has one or more X-ray powder diffraction characteristic peaks selected from the group consisting of: 9.887°±0.2°, 14.008°±0.2°, 18.024°±0.2°, 14.638°±0.2°, 7.013°±0.2°, 26.786°±0.2°, 7.43°±0.2°, 11.851°±0.2°, 9.133°±0.2°, 19.462°±0.2°, 9.364°±0.2°, 22.721°±0.2°, 16.517°±0.2°, 22.23°±0.2°, 6.089°±0.2°, 20.958°±0.2°, 24.636°±0.2°, 17.356°±0.2°, 15.702°±0.2°, 26.053°±0.2°, 18.609°±0.2°, 33.042°±0.2°, 12.943°±0.2°, 13.338°±0.2°, 19.81°±0.2°, 12.564°±0.2°;
the crystal form is the salt crystal form VIII-C, and the salt crystal form VIII-C further has one or more X-ray powder diffraction characteristic peaks selected from the group consisting of: 4.296 ° ± 0.2 °, 16.981 ° ± 0.2 °, 21.325 ° ± 0.2 °, 19.842 ° ± 0.2 °, 13.995 ° ± 0.2 °, 8.468 ° ± 0.2 °, 12.953 ° ± 0.2 °, 23.516 ° ± 0.2 °, 26.44 ° ± 0.2 °, 12.733 ° ± 0.2 °, 24.186 ° ± 0.2 °, 21.759 ° ± 0.2 °, 14.942 ° ± 0.2 °, 3.343 ° ± 0.2 ° °, 27.226 ° ± 0.2 °, 22.526 ° ± 0.2 °, 10.151 ° ± 0.2 °, 28.815 ° ± 0.2 °, 11.717 ° ± 0.2 °, 7.66 ° ± 0.2 °, 6.606 ° ± 0.2 °, 14.69 ° ± 0.2 °, 28.114 ° ± 0.2 °, 10.855 ° ± 0.2 °;
the crystal form is the salt crystal form IX-A, and the salt crystal form IX-A further has one or more X-ray powder diffraction characteristic peaks selected from the group consisting of: 18.824°±0.2°, 9.694°±0.2°, 7.013°±0.2°, 27.369°±0.2°, 20.086°±0.2°, 22.964°±0.2°, 16.176°±0.2°, 24.522°±0.2°, 12.558°±0.2°, 21.268°±0.2°, 24.93°±0.2°, 11.528°±0.2°, 18.08°±0.2°, 28.083°±0.2°, 16.532°±0.2°, 28.307°±0.2°, 20.459°±0.2°, 40.496°±0.2°, 25.827°±0.2°, 35.1°±0.2°, 32.649°±0.2°;
the crystal form is the salt crystal form IX-B, and the salt crystal form IX-B further has one or more X-ray powder diffraction characteristic peaks selected from the group consisting of: 11.265°±0.2°, 24.592°±0.2°, 14.698°±0.2°, 6.767°±0.2°, 6.283°±0.2°, 24.307°±0.2°, 12.566°±0.2°, 17.252°±0.2°, 21.169°±0.2°, 22.434°±0.2°, 15.562°±0.2°, 8.826°±0.2°, 19.852°±0.2°, 26.598°±0.2°, 25.069°±0.2°, 22.904°±0.2°, 14.075°±0.2°, 14.285°±0.2°, 20.468°±0.2°, 25.677°±0.2°, 27.967°±0.2°, 10.209°±0.2°, 16.935°±0.2°, 27.314°±0.2°, 13.651°±0.2°, 19.027°±0.2°, 9.608°±0.2°, 16.506°±0.2°, 31.547°±0.2°, 26.026°±0.2°, 28.35°±0.2°, 29.367°±0.2°, 34.221°±0.2°;
the crystal form is the salt crystal form IX-C, and the salt crystal form IX-C further has one or more X-ray powder diffraction characteristic peaks selected from the group consisting of: 10.123°±0.2°, 9.772°±0.2°, 24.632°±0.2°, 15.856°±0.2°, 7.962°±0.2°, 18.366°±0.2°, 6.476°±0.2°, 12.023°±0.2°, 24.033°±0.2°, 13.145°±0.2°, 27.497°±0.2°, 16.832°±0.2°, 26.629°±0.2°, 12.631°±0.2°, 21.536°±0.2°, 6.052°±0.2°, 24.943°±0.2°, 14.943°±0.2°, 25.899°±0.2°, 6.984°±0.2°, 16.419°±0.2°, 14.218°±0.2°, 22.661°±0.2°, 13.743°±0.2°, 11.453°±0.2°, 28.746°±0.2°;
the crystal form is the salt crystal form IX-D, and the salt crystal form IX-D further has one or more X-ray powder diffraction characteristic peaks selected from the group consisting of: 19.966°±0.2°, 14.067°±0.2°, 24.809°±0.2°, 25.866°±0.2°, 8.338°±0.2°, 17.595°±0.2°, 28.502°±0.2°, 27.737°±0.2°, 13.596°±0.2°, 11.512°±0.2°;
the crystal form is the salt crystal form IX-E, and the salt crystal form IX-E further has one or more X-ray powder diffraction characteristic peaks selected from the group consisting of: 23.237°±0.2°, 25.721°±0.2°, 16.1°±0.2°, 9.191°±0.2°, 8.322°±0.2°, 24.92°±0.2°, 27.119°±0.2°, 13.894°±0.2°, 19.383°±0.2°, 22.862°±0.2°, 24.401°±0.2°, 29.059°±0.2°, 16.686°±0.2°, 9.99°±0.2°, 12.063°±0.2°, 26.392°±0.2°, 28.021°±0.2°, 15.79°±0.2°, 12.411°±0.2°, 32.014°±0.2°, 13.014°±0.2°, 14.366°±0.2°, 14.94°±0.2°, 29.918°±0.2°;
the crystal form is the salt crystal form IX-F, and the salt crystal form IX-F further has one or more X-ray powder diffraction characteristic peaks selected from the group consisting of: 22.74°±0.2°, 10.901°±0.2°, 15.633°±0.2°, 17.35°±0.2°, 12.98°±0.2°, 21.762°±0.2°, 23.625°±0.2°, 14.101°±0.2°, 24.702°±0.2°, 24.398°±0.2°, 7.543°±0.2°, 13.187°±0.2°, 14.686°±0.2°, 29.425°±0.2°, 8.95°±0.2°, 16.965°±0.2°, 5.441°±0.2°, 19.89°±0.2°, 9.46°±0.2°, 28.127°±0.2°, 27.504°±0.2°, 29.188°±0.2°, 18.622°±0.2°, 11.528°±0.2°, 28.477°±0.2°, 12.672°±0.2°, 15.141°±0.2°, 39.475°±0.2°, 32.522°±0.2°;
the crystal form is the free base crystal form Form A, and the free base crystal form Form A further has one or more X-ray powder diffraction characteristic peaks selected from the group consisting of: 11.738°±0.2°, 15.33°±0.2°, 16.072°±0.2°, 19.858°±0.2°, 14.126°±0.2°, 8.676°±0.2°, 23.15°±0.2°, 12.77°±0.2°, 14.805°±0.2°, 25.859°±0.2°, 18.855°±0.2°, 5.29°±0.2°, 18.263°±0.2°, 8.445°±0.2°, 10.682°±0.2°, 21.863°±0.2°, 26.213°±0.2°, 24.796°±0.2°, 12.323°±0.2°, 28.899°±0.2°, 26.538°±0.2°, 31.755°±0.2°, 7.602°±0.2°, 32.581°±0.2°;
the crystal form is the free base crystal form Form C, and the free base crystal form Form C further has one or more X-ray powder diffraction characteristic peaks selected from the group consisting of: 11.736°±0.2°, 15.654°±0.2°, 22.017°±0.2°, 19.347°±0.2°, 22.166°±0.2°, 8.954°±0.2°, 9.835°±0.2°, 19.874°±0.2°, 18.853°±0.2°, 23.06°±0.2°, 25.8°±0.2°, 18.129°±0.2°, 14.094°±0.2°, 28.368°±0.2°, 14.724°±0.2°, 8.601°±0.2°, 15.353°±0.2°, 12.754°±0.2°, 24.977°±0.2°, 11.035°±0.2°, 24.57°±0.2°, 5.254°±0.2°, 8.395°±0.2°, 14.996°±0.2°, 12.273°±0.2°, 27.18°±0.2°, 40.473°±0.2°, 23.782°±0.2°, 32.539°±0.2°, 41.542°±0.2°, 31.819°±0.2°;
the crystal form is the free base crystal form Form D, and the free base crystal form Form D further has one or more X-ray powder diffraction characteristic peaks selected from the group consisting of: 10.025°±0.2°, 25.554°±0.2°, 19.364°±0.2°, 8.401°±0.2°, 11.974°±0.2°, 19.86°±0.2°, 8.191°±0.2°, 13.312°±0.2°, 14.52°±0.2°, 25.035°±0.2°, 24.033°±0.2°, 26.213°±0.2°, 16.529°±0.2°, 23.323°±0.2°, 26.933°±0.2°, 15.982°±0.2°, 18.898°±0.2°, 17.711°±0.2°, 7.882°±0.2°, 21.949°±0.2°, 32.042°±0.2°, 22.526°±0.2°, 33.893°±0.2°, 27.981°±0.2°, 32.821°±0.2°, 41.062°±0.2°, 11.008°±0.2°, 27.218°±0.2°, 33.206°±0.2°, 39.812°±0.2°;
the crystal form is the free base crystal form Form E, and the free base crystal form Form E also has one or more X-ray powder diffraction characteristic peaks selected from the group consisting of: 18.511°±0.2°, 13.521°±0.2°, 15.128°±0.2°, 11.724°±0.2°, 4.23°±0.2°, 24.687°±0.2°, 21.235°±0.2°, 16.27°±0.2°, 17.677°±0.2°, 23.693°±0.2°, 20.014°±0.2°, 15.483°±0.2°, 16.887°±0.2°, 7.482°±0.2°, 22.342°±0.2°, 24.031°±0.2°, 9.838°±0.2°, 18.137°±0.2°, 22.826°±0.2°, 19.427°±0.2°, 35.733°±0.2°, 41.301°±0.2°, 39.689°±0.2°, 30.603°±0.2°;
the crystal form is the free base crystal form Form F, and the free base crystal form Form F further has one or more X-ray powder diffraction characteristic peaks selected from the group consisting of: 39.352°±0.2°, 23.083°±0.2°, 17.224°±0.2°, 16.746°±0.2°, 20.632°±0.2°, 9.31°±0.2°, 18.407°±0.2°, 17.04°±0.2°, 14.49°±0.2°, 16.186°±0.2°, 32.354°±0.2°, 33.337°±0.2°, 35.404°±0.2°, 10.229°±0.2°, 18.753°±0.2°, 30.373°±0.2°, 36.679°±0.2°, 8.413°±0.2°, 15.142°±0.2°, 17.446°±0.2°, 17.87°±0.2°, 18.054°±0.2°, 23.348°±0.2°, 23.658°±0.2°, 24.109°±0.2°, 25.216°±0.2°, 26.831°±0.2°, 28.946°±0.2°, 29.351°±0.2°, 32.555°±0.2°, 33.903°±0.2°, 38.715°±0.2°, 38.9°±0.2°;
the crystal form is the free base crystal form Form G, and the free base crystal form Form G further has one or more X-ray powder diffraction characteristic peaks selected from the group consisting of: 21.414°±0.2°, 22.713°±0.2°, 15.917°±0.2°, 8.418°±0.2°, 18.689°±0.2°, 19.376°±0.2°, 23.137°±0.2°, 23.553°±0.2°, 9.86°±0.2°, 15.116°±0.2°, 20.615°±0.2°, 22.229°±0.2°, 15.462°±0.2°, 11.505°±0.2°, 28.21°±0.2°, 12.367°±0.2°, 25.112°±0.2°, 24.068°±0.2°, 13.687°±0.2°, 25.59°±0.2°, 10.954°±0.2°, 29.506°±0.2°, 27.964°±0.2°, 13.981°±0.2°, 26.905°±0.2°, 33.657°±0.2°, 34.776°±0.2°, 26.667°±0.2°, 14.35°±0.2°, 36.659°±0.2°, 17.671°±0.2°, 30.615°±0.2°;
the crystal form is the free base crystal form Form H, and the free base crystal form Form H further has one or more X-ray powder diffraction characteristic peaks selected from the group consisting of: 20.628°±0.2°, 17.222°±0.2°, 14.497°±0.2°, 16.169°±0.2°, 8.961°±0.2°, 17.01°±0.2°, 18.425°±0.2°, 9.229°±0.2°, 5.278°±0.2°, 16.826°±0.2°, 29.316°±0.2°, 10.67°±0.2°, 23.972°±0.2°, 15.318°±0.2°, 15.698°±0.2°, 23.639°±0.2°, 26.846°±0.2°, 6.353°±0.2°, 14.113°±0.2°, 15.054°±0.2°, 9.854°±0.2°, 11.773°±0.2°, 24.476°±0.2°, 24.963°±0.2°, 10.241°±0.2°, 13.111°±0.2°, 8.699°±0.2°, 39.333°±0.2°, 19.862°±0.2°, 21.147°±0.2°, 25.584°±0.2°, 26.519°±0.2°, 32.396°±0.2°, 26.134°±0.2°, 27.909°±0.2°, 14.765°±0.2°, 18.846°±0.2°, 21.522°±0.2°, 3.68°±0.2°, 24.769°±0.2°, 28.977°±0.2°, 30.354°±0.2°, 25.208°±0.2°, 30.091°±0.2°, 38.922°±0.2°, 27.214°±0.2°, 20.272°±0.2°, 21.902°±0.2°, 35.705°±0.2°, 36.765°±0.2°, 38.715°±0.2°, 37.543°±0.2°, 8.435°±0.2°, 28.386°±0.2°, 7.593°±0.2°, 17.851°±0.2°, 31.755°±0.2°, 30.955°±0.2°, 31.366°±0.2°, 34.492°±0.2°, 34.827°±0.2°, 40.054°±0.2°, 40.438°±0.2°;
the crystal form is the free base crystal form Form I, and the free base crystal form Form I further has one or more X-ray powder diffraction characteristic peaks selected from the group consisting of: 17.029°±0.2°, 14.962°±0.2°, 19.141°±0.2°, 22.563°±0.2°, 22.769°±0.2°, 11.299°±0.2°, 16.466°±0.2°, 5.593°±0.2°, 26.548°±0.2°, 8.231°±0.2°, 25.139°±0.2°, 6.617°±0.2°, 15.768°±0.2°, 23.72°±0.2°, 15.292°±0.2°, 23.975°±0.2°, 18.006°±0.2°, 21.324°±0.2°, 24.849°±0.2°, 24.623°±0.2°, 40.591°±0.2°, 26.263°±0.2°, 27.121°±0.2°, 27.369°±0.2°, 18.441°±0.2°, 19.907°±0.2°, 10.011°±0.2°, 14.558°±0.2°, 28.997°±0.2°, 23.286°±0.2°, 9.288°±0.2°, 14.109°±0.2°, 29.31°±0.2°, 32.007°±0.2°, 30.722°±0.2°;
the crystal form is the free base crystal form Form J, and the free base crystal form Form J further has one or more X-ray powder diffraction characteristic peaks selected from the group consisting of: 9.63°±0.2°, 9.118°±0.2°, 5.655°±0.2°, 8.351°±0.2°, 16.869°±0.2°, 15.439°±0.2°, 9.895°±0.2°, 16.151°±0.2°, 19.388°±0.2°, 12.415°±0.2°, 19.789°±0.2°, 25.318°±0.2°, 13.448°±0.2°, 10.481°±0.2°, 18.763°±0.2°, 11.187°±0.2°, 14.2°±0.2°;
the crystal form is the free base crystal form Form K, and the free base crystal form Form K further has one or more X-ray powder diffraction characteristic peaks selected from the group consisting of: 20.625°±0.2°, 22.089°±0.2°, 14.482°±0.2°, 25.895°±0.2°, 18.418°±0.2°, 12.808°±0.2°, 9.272°±0.2°, 8.948°±0.2°, 16.154°±0.2°, 21.14°±0.2°, 20.21°±0.2°, 16.539°±0.2°, 22.61°±0.2°, 18.109°±0.2°, 29.288°±0.2°, 7.931°±0.2°, 23.92°±0.2°, 19.98°±0.2°, 15.512°±0.2°, 8.35°±0.2°, 26.836°±0.2°, 15.867°±0.2°, 23.606°±0.2°, 26.668°±0.2°, 24.888°±0.2°, 32.296°±0.2°, 29.096°±0.2°, 10.215°±0.2°, 11.757°±0.2°, 24.467°±0.2°, 15.029°±0.2°, 39.238°±0.2°, 28.325°±0.2°, 27.845°±0.2°, 40.494°±0.2°;
the crystal form is the free base crystal form Form L, and the free base crystal form Form L further has one or more X-ray powder diffraction characteristic peaks selected from the group consisting of: 16.978°±0.2°, 15.741°±0.2°, 20.074°±0.2°, 11.225°±0.2°, 22.736°±0.2°, 15.252°±0.2°, 17.989°±0.2°, 5.574°±0.2°, 26.461°±0.2°, 8.088°±0.2°, 18.437°±0.2°, 21.277°±0.2°, 18.632°±0.2°, 23.539°±0.2°, 10.961°±0.2°, 24.576°±0.2°, 16.091°±0.2°, 20.459°±0.2°, 25.703°±0.2°, 9.279°±0.2°, 23.91°±0.2°, 14.018°±0.2°, 28.958°±0.2°, 24.86°±0.2°, 32.879°±0.2°, 27.314°±0.2°, 35.773°±0.2°, 33.542°±0.2°, 29.252°±0.2°, 40.574°±0.2°;
the crystal form is the free base crystal form Form M, and the free base crystal form Form M further has one or more X-ray powder diffraction characteristic peaks selected from the group consisting of: 21.173°±0.2°, 9.041°±0.2°, 8.828°±0.2°, 15.34°±0.2°, 16.449°±0.2°, 26.113°±0.2°, 16.099°±0.2°, 19.436°±0.2°, 26.406°±0.2°, 9.717°±0.2°, 5.282°±0.2°, 21.946°±0.2°, 14.107°±0.2°, 23.263°±0.2°, 10.669°±0.2°, 24.735°±0.2°, 11.76°±0.2°, 18.278°±0.2°;
the crystal form is the free base crystal form Form N, and the free base crystal form Form N further has one or more X-ray powder diffraction characteristic peaks selected from the group consisting of: 19.353°±0.2°, 15.677°±0.2°, 18.461°±0.2°, 23.017°±0.2°, 18.837°±0.2°, 23.23°±0.2°, 9.851°±0.2°, 10.726°±0.2°, 24.261°±0.2°, 21.615°±0.2°, 25.889°±0.2°, 26.902°±0.2°, 18.255°±0.2°, 23.903°±0.2°, 15.371°±0.2°, 22.713°±0.2°, 19.864°±0.2°, 25.661°±0.2°, 14.112°±0.2°, 14.798°±0.2°, 12.769°±0.2°, 29.013°±0.2°, 12.46°±0.2°, 24.799°±0.2°, 26.464°±0.2°, 11.751°±0.2°, 13.143°±0.2°, 5.108°±0.2°, 30.667°±0.2°, 34.665°±0.2°, 33.332°±0.2°, 28.357°±0.2°, 8.608°±0.2°, 39.721°±0.2°;
the crystal form is the free base crystal form Form O, and the free base crystal form Form O further has one or more X-ray powder diffraction characteristic peaks selected from the group consisting of: 9.294°±0.2°, 22.47°±0.2°, 11.139°±0.2°, 22.635°±0.2°, 15.11°±0.2°, 19.334°±0.2°, 20.676°±0.2°, 16.21°±0.2°, 26.024°±0.2°, 18.318°±0.2°, 8.19°±0.2°, 13.891°±0.2°, 22.05°±0.2°, 23.086°±0.2°, 5.499°±0.2°, 15.632°±0.2°, 16.053°±0.2°, 24.463°±0.2°, 8.501°±0.2°, 12.827°±0.2°, 28.113°±0.2°, 28.729°±0.2°, 17.216°±0.2°, 28.978°±0.2°, 20.921°±0.2°, 10.828°±0.2°, 14.485°±0.2°, 30.779°±0.2°, 27.64°±0.2°, 29.609°±0.2°, 23.81°±0.2°, 32.367°±0.2°, 23.654°±0.2°, 33.6°±0.2°, 31.634°±0.2°, 25.085°±0.2°, 37.002°±0.2°, 11.83°±0.2°, 36.126°±0.2°, 40.712°±0.2°, 41.521°±0.2°;
the crystal form is the free base crystal form Form P, and the free base crystal form Form P further has one or more X-ray powder diffraction characteristic peaks selected from the group consisting of: 24.844°±0.2°, 22.643°±0.2°, 23.927°±0.2°, 15.998°±0.2°, 5.413°±0.2°, 11.243°±0.2°, 19.109°±0.2°, 15.204°±0.2°, 26.032°±0.2°, 20.313°±0.2°, 23.474°±0.2°, 18.855°±0.2°, 11.485°±0.2°, 19.5°±0.2°, 27.044°±0.2°, 30.158°±0.2°, 10.949°±0.2°, 25.584°±0.2°, 35.902°±0.2°, 28.805°±0.2°, 26.693°±0.2°, 27.751°±0.2°, 35.669°±0.2°, 9.756°±0.2°, 12.331°±0.2°, 34.452°±0.2°, 14.013°±0.2°, 32.583°±0.2°, 33.313°±0.2°, 39.326°±0.2°, 13.376°±0.2°, 31.555°±0.2°, 17.643°±0.2°;
the crystal form is the free base crystal form Form Q, and the free base crystal form Form Q further has one or more X-ray powder diffraction characteristic peaks selected from the group consisting of: 19.354°±0.2°, 18.995°±0.2°, 11.472°±0.2°, 8.367°±0.2°, 18.593°±0.2°, 16.688°±0.2°, 9.591°±0.2°, 8.825°±0.2°, 26.164°±0.2°, 26.574°±0.2°, 17.931°±0.2°, 24.918°±0.2°, 11.864°±0.2°, 17.354°±0.2°, 5.679°±0.2°, 17.66°±0.2°, 20.677°±0.2°, 15.589°±0.2°, 32.45°±0.2°, 27.084°±0.2°, 11.151°±0.2°, 31.897°±0.2°, 23.703°±0.2°, 35.188°±0.2°, 14.914°±0.2°, 15.758°±0.2°, 30.14°±0.2°, 27.238°±0.2°, 6.581°±0.2°, 23.209°±0.2°, 28.785°±0.2°, 34.05°±0.2°, 7.724°±0.2°, 14.273°±0.2°, 28.179°±0.2°, 29.984°±0.2°, 35.767°±0.2°, 24.539°±0.2°, 25.337°±0.2°, 39.77°±0.2°, 29.683°±0.2°, 31.355°±0.2°, 32.727°±0.2°, 38.588°±0.2°;
the crystal form is the free base crystal form Form R, and the free base crystal form Form R further has one or more X-ray powder diffraction characteristic peaks selected from the group consisting of: 6.579°±0.2°, 17.62°±0.2°, 13.831°±0.2°, 25.901°±0.2°, 24.013°±0.2°, 9.447°±0.2°, 28.991°±0.2°, 8.241°±0.2°, 13.556°±0.2°, 23.343°±0.2°, 20.822°±0.2°, 13.345°±0.2°, 16.664°±0.2°, 20.571°±0.2°, 19.406°±0.2°, 24.358°±0.2°, 24.919°±0.2°, 8.897°±0.2°, 39.905°±0.2°, 15.257°±0.2°, 28.601°±0.2°, 20.063°±0.2°, 33.028°±0.2°, 30.525°±0.2°, 21.408°±0.2°, 25.383°±0.2°, 36.219°±0.2°, 40.32°±0.2°, 30.27°±0.2°, 33.741°±0.2°, 32.489°±0.2°, 31.21°±0.2°, 26.826°±0.2°, 34.552°±0.2°, 12.777°±0.2°, 18.963°±0.2°, 31.607°±0.2°;
the crystal form is the free base crystal form Form S, and the free base crystal form Form S further has one or more X-ray powder diffraction characteristic peaks selected from the group consisting of: 18.103°±0.2°, 15.674°±0.2°, 7.917°±0.2°, 17.512°±0.2°, 21.377°±0.2°, 19.485°±0.2°, 16.025°±0.2°, 11.055°±0.2°, 18.622°±0.2°, 20.009°±0.2°, 23.269°±0.2°, 17.015°±0.2°, 12.162°±0.2°, 15.002°±0.2°, 8.735°±0.2°, 27.664°±0.2°, 8.239°±0.2°, 19.758°±0.2°, 24.248°±0.2°, 36.703°±0.2°, 25.547°±0.2°, 12.903°±0.2°.

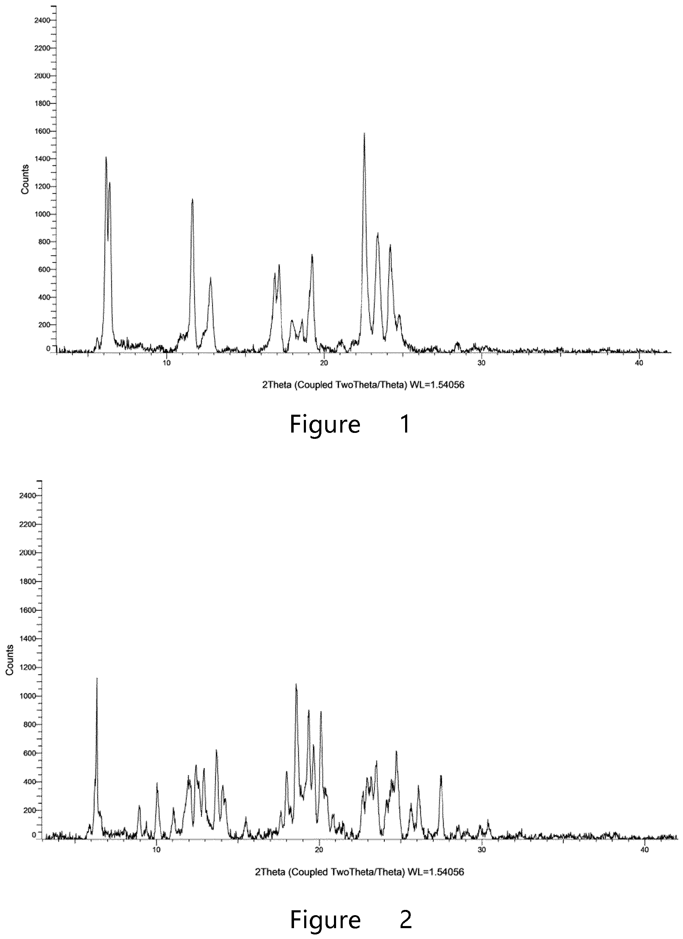

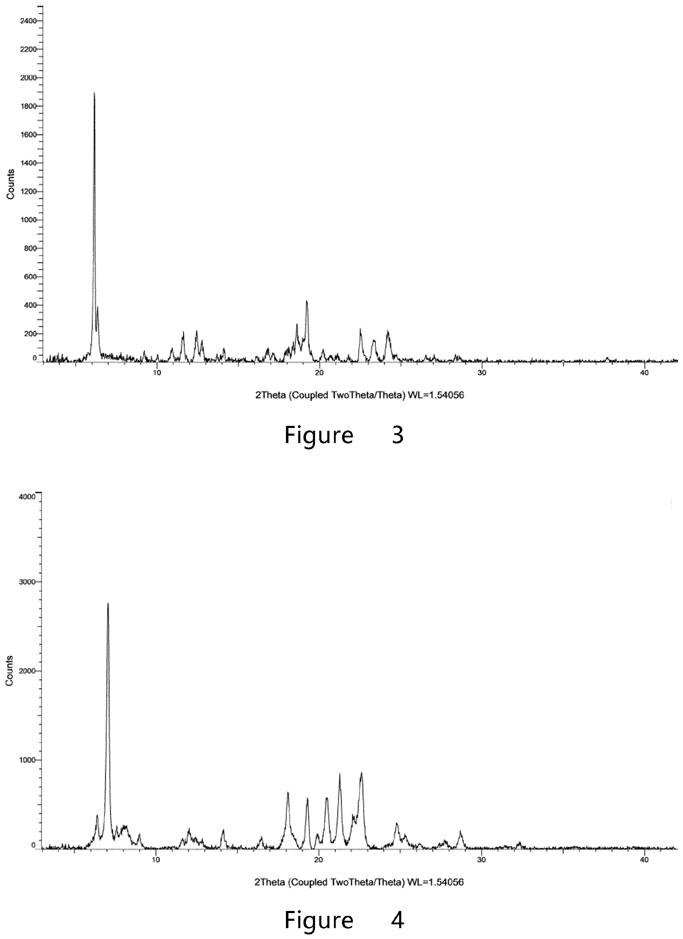

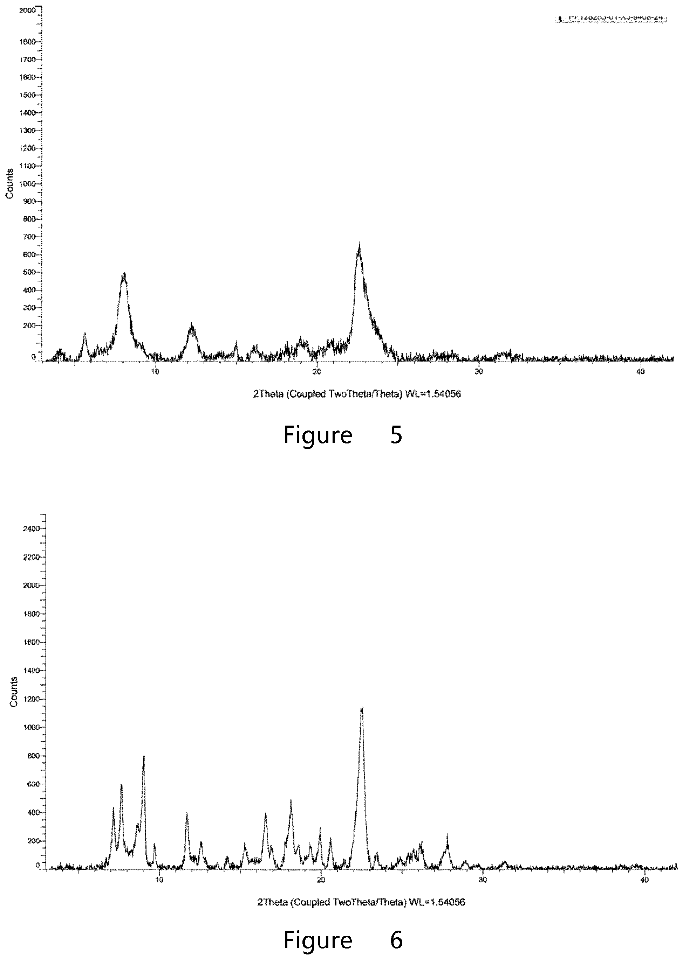

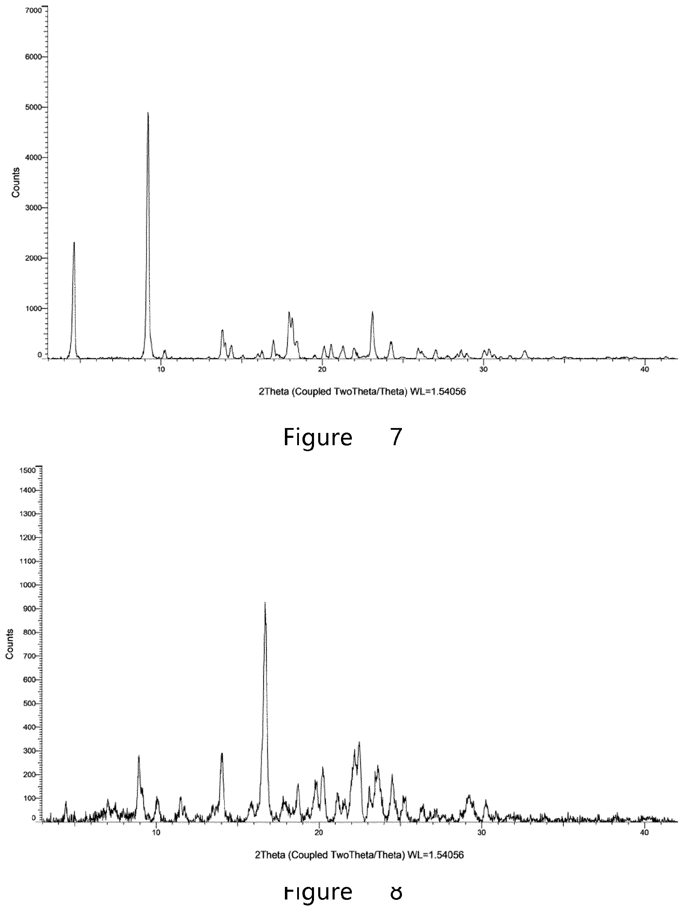

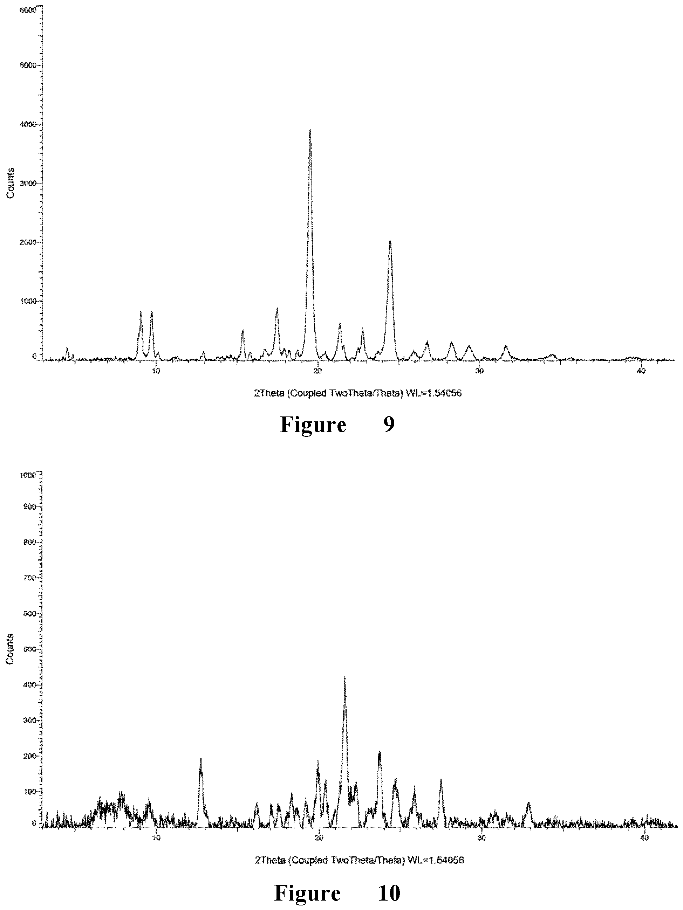

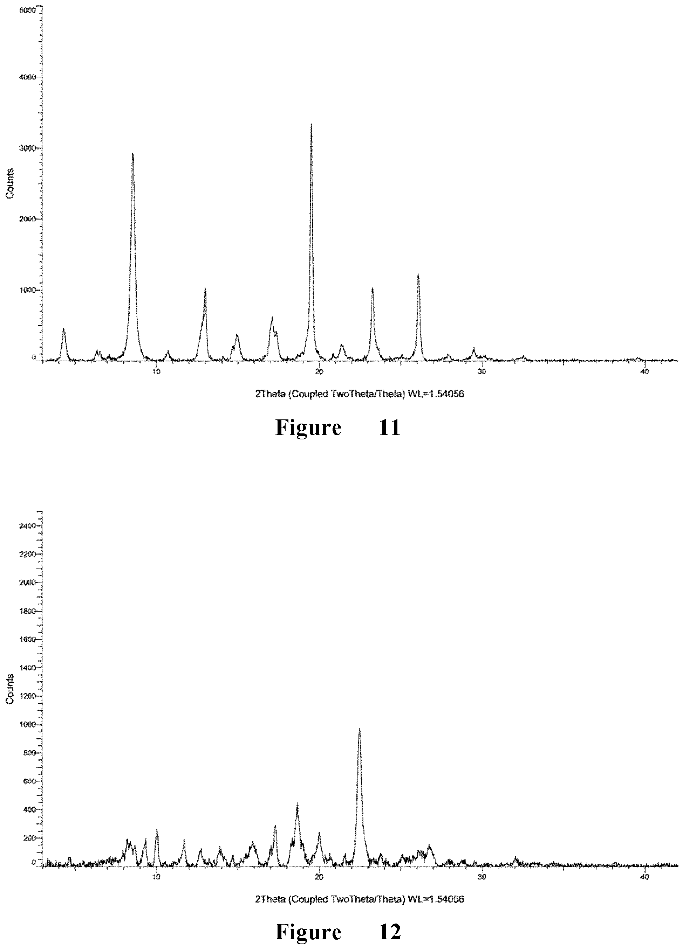

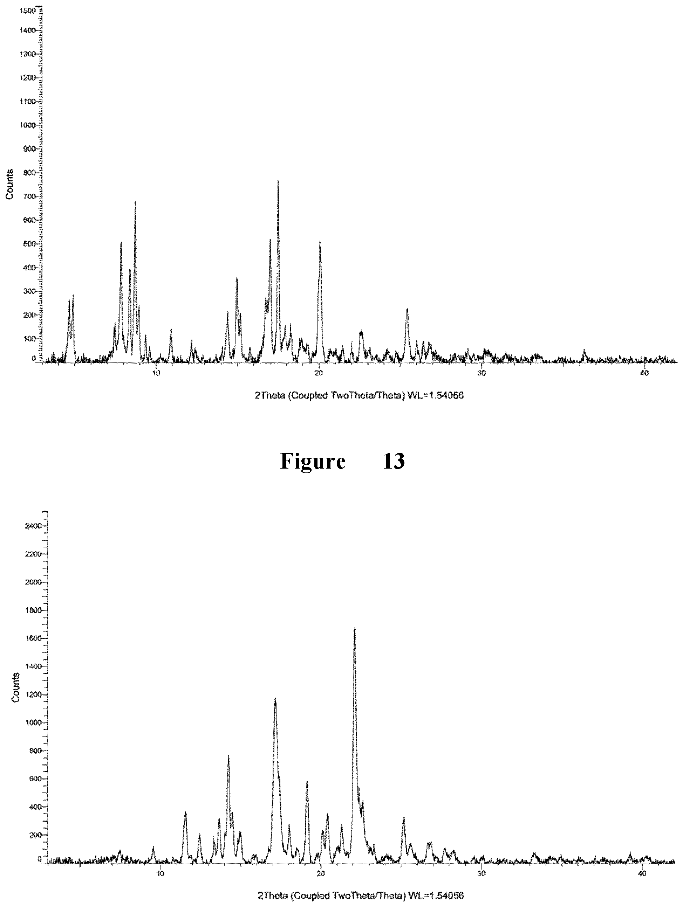

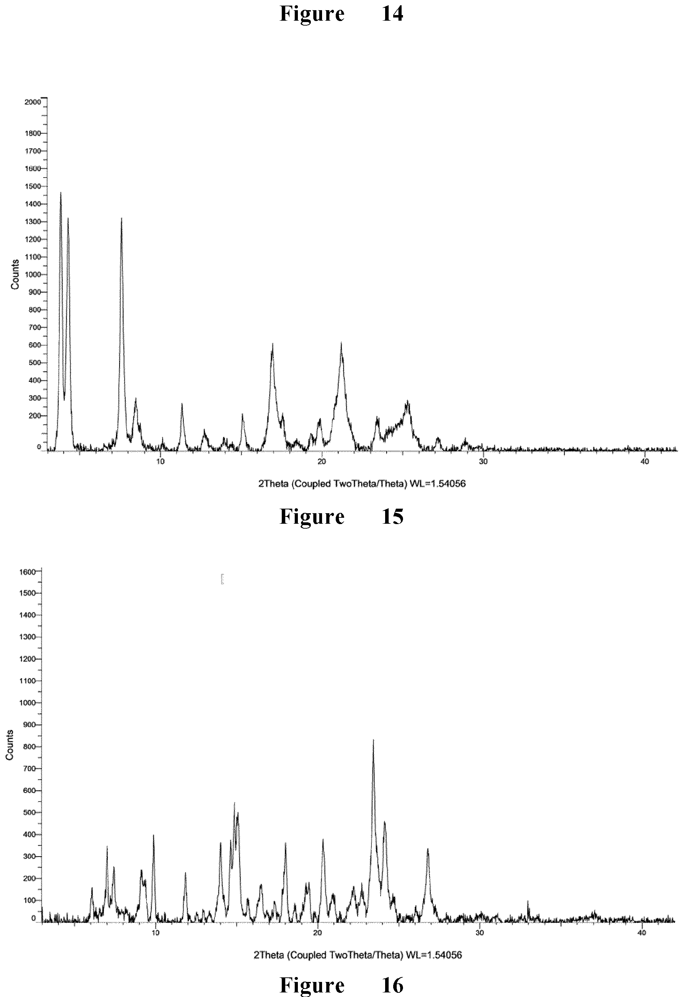

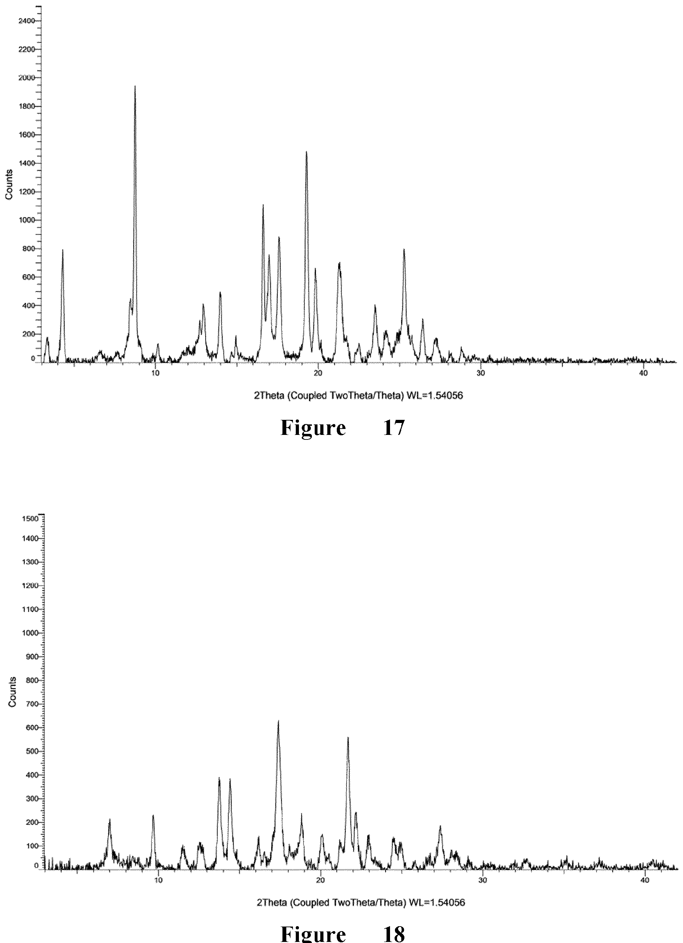

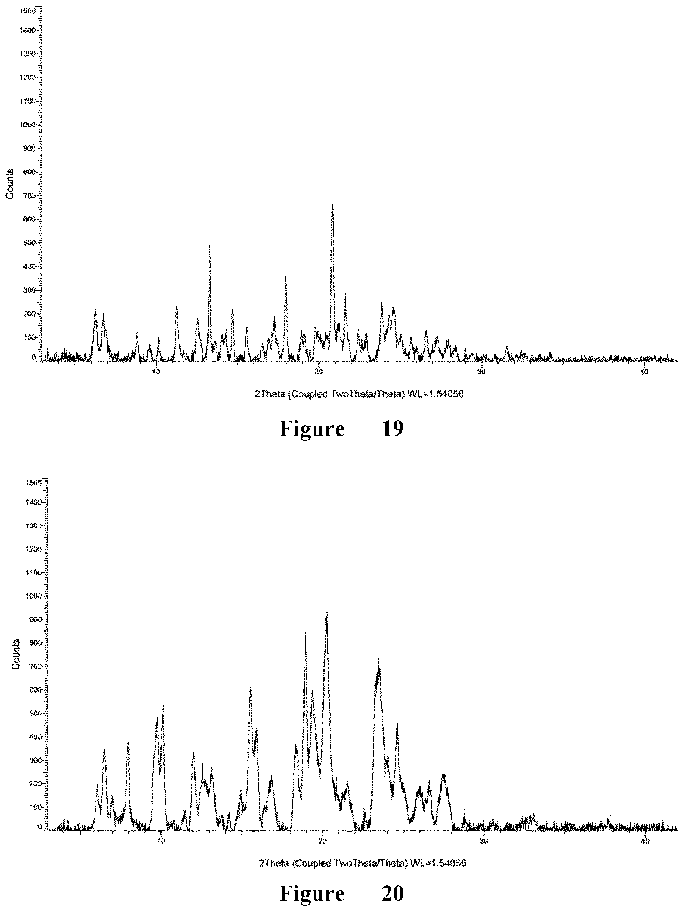

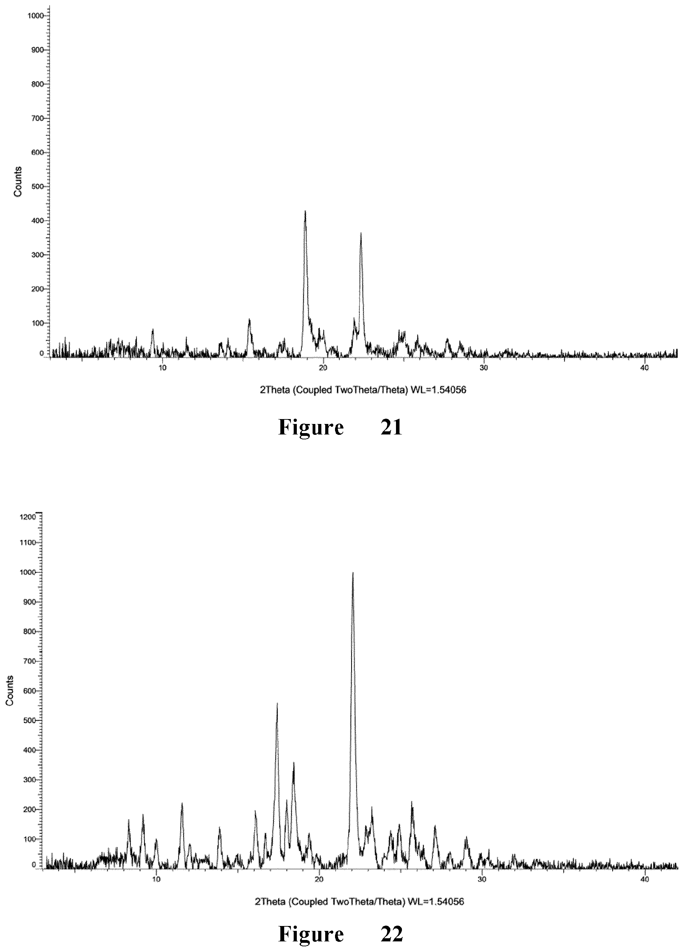

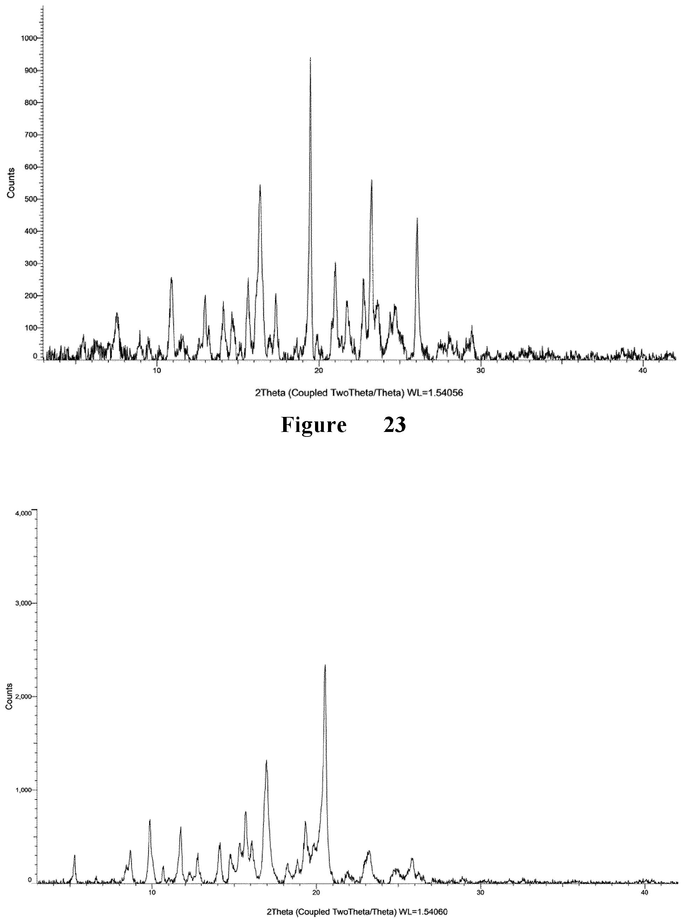

In another preferred embodiment,
(I-A) The crystal form is the salt crystal form I-A, and the salt crystal form I-A further comprises one or more of the following features:
   (1) The salt crystal form I-A has the XRPD data basically as shown in Table 1; and/or
   (2) The salt crystal form I-A has an XRPD pattern basically as shown in Figure 1;
(I-B) The crystal form is the salt crystal form I-B, and the salt crystal form I-B further comprises one or more of the following features:
   (1) The salt crystal form I-B has the XRPD data basically as shown in Table 2; and/or
   (2) The salt crystal form I-B has an XRPD pattern basically as shown in Figure 2;
(I-C) The crystal form is the salt crystal form I-C, and the salt crystal form I-C further comprises one or more of the following features:
   (1) The salt crystal form I-C has the XRPD data basically as shown in Table 3; and/or
   (2) The salt crystal form I-C has an XRPD pattern basically as shown in Figure 3;
(II-A) The crystal form is the salt crystal form II-A, and the salt crystal form II-A further comprises one or more of the following features:
   (1) The salt crystal form II-A has the XRPD data basically as shown in Table 4; and/or
   (2) The salt crystal form II-A has an XRPD pattern basically as shown in Figure 4;
(II-B) The crystal form is the salt crystal form II-B, and the salt crystal form II-B further comprises one or more of the following features:
   (1) The salt crystal form II-B has the XRPD data basically as shown in Table 5; and/or
   (2) The salt crystal form II-B has an XRPD pattern basically as shown in Figure 5;
(II-C) The crystal form is the salt crystal form II-C, and the salt crystal form II-C further comprises one or more of the following features:
   (1) The salt crystal form II-C has the XRPD data basically as shown in Table 6; and/or
   (2) The salt crystal form II-C has an XRPD pattern basically as shown in Figure 6;
(III-A) The crystal form is the salt crystal form III-A, and the salt crystal form III-A further comprises one or more of the following features:
   (1) The salt crystal form III-A has the XRPD data basically as shown in Table 7; and/or
   (2) The salt crystal form III-A has an XRPD pattern basically as shown in Figure 7;
(III-B) The crystal form is the salt crystal form II-B, and the salt crystal form III-B further comprises one or more of the following features:
   (1) The salt crystal form III-B has the XRPD data basically as shown in Table 8; and/or
   (2) The salt crystal form III-B has an XRPD pattern basically as shown in Figure 8;
(III-C) The crystal form is the salt crystal form II-C, and the salt crystal form III-C further comprises one or more of the following features:
   (1) The salt crystal form III-C has the XRPD data basically as shown in Table 9; and/or
   (2) The salt crystal form III-C has an XRPD pattern basically as shown in Figure 9;
(IV-A) The crystal form is the salt crystal form IV-A, and the salt crystal form IV-A further comprises one or more of the following features:
   (1) The salt crystal form IV-A has the XRPD data basically as shown in Table 10; and/or
   (2) The salt crystal form IV-A has an XRPD pattern basically as shown in Figure 10;
(V-A) The crystal form is the salt crystal form V-A, and the salt crystal form V-A further comprises one or more of the following features:
   (1) The salt crystal form V-A has the XRPD data basically as shown in Table 11; and/or
   (2) The salt crystal form V-A has an XRPD pattern basically as shown in Figure 11;
(V-B) The crystal form is the salt crystal form V-B, and the salt crystal form V-B further comprises one or more of the following features:
   (1) The salt crystal form V-B has the XRPD data basically as shown in Table 12; and/or
   (2) The salt crystal form V-B has an XRPD pattern basically as shown in Figure 12;
(VI-A) The crystal form is the salt crystal form VI-A, and the salt crystal form VI-A further comprises one or more of the following features:
   (1) The salt crystal VI-A has the XRPD data basically as shown in Table 13; and/or
   (2) The salt crystal VI-A has an XRPD pattern basically as shown in Figure 13;
(VII-A) The crystal form is the salt crystal form VII-A, and the salt crystal form VII-A further comprises one or more of the following features:
   (1) The salt crystal form VII-A has the XRPD data basically as shown in Table 14; and/or
   (2) The salt crystal form VII-A has an XRPD pattern basically as shown in Figure 14;
(VIII-A) The crystal form is the salt crystal form VIII-A, and the salt crystal form VIII-A further comprises one or more of the following features:
   (1) The salt crystal form VIII-A has the XRPD data basically as shown in Table 15; and/or
   (2) The salt crystal form VIII-A has an XRPD pattern basically as shown in Figure 15;
(VIII-B) The crystal form is the salt crystal form VIII-B, and the salt crystal form VIII-B further comprises one or more of the following features:
   (1) The salt crystal form VIII-B has the XRPD data basically as shown in Table 16; and/or
   (2) The salt crystal form VIII-B has an XRPD pattern basically as shown in Figure 16;
(VIII-C) The crystal form is the salt crystal form VIII-C, and the salt crystal form VIII-C further comprises one or more of the following features:
   (1) The salt crystal form VIII-C has the XRPD data basically as shown in Table 17; and/or
   (2) The salt crystal form VIII-C has an XRPD pattern basically as shown in Figure 17;
(IX-A) The crystal form is the salt crystal form IX-A, and the salt crystal form IX-A further comprises one or more of the following features:
   (1) The salt crystal form IX-A has the XRPD data basically as shown in Table 18; and/or
   (2) The salt crystal form IX-A has an XRPD pattern basically as shown in Figure 18;
(IX-B) The crystal form is the salt crystal form IX-B, and the salt crystal form IX-B further comprises one or more of the following features:
   (1) The salt crystal form IX-B has the XRPD data basically as shown in Table 19; and/or
   (2) The salt crystal form IX-B has an XRPD pattern basically as shown in Figure 19;
(IX-C) The crystal form is the salt crystal form IX-C, and the salt crystal form IX-C further comprises one or more of the following features:
   (1) The salt crystal form IX-C has the XRPD data basically as shown in Table 20; and/or
   (2) The salt crystal form IX-C has an XRPD pattern basically as shown in Figure 20;
(IX-D) The crystal form is the salt crystal form IX-D, and the salt crystal form IX-D further comprises one or more of the following features:
   (1) The salt crystal form IX-D has the XRPD data basically as shown in Table 21; and/or
   (2) The salt crystal form IX-D has an XRPD pattern basically as shown in Figure 21;
(IX-E) The crystal form is the salt crystal form IX-E, and the salt crystal form IX-E further comprises one or more of the following features:
   (1) The salt crystal form IX-E has the XRPD data basically as shown in Table 22; and/or
   (2) The salt crystal form IX-E has an XRPD pattern basically as shown in Figure 22;
(IX-F) The crystal form is the salt crystal form IX-F, and the salt crystal form IX-F further comprises one or more of the following features:
   (1) The salt crystal form IX-F has the XRPD data basically as shown in Table 23; and/or
   (2) The salt crystal form IX-F has an XRPD pattern basically as shown in Figure 23;

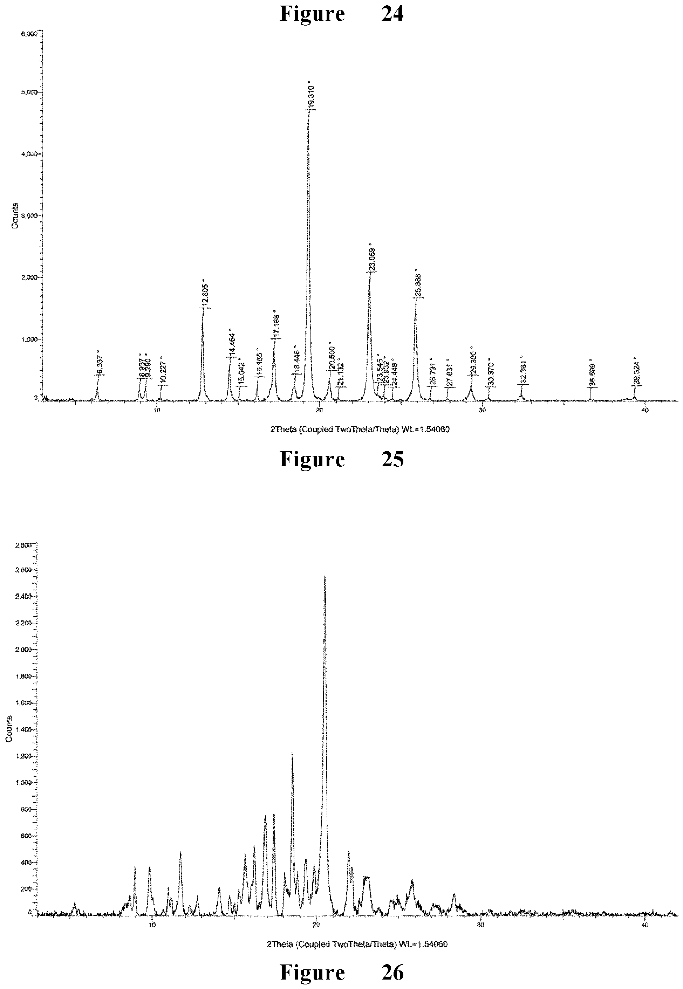

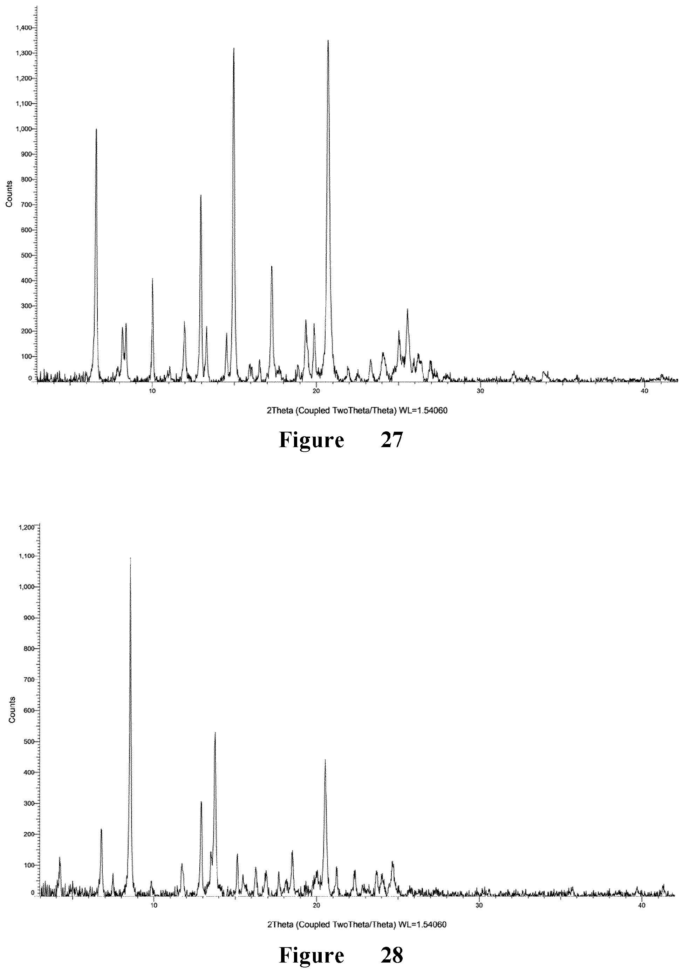

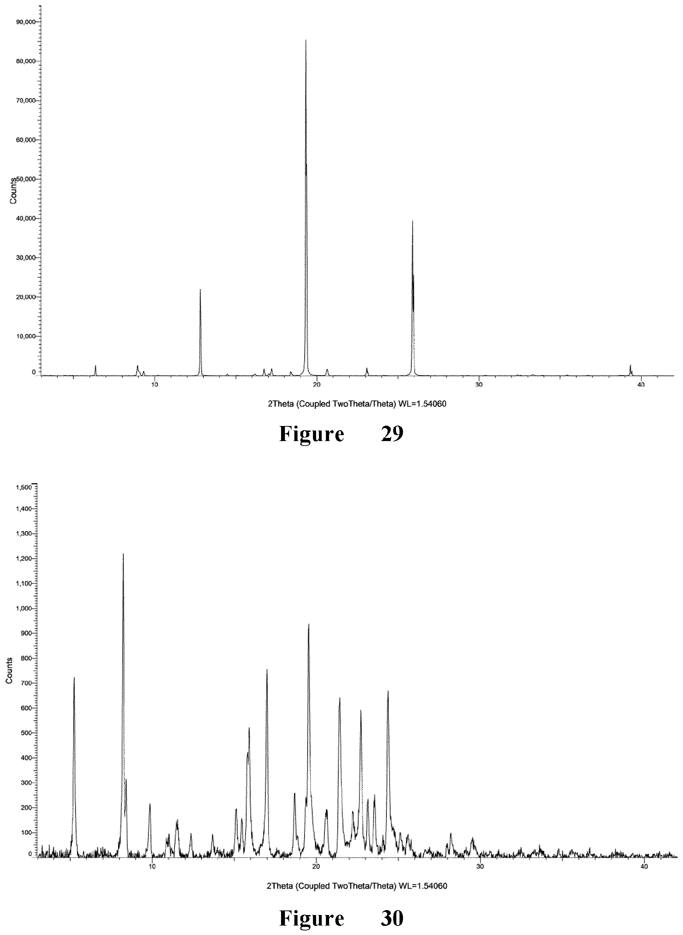

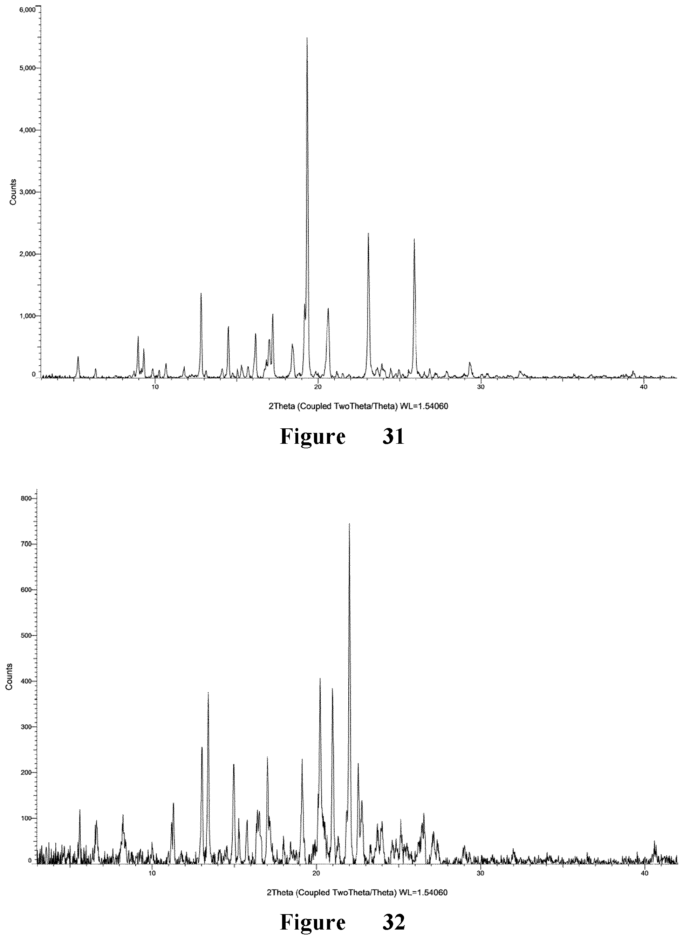

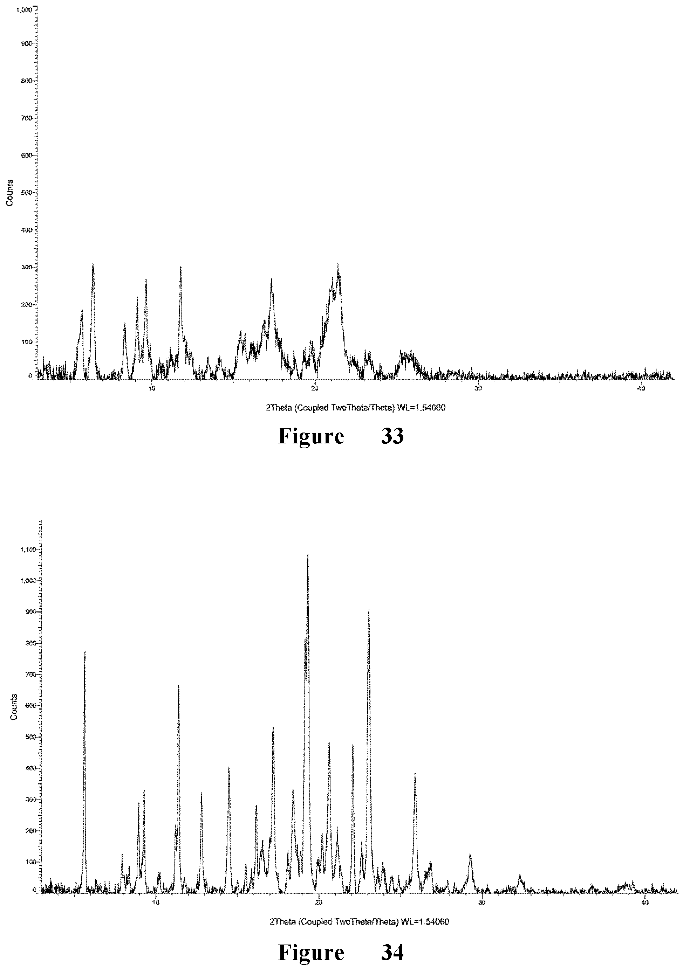

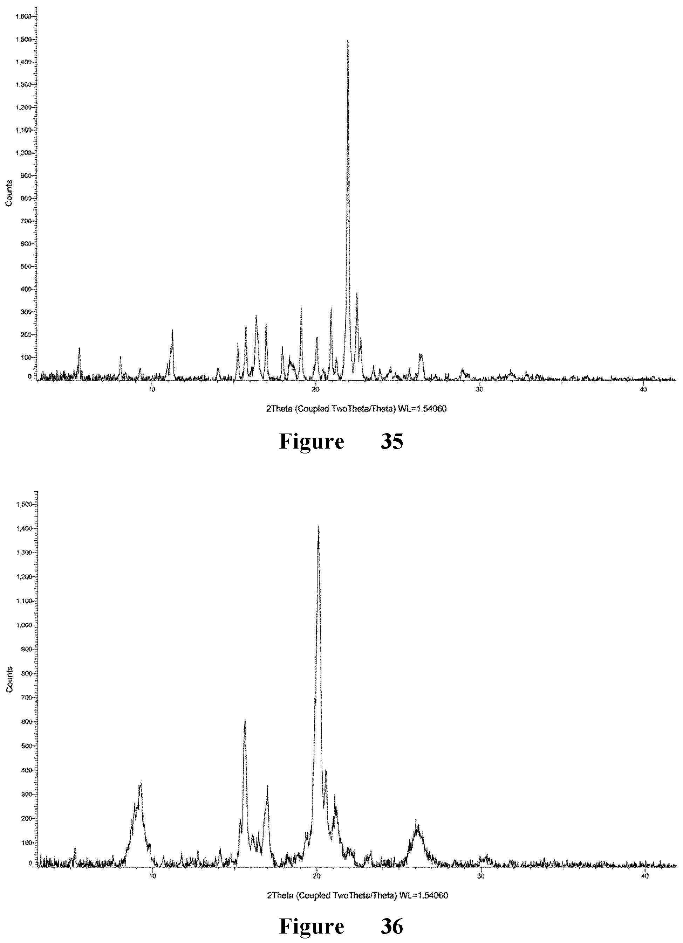

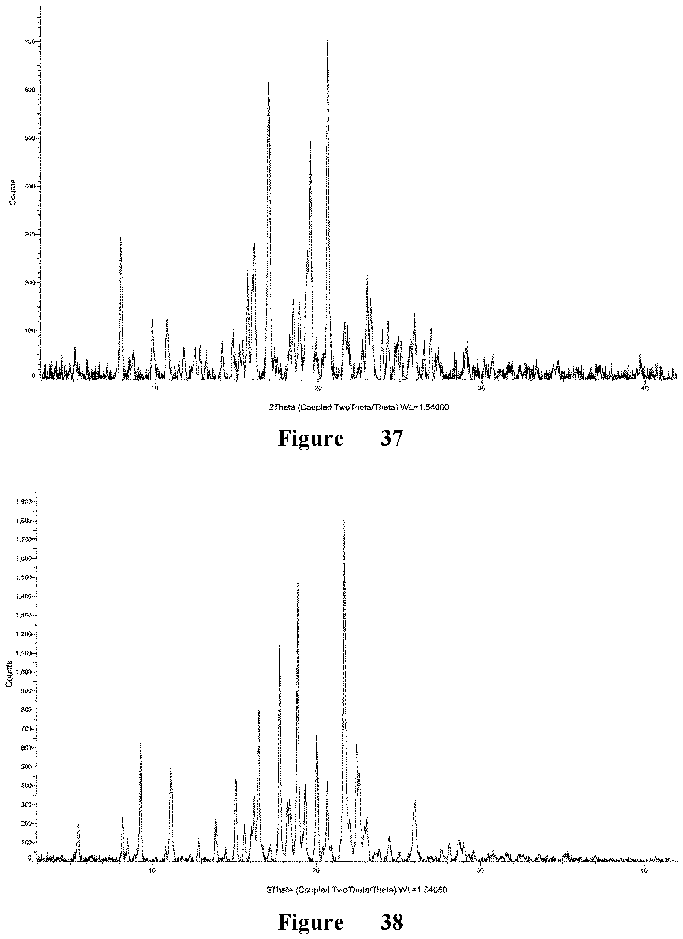

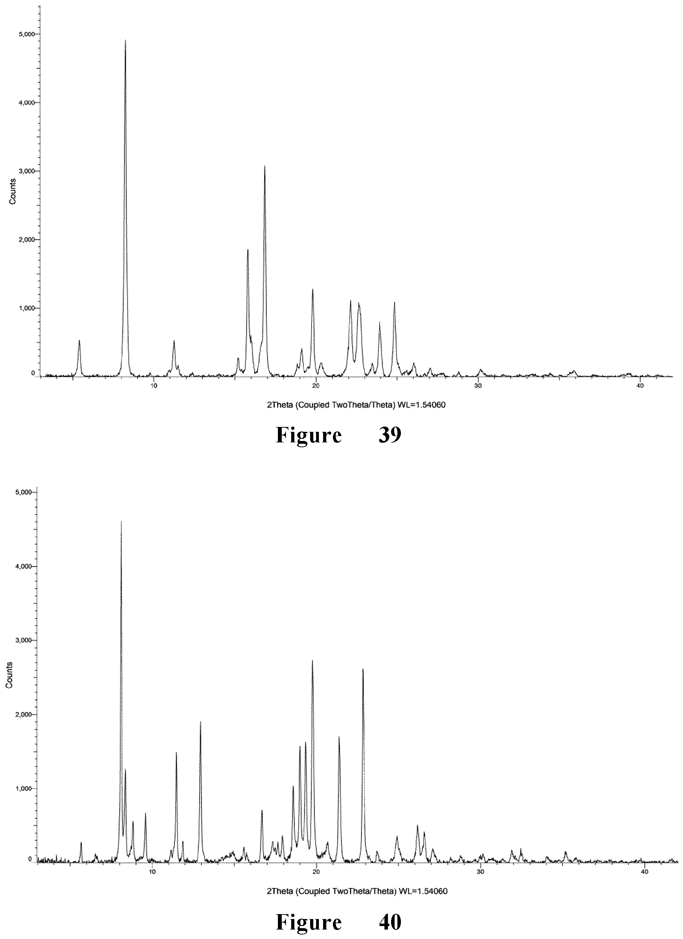

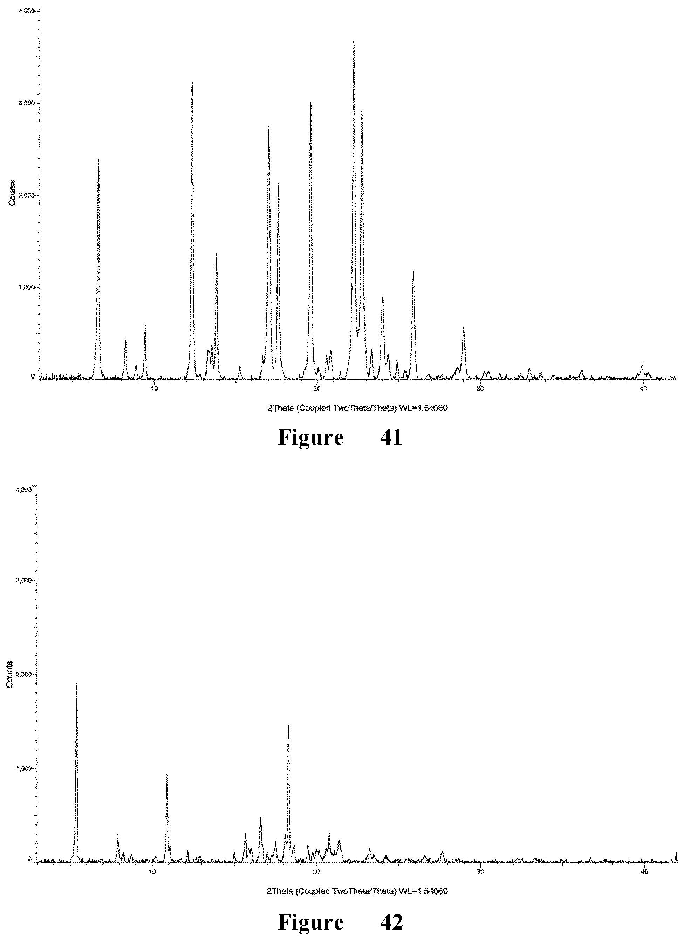

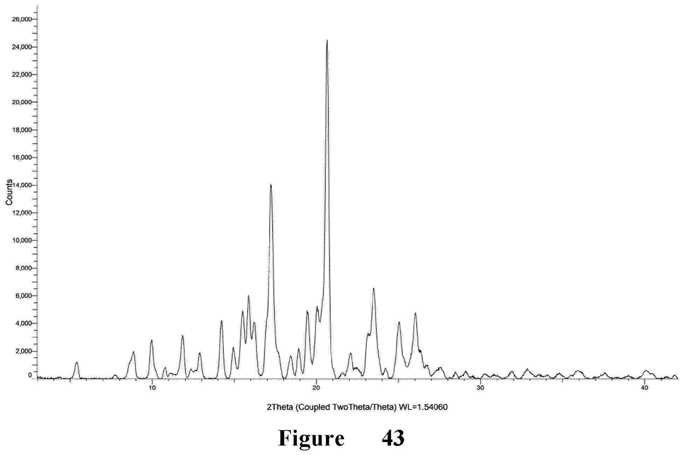

(A) The crystal form is the free base crystal form Form A, and the free base crystal form Form A further comprises one or more of the following features:
   (1) The free base crystal form Form A has the XRPD data basically as shown in Table 0; and/or
   (2) The free base crystal form Form A has an XRPD pattern basically as shown in Figure 24;
(B) The crystal form is the free base crystal form Form B, and the free base crystal form Form B further comprises one or more of the following features:
   (1) The free base crystal form Form B has the XRPD data basically as shown in Table 27; and/or
   (2) The free base crystal form Form B has an XRPD pattern basically as shown in Figure 25;
(C) The crystal form is the free base crystal form Form C, and the free base crystal form Form C further comprises one or more of the following features:
   (1) The free base crystal form Form C has the XRPD data basically as shown in Table 28; and/or
   (2) The free base crystal form Form C has an XRPD pattern basically as shown in Figure 26;
(D) The crystal form is the free base crystal form Form D, and the free base crystal form Form D further comprises one or more of the following features:
   (1) The free base crystal form Form D has the XRPD data basically as shown in Table 29; and/or
   (2) The free base crystal form Form D has an XRPD pattern basically as shown in Figure 27;
(E) The crystal form is the free base crystal form Form E, and the free base crystal form Form E further comprises one or more of the following features:
   (1) The free base crystal form Form E has the XRPD data basically as shown in Table 30; and/or
   (2) The free base crystal form Form E has an XRPD pattern basically as shown in Figure 28;
(F) The crystal form is the free base crystal form Form F, and the free base crystal form Form F further comprises one or more of the following features:
   (1) The free base crystal form Form F has the XRPD data basically as shown in Table 31; and/or
   (2) The free base crystal form Form F has an XRPD pattern basically as shown in Figure 29;
(G) The crystal form is the free base crystal form Form G, and the free base crystal form Form G further comprises one or more of the following features:
   (1) The free base crystal form Form G has the XRPD data basically as shown in Table 32; and/or
   (2) The free base crystal form Form G has an XRPD pattern basically as shown in Figure 30;
(H) The crystal form is the free base crystal form Form H, and the free base crystal form Form H further comprises one or more of the following features:
   (1) The free base crystal form Form H has the XRPD data basically as shown in Table 33; and/or
   (2) The free base crystal form Form H has an XRPD pattern basically as shown in Figure 31;
(I) The crystal form is the free base crystal form Form I, and the free base crystal form Form I further comprises one or more of the following features:
   (1) The free base crystal form Form I has the XRPD data basically as shown in Table 34; and/or
   (2) The free base crystal form Form I has an XRPD pattern as shown in Figure 32;
(J) The crystal form is the free base crystal form Form J, and the free base crystal form Form J further comprises one or more of the following features:
   (1) The free base crystal form Form J has the XRPD data basically as shown in Table 35; and/or
   (2) The free base crystal form Form J has an XRPD pattern basically as shown in Figure 33;
(K) The crystal form is the free base crystal form Form K, and the free base crystal form Form K further comprises one or more of the following features:
   (1) The free base crystal form Form K has the XRPD data basically as shown in Table 36; and/or
   (2) The free base crystal form Form K has an XRPD pattern basically as shown in Figure 34;
(L) The crystal form is the free base crystal form Form L, and the free base crystal form Form L further comprises one or more of the following features:
   (1) The free base crystal form Form L has the XRPD data basically as shown in Table 37; and/or
   (2) The free base crystal form Form L has an XRPD pattern basically as shown in Figure 35;
(M) The crystal form is the free base crystal form Form M, and the free base crystal form Form M further comprises one or more of the following features:
   (1) The free base crystal form Form M has the XRPD data basically as shown in Table 38; and/or
   (2) The free base crystal form Form M has an XRPD pattern basically as shown in Figure 36;
(N) The crystal form is the free base crystal form Form N, and the free base crystal form Form N further comprises one or more of the following features:
   (1) The free base crystal form Form N has the XRPD data basically as shown in Table 39; and/or
   (2) The free base crystal form Form N has an XRPD pattern basically as shown in Figure 37;
(O) The crystal form is the free base crystal form Form O, and the free base crystal form Form O further comprises one or more of the following features:
   (1) The free base crystal form Form O has the XRPD data basically as shown in Table 40; and/or
   (2) The free base crystal form Form O has an XRPD pattern basically as shown in Figure 38;
(P) The crystal form is the free base crystal form Form P, and the free base crystal form Form P further comprises one or more of the following features:
   (1) The free base crystal form Form P has the XRPD data basically as shown in Table 41; and/or
   (2) The free base crystal form Form P has an XRPD pattern basically as shown in Figure 39;
(Q) The crystal form is the free base crystal form Form Q, and the free base crystal form Form Q further comprises one or more of the following features:
   (1) The free base crystal form Form Q has the XRPD data basically as shown in Table 42; and/or
   (2) The free base crystal form Form Q has an XRPD pattern basically as shown in Figure 40;
(R) The crystal form is the free base crystal form Form R, and the free base crystal form Form R further comprises one or more of the following features:
   (1) The free base crystal form Form R has the XRPD data basically as shown in Table 43; and/or
   (2) The free base crystal form Form R has an XRPD pattern basically as shown in Figure 41;
(S) The crystal form is the free base crystal form Form S, and the free base crystal form Form S further comprises one or more of the following features:
   (1) The free base crystal form Form S has the XRPD data basically as shown in Table 44; and/or
   (2) The free base crystal form Form S has an XRPD pattern basically as shown in Figure 42;
(T) The crystal form is the free base crystal form Form T, and the free base crystal form Form T further comprises one or more of the following features:
   (1) The free base crystal form Form T has the XRPD data basically as shown in Table 45; and/or
   (2) The free base crystal form Form T has an XRPD pattern basically as shown in Figure 43.

In the fourth aspect of the present invention, provided is a method for preparing the crystal form described in the first aspect of the present invention, wherein the crystal form is Form T, and the method comprises steps of:
(i) dissolving the free base Form A in the fifth solvent, adding with trifluoroacetic acid and filtering;
(ii) keeping the filtrate at 35-45 °C for 1-2h, and then adjusting the filtrate to pH 7-8;
(iii) keeping the filtrate 35-45 °C for 1-3h, continuously adding dropwise with alkaline solution, and after dropwise addition, maintaining the temperature for 1-2 h;
(v) slowly cooling the filtrate down to 0-10 °C, maintaining the temperature and stirring for 1-3 h to obtain the free base crystal form Form T.

In another preferred embodiment, the fifth solvent is water.

In another preferred embodiment, in step (ii), the pH adjustment is carried out using an alkaline solution, and the alkaline solution is a sodium carbonate solution.

In the fifth aspect of the present invention, provided is a method for preparing the crystal form according to the second aspect of the present invention, wherein the crystal form is a free base crystal form B, and the method comprises any one of steps (a), (b), (c), (d) and (e):
**(a) Suspension crystallization method:** dissolving the free base Form A in the first solvent to form a suspension, then leaving the suspension at 25 ° C-50 °C, optionally conducting suspension stirring, and then subjecting to filtration to obtain the free base crystal form Form B;

In another preferred embodiment, in the case that suspension stirring is not conducted, performing filtration after leaving the suspension for 6-8 days.

In another preferred embodiment, in the case that suspension stirring is conducted, performing filtration after conducting suspension stirring for 2-4 days.
**(b) Gas-liquid diffusion:** Dissolving the free base Form A in a liquid-phase vial containing the first good solvent, and then keeping the liquid-phase vial open and placing it in a large flask containing the first anti-solvent, sealing the large flask, and leaving it at room temperature for one week to obtain the free base crystal form Form B;

In another preferred embodiment, in step (b), the concentration of Form A in the liquid-phase vial is 40-60mg/mL.
**(c) Gas solid diffusion:** Placing the free base Form A in a liquid-phase vial, and then keeping it open and placing in a large flask containing the third solvent, sealing the large flask, and leaving it at room temperature for one week to obtain the free base crystal form Form B;
**(d) Slow volatilization:** Placing the free base Form A in a liquid-phase vial, adding the fourth solvent to dissolve, and then slowly evaporating the solvent at room temperature (under N₂ protection) to obtain the free base crystal form Form B;

In another preferred embodiment, the fourth solvent is THF, MIBK, or a combination thereof.
**(e) Dissolution crystallization:** Placing the free base Form A in a vial and dissolving it with the second good solvent. Quickly adding the second anti-solvent, stirring overnight, and then subjecting to filtration to obtain the free base crystal form Form B.

In another preferred embodiment, the starting material compound of formula I is the free base crystal form Form A of the compound of formula I.

In another preferred embodiment, when the method adopts step (a), the first solvent is selected from the group consisting of: EtOH, IPA, MTBE, EA, heptane, IPAC, ethyl formate, anisole, MIBK, a mixed solvent of IPA and ACN, a mixed solvent of DMSO and H₂O, and a mixed solvent of EtOH and H₂O.

In another preferred embodiment, when the temperature is 25 °C, the first solvent is selected from the group consisting of: EtOH, IPA, MTBE, EA, IPAC, ethyl formate, MIBK, a mixed solvent of IPA and ACN, and a mixed solvent of DMSO and H₂O.

In another preferred embodiment, when the temperature is 50 °C, the first solvent is selected from the group consisting of: EtOH, MTBE, EA, heptane, IPAC, ethyl formate, MIBK, anisole, a mixed solvent of DMSO and H₂O, and a mixed solvent of EtOH and H₂O.

In another preferred embodiment, when the method adopts step (b), the first good solvent is NPA, and the first anti-solvent is selected from the group consisting of: ACN, acetone, EA, and a combination thereof;
when the method adopts step (c), the third solvent is ethyl formate;
when the method adopts step (d), the fourth solvent is THF, MIBK, or a combination thereof;
when the method adopts step (e), the second good solvent is selected from the group consisting of: THF, NPA, and a combination thereof; and the second anti-solvent is selected from the group consisting of: ACN, EA, IPAC, MTBE, n-heptane, and a combination thereof.

In the sixth aspect of the present invention, provided is a pharmaceutical composition, which comprises (a) a crystal form as described in the first, second, or third aspect of the present invention as the active ingredient; and (b) pharmaceutically acceptable carriers.

In another preferred embodiment, the dosage form of the pharmaceutical composition or formulation is selected from the group consisting of: powders, capsules, granules, tablets, pills and injections.

It should be understood that within the scope of the present invention, the above-mentioned technical features of the present invention and the specific technical features described below (as embodiments) can be combined with each other to form a new or preferred technical solution. Due to space limitations, it will not be repeated here.

### BRIEF DESCRIPTION OF THE DRAWINGS

Figure 1 shows the XRPD pattern of the salt crystal form I-A of the present invention;
Figure 2 shows the XRPD pattern of the salt crystal form I-B of the present invention;
Figure 3 shows the XRPD pattern of the salt crystal form I-C of the present invention;
Figure 4 shows the XRPD pattern of the salt crystal form II-A of the present invention;
Figure 5 shows the XRPD pattern of the salt crystal form II-B of the present invention;
Figure 6 shows the XRPD pattern of the salt crystal form II-C of the present invention;
Figure 7 shows the XRPD pattern of the salt crystal form III-A of the present invention;
Figure 8 shows the XRPD pattern of the salt crystal form III-B of the present invention;
Figure 9 shows the XRPD pattern of the salt crystal form III-C of the present invention;
Figure 10 shows the XRPD pattern of the salt crystal form IV-A of the present invention;
Figure 11 shows the XRPD pattern of the salt crystal form V-A of the present invention;
Figure 12 shows the XRPD pattern of the salt crystal form V-B of the present invention;
Figure 13 shows the XRPD pattern of the salt crystal form VI-A of the present invention;
Figure 14 shows the XRPD pattern of the salt crystal form VII-A of the present invention;
Figure 15 shows the XRPD pattern of the salt crystal form VIII-A of the present invention;
Figure 16 shows the XRPD pattern of the salt crystal form VIII-B of the present invention;
Figure 17 shows the XRPD pattern of the salt crystal form VIII-C of the present invention;
Figure 18 shows the XRPD pattern of the salt crystal form IX-A of the present invention;
Figure 19 shows the XRPD pattern of the salt crystal form IX-B of the present invention;
Figure 20 shows the XRPD pattern of the salt crystal form IX-C of the present invention;
Figure 21 shows the XRPD pattern of the salt crystal form IX-D of the present invention;
Figure 22 shows the XRPD pattern of the salt crystal form IX-E of the present invention;
Figure 23 shows the XRPD pattern of the salt crystal form IX-F of the present invention;
Figure 24 shows the XRPD pattern of the free base crystal form Form A of the present invention;
Figure 25 shows the XRPD pattern of the free base crystal form Form B of the present invention;
Figure 26 shows the XRPD pattern of the free base crystal form Form C of the present invention;
Figure 27 shows the XRPD pattern of the free base crystal form Form D of the present invention;
Figure 28 shows the XRPD pattern of the free base crystal form Form E of the present invention;
Figure 29 shows the XRPD pattern of the free base crystal form Form F of the present invention;
Figure 30 shows the XRPD pattern of the free base crystal form Form G of the present invention;
Figure 31 shows the XRPD pattern of the free base crystal form Form H of the present invention;
Figure 32 shows the XRPD pattern of the free base crystal form Form I of the present invention;
Figure 33 shows the XRPD pattern of the free base crystal form Form J of the present invention;
Figure 34 shows the XRPD pattern of the free base crystal form Form K of the present invention;
Figure 35 shows the XRPD pattern of the free base crystal form Form L of the present invention;
Figure 36 shows the XRPD pattern of the free base crystal form Form M of the present invention;
Figure 37 shows the XRPD pattern of the free base crystal form Form N of the present invention;
Figure 38 shows the XRPD pattern of the free base crystal form Form O of the present invention;
Figure 39 shows the XRPD pattern of the free base crystal form Form P of the present invention;
Figure 40 shows the XRPD pattern of the free base crystal form Form Q of the present invention;
Figure 41 shows the XRPD pattern of the free base crystal form Form R of the present invention;
Figure 42 shows the XRPD pattern of the free base crystal form Form S of the present invention;
Figure 43 shows the XRPD pattern of the free base crystal form Form T of the present invention.

### DETAILED DESCRIPTION

After long-term and in-depth research, the inventor provides salt crystal forms and free base crystal forms of the compound of the formula I. These crystal forms have advantages in stability, solubility, hygroscopicity, mechanical stability, tabletting stability, fluidity, process development, formulation developmentability, and powder processing performance. Especially, crystal forms B and T have significant advantages in preparation process and stability. Based on the above findings, the inventor completed the present invention.

### Term

In this article, unless otherwise specified, all abbreviations have common meanings understood by those skilled in the art.

As used herein, unless otherwise specified, the way for adding solvent or solution is adding by directly pouring or adding at a constant rate.

As used herein, the term "room temperature" generally refers to 4-30 °C, preferably 20 ± 5 °C.

As used herein, the way of "slow addition" includes but is not limited to: adding dropwise, slowly adding along the container wall, etc.

### Compound of formula I

As used herein, the term "compound of formula I" refers to a compound as shown in the following formula (i.e., 3-ethyl-8- (4-(4-(4-ethylpiperazin-1-yl) piperidin-1-yl) -3-methoxyphenyl) - N2- (tetrahydro-2H-pyran-4-yl) pyridino [3,4-b] pyrazine-2,5-diamine):

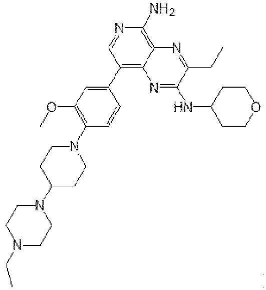

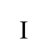

The above compound was firstly reported in PCT/CN2021/107759 and can be prepared using the method described in this literature. In this article, unless otherwise specified, the term "compound of formula I raw material" refers to the free base crystal form Form A of the compound of formula I, and the XRPD data thereof is shown in Table 0 and the XRPD pattern is shown in Figure 24. This crystal form is a free base synthesized and separated according to the preparation method of PCT/CN2021/107759. In the examples, Form A and API are interchangeable.

**Table 0**

| 2θ | Intensity |
|---|---|
| 20.536 | 100.00% |
| 16.957 | 55.50% |
| 15.689 | 31.50% |
| 19.34 | 28.40% |
| 9.858 | 25.40% |
| 11.738 | 24.60% |
| 15.33 | 17.60% |
| 16.072 | 17.60% |
| 19.858 | 16.50% |
| 14.126 | 16.10% |
| 8.676 | 15.00% |
| 23.15 | 13.00% |
| 12.77 | 12.60% |
| 14.805 | 12.30% |
| 25.859 | 10.80% |
| 18.855 | 10.20% |
| 5.29 | 10.10% |
| 18.263 | 9.40% |
| 8.445 | 7.90% |
| 10.682 | 6.50% |
| 21.863 | 6.30% |
| 26.213 | 6.00% |
| 24.796 | 5.70% |
| 12.323 | 5.00% |
| 28.899 | 3.50% |
| 26.538 | 3.40% |
| 31.755 | 2.50% |
| 7.602 | 1.60% |
| 32.581 | 1.60% |

### Pharmaceutical composition containing crystal forms of the compound of formula I

Another aspect of the present invention provides a pharmaceutical composition comprising a therapeutically effective amount of a crystal form of the compound of formula I as described in the present invention, and optionally, one or more pharmaceutically acceptable carriers, excipients, adjuvants, excipients, and/or diluents. The excipients include flavors, fragrances, sweeteners, etc.

The pharmaceutical composition provided by the present invention preferably contains active ingredients in a weight ratio of 1-99%, the preferred ratio is that the compound of formula I as active ingredient accounts for 65wt% to 99wt% of the total weights, and the remaining portion is pharmaceutically acceptable carriers, diluents, or solutions or salt solutions. The compound and pharmaceutical composition provided by the present invention can be in various forms, such as tablets, capsules, powders, syrups, solutions, suspensions, and aerosols, and can exist in suitable solid or liquid carriers or dilutions and in suitable disinfectants for injection or infusion.

The various dosage forms of the pharmaceutical composition of the present invention can be prepared according to conventional preparation methods in the field of pharmacy. The unit dose of its formulation includes 1 mg to 700 mg of the compound of formula I, and preferably, the unit dose of the formulation includes 25 mg to 300 mg of the compound of formula I.

The compound and pharmaceutical composition of the present invention can be clinically used in mammals, including humans and animals, and can be administered through route of administration such as oral, nasal, skin, lung, or gastrointestinal routes. The most preferred route is oral administration. The optimal daily dosage is 50-1400 mg/kg body weight, taken in a single dose, or 25-700 mg/kg body weight in multiple doses. Regardless of the method of administration, the optimal dosage for individuals should be determined based on specific treatment. It is usual to start with a small dose and gradually increase the dose until the most suitable dose is found.

In the present invention, unless otherwise specified, the method used for drying is a conventional drying method in the art. For example, in embodiments of the present invention, drying refers to vacuum drying or ambient pressure drying using a conventional drying oven. Generally, drying 0.1-50 hours or 1-30 hours.

The present invention is further described below in conjunction with specific embodiments. It should be understood that these embodiments are only used to illustrate the present invention and not to limit the scope of the present invention. The experimental methods in the following embodiments that do not specify specific conditions are usually based on conventional conditions or conditions recommended by the manufacturer. Unless otherwise specified, percentages and portions are calculated by weight.

Unless otherwise defined, all professional and scientific terms used herein have the same meanings as commonly understood by those skilled in the art. In addition, any methods and materials similar or equivalent to those described can be applied to the method of the present invention. The preferred embodiments and materials described herein are for exemplary purposes only.

### General methods and reagents

The solvents used in this invention are all analytically pure, with a water content of approximately 0.1%. The compound of formula (I) used as raw materials in the examples are all purchased.

All testing methods of the present invention are common methods, and the testing parameters are as follows:
X-ray powder diffraction instrument measurement method: An X-ray powder diffraction instrument is used to analyze the crystal form of the raw materials. The samples are scanned at 2θ angles from 3° to 42° with a scanning step size of 0.02° and a scanning time of 0.2 s per step. The voltage and current of the light tube are 40 kV and 40 mA, respectively. During sample preparation, an appropriate amount of sample is placed on the sample tray and flattened using tools such as spoons or glass sheets, ensuring a smooth and even surface.

TGA pattern determination method: TA Instruments TGA Discovery 550 is used to analyze the sample. The sample is placed in an aluminum plate with tare weight removed, and the system will automatically weigh it. Then, under the protection of nitrogen, the sample is heated to 300 °C at a rate of 10 °C/min.

DSC pattern determination method: TA Instruments Discovery DSC 25 is used to analyze the sample. The weighed sample is placed on a sample tray and heated to 300 °C at a rate of 10 ° C/min under the protection of nitrogen gas (50ml/min).

Nuclear magnetic resonance hydrogen spectrum data (1H NMR): The instrument used for 1H NMR analysis is the Bruker Ascend 400MH NMR analyzer.

DSV pattern determination method: Dynamic moisture adsorption was performed using Intrinsic DVS. In step mode, the sample is subjected to loop testing at a relative humidity of 0-90%. The sample is analyzed at 10% RH. The dm/dt is set to 0.002 to achieve equilibrium between the sample and the room environment.

### Example 1: Preparation of p-toluenesulfonate crystal forms I-A, I-B, and I-C

At 0 °C, approximately 25 mg of API was weighed and added to ethanol (0.5 mL), followed by the addition of 1.2 eq of p-toluenesulfonic acid. The mixture was reacted at 50 °C for 3 hours under magnetic stirring, and then cooled down to room temperature. After reacting overnight, the mixture was filtered to obtain phosphate crystal form I-A;

At 0 °C, approximately 25 mg of API was weighed and added to THF (0.5 mL), followed by the addition of 1.2 eq of p-toluenesulfonic acid. The mixture was reacted at 50 °C for 3 hours under magnetic stirring, and then cooled down to room temperature. After reacting overnight, the reaction was filtered to obtain phosphate crystal form I-B;

At 0 °C, approximately 25 mg of API was weighed and added to ethyl formate (0.5 mL) (DCM: MeOH (1:1) (0.5 mL)), followed by the addition of 1.2 eq of p-toluenesulfonic acid. The mixture was reacted at 50 °C for 3 hours under magnetic stirring, and then cooled down to room temperature. After reacting overnight, the mixture was filtered to obtain phosphate crystal form I-C;

Various characterizations were performed on the crystal forms respectively. From the analysis results, it can be seen that there are chemical shift deviations in the nuclear magnetic spectra, indicating that the free bases have undergone salt formation reaction. The XRPD data are shown in Tables 1-3, respectively, and from the DSC results, it can be seen that the obtained p-toluenesulfonate salts have two or more endothermic peaks, which may be a mixed crystal.

**Table 1**

| 2θ | Intensity |
|---|---|
| 22.558 | 100.00% |
| 6.153 | 88.60% |
| 6.365 | 77.30% |
| 11.62 | 69.00% |
| 23.392 | 52.60% |
| 24.186 | 47.60% |
| 19.235 | 42.30% |
| 17.135 | 39.90% |
| 16.848 | 34.20% |
| 12.786 | 34.00% |
| 24.743 | 17.00% |
| 17.992 | 14.10% |
| 18.56 | 13.60% |
| 12.375 | 8.90% |
| 5.584 | 6.80% |
| 10.978 | 6.20% |
| 21.829 | 5.60% |
| 28.451 | 4.90% |
| 21.034 | 3.50% |
| 19.832 | 2.90% |

**Table 2**

| 2θ | Intensity |
|---|---|
| 6.349 | 100.00% |
| 18.611 | 99.40% |
| 20.111 | 83.70% |
| 19.354 | 83.50% |
| 19.659 | 61.20% |
| 24.741 | 57.80% |
| 13.702 | 57.70% |
| 23.493 | 48.50% |
| 12.931 | 45.70% |
| 18.012 | 44.70% |
| 23.206 | 40.70% |
| 12.482 | 40.20% |
| 27.48 | 37.50% |
| 23.009 | 37.30% |
| 24.5 | 36.00% |
| 14.085 | 35.40% |
| 12.002 | 35.20% |
| 26.106 | 33.70% |
| 20.379 | 33.10% |
| 10.064 | 31.10% |
| 22.71 | 30.40% |
| 25.653 | 23.10% |
| 24.151 | 22.20% |
| 8.967 | 20.60% |
| 11.06 | 19.80% |
| 6.56 | 18.00% |
| 17.675 | 16.80% |
| 20.886 | 15.40% |
| 15.52 | 12.00% |
| 9.39 | 11.20% |
| 30.381 | 10.40% |
| 28.579 | 9.30% |
| 5.902 | 9.20% |
| 29.863 | 8.10% |
| 22.001 | 7.10% |
| 21.463 | 6.40% |
| 16.3 | 5.80% |
| 26.746 | 5.10% |
| 32.347 | 3.50% |
| 35.995 | 3.00% |

**Table 3**

| 2θ | Intensity |
|---|---|
| 6.156 | 100.00% |
| 19.228 | 22.00% |
| 6.348 | 20.00% |
| 18.607 | 14.30% |
| 24.203 | 11.30% |
| 12.443 | 10.60% |
| 22.553 | 10.50% |
| 11.588 | 9.30% |
| 12.764 | 7.90% |
| 23.357 | 7.70% |
| 10.93 | 5.30% |
| 14.126 | 5.10% |
| 16.8 | 4.90% |
| 20.219 | 4.80% |
| 18.114 | 4.40% |
| 9.22 | 4.00% |
| 17.146 | 3.00% |
| 10.033 | 2.90% |
| 21.797 | 2.80% |
| 26.531 | 2.60% |
| 20.676 | 2.30% |
| 16.128 | 1.70% |
| 21.078 | 1.50% |
| 15.292 | 1.30% |
| 37.716 | 1.30% |

### Example 2: Preparation of fumarate crystal forms II-A, II-B, and II-C

At 0 °C, approximately 25 mg of API was weighed and added to ethanol (0.5 mL), followed by the addition of 1.2 eq of fumaric acid. The mixture was reacted at 50 °C for 3 hours under magnetic stirring, and then cooled down to room temperature. After reacting overnight, the mixture was filtered to obtain phosphate crystal form II-A;

At 0 °C, approximately 25 mg of API was weighed and added to THF (0.5 mL), followed by the addition of 1.2 eq of fumaric acid. The mixture was reacted at 50 °C for 3 hours under magnetic stirring, and then cooled down to room temperature. After reacting overnight, the mixture was filtered to obtain phosphate crystal form II-B;

At 0 °C, approximately 25 mg of API was weighed and added to 0.5 mL of ethyl formate, followed by the addition of 1.2 eq of fumaric acid. The mixture was reacted at 50 °C for 3 hours under magnetic stirring, and then cooled down to room temperature. After reacting overnight, the mixture was filtered to obtain phosphate crystal form II-C;

Various characterizations were performed on the crystal forms respectively. From the analysis results, it can be seen that there are chemical shift deviations in the nuclear magnetic spectra, indicating that the free bases have undergone salt formation reaction. The XRPD data are shown in Tables 4-6. According to the DSC results, the fumarate crystal form II-A has two endothermic peaks, which may be a mixed crystal. According to the DSC&TGA results of fumarate crystal form II-C, its melting point is 204.09 °C, corresponding weight loss is 6.826%. According to the DSC results, the XRPD of fumarate crystal form II-C was tested after heating to 150 °C. The results showed that the crystal form changed after solvent removal treatment, indicating that fumarate crystal form II-C may be a solvate or hydrate.

**Table 4**

| 2θ | Intensity |
|---|---|
| 7.068 | 100.00% |
| 22.629 | 31.10% |
| 21.298 | 30.60% |
| 18.108 | 23.10% |
| 19.312 | 20.60% |
| 20.504 | 20.50% |
| 6.404 | 13.60% |
| 22.168 | 13.40% |
| 24.809 | 10.50% |
| 8.079 | 9.50% |
| 12.049 | 8.50% |
| 7.599 | 8.40% |
| 14.156 | 7.90% |
| 28.701 | 7.00% |
| 19.947 | 6.10% |
| 25.28 | 6.00% |
| 8.973 | 5.60% |
| 16.494 | 4.60% |
| 12.411 | 4.40% |
| 11.643 | 4.10% |
| 27.741 | 3.30% |
| 12.804 | 2.40% |
| 32.311 | 2.30% |
| 26.183 | 2.10% |

**Table 5**

| 2θ | Intensity |
|---|---|
| 22.639 | 100.00% |
| 8.057 | 76.50% |
| 12.303 | 30.70% |
| 5.666 | 24.90% |
| 18.977 | 19.60% |
| 20.904 | 15.60% |
| 9.02 | 15.00% |
| 14.963 | 12.90% |
| 16.106 | 9.10% |
| 18.159 | 7.40% |
| 4.093 | 7.10% |

**Table 6**

| 2θ | Intensity |
|---|---|
| 22.482 | 100.00% |
| 9.03 | 69.70% |
| 7.668 | 51.80% |
| 18.149 | 42.40% |
| 7.162 | 37.80% |
| 11.704 | 34.70% |
| 16.564 | 32.30% |
| 8.665 | 27.70% |
| 19.939 | 26.00% |
| 27.791 | 21.60% |
| 20.587 | 19.30% |
| 12.603 | 16.20% |
| 15.285 | 14.30% |
| 16.911 | 14.30% |
| 19.336 | 14.30% |
| 18.563 | 14.10% |
| 26.158 | 13.80% |
| 9.711 | 13.30% |
| 25.703 | 11.30% |
| 23.442 | 10.50% |
| 14.203 | 8.30% |
| 24.9 | 7.00% |
| 28.92 | 4.90% |
| 31.336 | 4.40% |
| 13.62 | 3.60% |

### Example 3: Preparation of methanesulfonate crystal forms III-A, III-B, and III-C

At 0 °C, approximately 25 mg of API was weighed and added to THF (0.5 mL), followed by the addition of 1.2 eq of methanesulfonic acid. The mixture was reacted at 50 °C for 3 hours under magnetic stirring, and then cooled down to room temperature. After reacting overnight, the mixture was filtered to obtain the methanesulfonate crystal form III-A;
At 0 °C, approximately 25 mg of API was weighed and added to ethyl formate (0.5 mL), followed by the addition of 1.2 eq of methanesulfonic acid. The mixture was reacted at 50 °C for 3 hours under magnetic stirring, and then cooled down to room temperature. After reacting overnight, the mixture was filtered to obtain the methanesulfonate crystal form III-B;
At 0 °C, approximately 25 mg of API was weighed and added to ethanol (0.5 mL), followed by the addition of 2.2 eq of methanesulfonic acid. The mixture was reacted at 50 °C for 3 hours under magnetic stirring, and then cooled down to room temperature. After reacting overnight, the mixture was filtered to obtain the methanesulfonate crystal form III-C;
Various characterizations were performed on the crystal forms respectively, and the results are shown in the following figures. From the analysis results, it can be seen that there are chemical shift deviations in the nuclear magnetic spectra, indicating that the free bases have undergone salt formation reaction. The XRPD data are shown in Tables 7-9, respectively. From the DSC results, it can be seen that the methanesulfonate both crystal forms III-B and III-C have two endothermic peaks, indicating that they are mixed crystals. According to the DSC results, the XRPD of the methanesulfonate crystal form III-A is tested after heating to 150 °C. The results showed that the crystal form did not change after solvent removal treatment, indicating that the methanesulfonate crystal form III-A is anhydrous.

**Table 7**

| 2θ | Intensity |
|---|---|
| 9.199 | 100.00% |
| 4.609 | 47.40% |
| 23.106 | 18.90% |
| 18.031 | 14.20% |
| 13.798 | 11.60% |
| 16.97 | 7.60% |
| 18.427 | 7.10% |
| 24.243 | 6.70% |
| 20.545 | 5.60% |
| 20.124 | 5.30% |
| 21.289 | 5.30% |
| 14.368 | 5.20% |
| 25.954 | 4.60% |
| 21.976 | 4.30% |
| 30.329 | 4.00% |
| 28.621 | 3.60% |
| 10.253 | 3.50% |
| 27.03 | 3.50% |
| 32.555 | 3.20% |
| 16.261 | 3.10% |
| 30.07 | 3.00% |
| 28.953 | 2.00% |
| 19.523 | 1.50% |
| 15.063 | 1.30% |
| 27.784 | 1.20% |
| 31.627 | 1.20% |
| 41.318 | 1.10% |

**Table 8**

| 2θ | Intensity |
|---|---|
| 16.693 | 100.00% |
| 22.437 | 35.40% |
| 22.187 | 31.90% |
| 14.045 | 28.50% |
| 8.936 | 28.30% |
| 20.243 | 25.60% |
| 23.628 | 24.70% |
| 24.494 | 22.10% |
| 19.827 | 17.30% |
| 18.705 | 17.00% |
| 23.096 | 16.70% |
| 21.151 | 13.30% |
| 29.191 | 12.60% |
| 11.507 | 11.50% |
| 10.076 | 10.70% |
| 7.053 | 9.80% |
| 30.258 | 9.80% |
| 17.86 | 8.90% |
| 25.239 | 8.80% |
| 4.442 | 8.40% |
| 21.562 | 8.20% |
| 15.847 | 6.70% |
| 7.435 | 6.60% |
| 13.474 | 6.50% |
| 26.342 | 5.90% |
| 19.309 | 4.80% |
| 28.778 | 4.00% |
| 27.626 | 3.10% |
| 38.331 | 3.00% |

**Table 9**

| 2θ | Intensity |
|---|---|
| 19.533 | 100.00% |
| 24.465 | 51.80% |
| 17.496 | 22.70% |
| 9.057 | 21.20% |
| 9.74 | 20.00% |
| 21.36 | 16.20% |
| 22.78 | 13.90% |
| 15.371 | 12.60% |
| 28.275 | 8.10% |
| 26.74 | 7.40% |
| 31.615 | 6.20% |
| 29.332 | 6.00% |
| 4.507 | 5.40% |
| 22.485 | 5.40% |
| 17.935 | 5.20% |
| 16.759 | 4.70% |
| 18.25 | 4.30% |
| 18.746 | 4.30% |
| 25.926 | 4.30% |
| 12.932 | 4.10% |
| 10.125 | 3.90% |
| 15.807 | 3.80% |
| 23.727 | 3.80% |
| 34.512 | 2.60% |
| 4.866 | 2.40% |
| 20.415 | 2.40% |
| 14.639 | 2.00% |
| 39.286 | 1.70% |
| 11.223 | 1.10% |
| 30.33 | 1.00% |
| 35.619 | 1.00% |

### Example 4: Preparation of phosphate crystal form IV-A

At 0 °C, approximately 25 mg of API was weighed and added to methyl formate (0.5 mL), followed by the addition of 1.2 eq of phosphoric acid. The mixture was reacted at 50 °C for 3 hours under magnetic stirring, and then cooled down to room temperature. After reacting overnight, the mixture was filtered to obtain phosphate crystal form IV-A.

Various characterizations were performed on the crystal form. From the analysis results, there are chemical shift deviations in the nuclear magnetic spectrum, indicating that the free bases have undergone salt formation reaction. The XRPD data are shown in Table 10, and according to the DSC&TGA results, the melting point of phosphate crystal form IV-A is 184.94 °C, with a weight loss of 3.361% before 150 °C. According to the DSC results, the phosphate crystal form IV-A was heated to 150 °C and its XRPD was tested. The results showed that the crystal form did not change after solvent removal treatment, indicating that the phosphate crystal form IV-A is anhydrous.

**Table 10**

| 2θ | Intensity |
|---|---|
| 21.594 | 100.00% |
| 12.749 | 47.20% |
| 23.759 | 45.70% |
| 19.944 | 42.80% |
| 27.501 | 32.30% |
| 22.271 | 30.30% |
| 24.681 | 29.50% |
| 20.389 | 25.80% |
| 7.866 | 23.20% |
| 18.339 | 23.10% |
| 25.842 | 20.40% |
| 9.546 | 18.10% |
| 19.184 | 17.20% |
| 16.168 | 14.80% |
| 32.892 | 14.80% |
| 17.538 | 14.20% |
| 18.625 | 12.80% |
| 21.014 | 10.50% |
| 30.816 | 10.50% |
| 17.09 | 10.00% |
| 23.137 | 10.00% |
| 30.574 | 7.20% |

### Example 5: Preparation of maleate crystal Forms V-A and V-B

At 0 °C, approximately 25 mg of API was weighed and added to 0.5 mL of ethyl formate, followed by the addition of 1.2 eq of maleic acid. The mixture was reacted at 50 °C for 3 hours under magnetic stirring, and then cooled down to room temperature. After reacting overnight, the mixture was filtered to obtain salt crystal form V-A;
At 0 °C, approximately 25 mg of API was weighed and added to DCM: MeOH (1:1) (0.5 mL), followed by the addition of 1.2 eq of maleic acid. The mixture was reacted at 50 °C for 3 hours under magnetic stirring, and then cooled down to room temperature. After reacting overnight, the mixture was filtered to obtain salt crystal form V-B;
Various characterizations were performed on the crystal forms respectively. From the analysis results, it can be seen that there are chemical shift deviations in the nuclear magnetic spectra, indicating that the free bases have undergone salt formation reaction. The XRPD data are shown in Tables 11-12.

**Table 11**

| 2θ | Intensity |
|---|---|
| 19.511 | 100.00% |
| 8.538 | 87.30% |
| 26.084 | 36.50% |
| 12.999 | 30.60% |
| 23.284 | 29.50% |
| 17.104 | 18.70% |
| 4.31 | 13.40% |
| 17.374 | 12.30% |
| 14.939 | 11.10% |
| 21.4 | 6.60% |
| 29.51 | 5.70% |
| 6.319 | 4.20% |
| 10.728 | 4.10% |
| 6.524 | 3.90% |
| 30.089 | 2.60% |
| 27.924 | 2.50% |
| 18.693 | 2.40% |
| 7.084 | 2.30% |
| 32.535 | 2.30% |
| 25.051 | 2.20% |
| 14.103 | 1.80% |
| 20.847 | 1.80% |
| 20.158 | 1.70% |
| 39.538 | 1.60% |
| 30.432 | 0.90% |

**Table 12**

| 2θ | Intensity |
|---|---|
| 22.48 | 100.00% |
| 18.654 | 42.30% |
| 17.31 | 30.10% |
| 10.05 | 27.10% |
| 19.992 | 23.40% |
| 11.711 | 19.50% |
| 18.356 | 17.90% |
| 15.916 | 16.90% |
| 9.279 | 15.80% |
| 26.763 | 15.00% |
| 8.67 | 14.80% |
| 13.883 | 13.70% |
| 8.292 | 13.20% |
| 12.729 | 10.80% |
| 26.159 | 9.60% |
| 20.643 | 9.00% |
| 14.695 | 8.20% |
| 21.578 | 7.80% |
| 23.761 | 7.60% |
| 32.047 | 7.30% |
| 25.1 | 6.80% |
| 4.659 | 6.30% |
| 15.563 | 6.00% |
| 29.532 | 4.20% |

### Example 6: Preparation of citrate crystal VI-A

At 0 °C, approximately 25 mg of API was weighed and added to ethanol (0.5 mL), followed by the addition of 1.2 eq of citric acid. The mixture was reacted at 50 °C for 3 hours under magnetic stirring, and then cooled down to room temperature. After reacting overnight, the mixture was filtered to obtain citrate crystal form VI-A;
The citrate crystal form VI-A was subjected to various characterizations. From the analysis results, there are chemical shift deviations in the nuclear magnetic spectrum, indicating that the free base has undergone salt formation reaction. The XRPD data are shown in Table 13, and from the DSC results, it can be inferred that the peak melting point of the citrate crystal form VI-A is 191.36 °C. According to the DSC results, the XRPD of citrate crystal form VI-A was tested after heating to 150 °C. The results showed that the crystal form changed to amorphous after solvent removal treatment, indicating that citrate crystal form VI-A may be a solvate or hydrate.

**Table 13**

| 2θ | Intensity |
|---|---|
| 17.502 | 100.00% |
| 8.712 | 87.30% |
| 17.012 | 66.50% |
| 7.854 | 66.30% |
| 20.072 | 64.60% |
| 8.39 | 50.60% |
| 14.974 | 46.30% |
| 16.728 | 35.60% |
| 4.667 | 34.40% |
| 4.884 | 33.60% |
| 8.937 | 29.20% |
| 25.416 | 28.60% |
| 14.393 | 26.50% |
| 10.9 | 17.60% |
| 22.593 | 17.60% |
| 18.275 | 17.50% |
| 9.346 | 14.60% |
| 26.405 | 11.80% |
| 18.928 | 11.70% |
| 26.006 | 11.30% |
| 22.013 | 10.50% |

### Example 7: Preparation of malate crystal form VII-A

At 0 °C, approximately 25 mg of API was weighed and added to THF (0.5 mL), followed by the addition of 1.2 eq of malic acid. The mixture was reacted at 50 °C for 3 hours under magnetic stirring, and then cooled down to room temperature. After reacting overnight, the mixture was filtered to obtain the malate crystal form VII-A;
The malate crystal form was was subjected to various characterizations, and the results were shown in the following figure. From the analysis results, there are chemical shift deviations in the nuclear magnetic spectrum, indicating that the free base has undergone salt formation reaction. The XRPD data are shown in Table 14. According to the DSC&DSC results, the peak melting point of malate crystal form VII-A is 183.96 °C, and the weight loss before 150 °C is 1.954%. According to the DSC results, the XRPD of malate crystal form VII-A was tested after heating to 150 °C. The results showed that the crystal form did not change after solvent removal treatment, indicating that malate crystal form VII-A should be anhydrous.

**Table 14**

| 2θ | Intensity |
|---|---|
| 22.102 | 100.00% |
| 17.149 | 70.00% |
| 14.246 | 45.80% |
| 19.119 | 32.50% |
| 22.613 | 26.70% |
| 11.58 | 22.10% |
| 14.468 | 21.70% |
| 20.398 | 21.00% |
| 13.667 | 18.00% |
| 25.16 | 17.50% |
| 18.032 | 15.60% |
| 21.293 | 15.30% |
| 20.145 | 13.60% |
| 14.952 | 13.10% |
| 12.439 | 11.30% |
| 13.387 | 8.70% |
| 23.299 | 8.10% |
| 25.584 | 7.90% |
| 9.577 | 6.70% |
| 21.067 | 6.70% |
| 26.73 | 6.30% |
| 27.7 | 6.10% |
| 18.511 | 5.60% |
| 7.514 | 5.50% |
| 28.217 | 5.40% |
| 16.773 | 5.30% |
| 19.824 | 4.50% |
| 33.282 | 4.00% |
| 39.237 | 3.60% |
| 15.864 | 2.80% |
| 34.927 | 2.80% |
| 37.048 | 2.50% |
| 30.018 | 2.40% |
| 29.501 | 2.30% |

### Example 8: Preparation of salicylate crystal forms VIII-A, VIII-B, and VIII-C

At 0 °C, approximately 25 mg of API was weighed and added to ethanol (0.5 mL), followed by the addition of 1.2 eq of salicylic acid. The mixture was reacted at 50 °C for 3 hours under magnetic stirring, and then cooled down to room temperature. After reacting overnight, the mixture was filtered to obtain salicylate crystal form VIII-A;
At 0 °C, approximately 25 mg of API was weighed and added to THF (0.5 mL), followed by the addition of 1.2 eq of salicylic acid. The mixture was reacted at 50 °C for 3 hours under magnetic stirring, and then cooled down to room temperature. After reacting overnight, the mixture was filtered to obtain salicylate crystal form VIII-B;
At 0 °C, approximately 25 mg of API was weighed and added to DCM: MeOH (0.5 mL), followed by the addition of 1.2 eq of salicylic acid. The mixture was reacted at 50 °C for 3 hours under magnetic stirring, and then cooled down to room temperature. After reacting overnight, the mixture was filtered to obtain salicylate crystal form VIII-C;
The crystal forms were subjected to various characterizations respectively. The XRPD data are shown in Tables 15-17, and from the analysis results, there are chemical shift deviations in all the nuclear magnetic spectrums, indicating that the free base has undergone salt formation reaction.

**Table 15**

| 2θ | Intensity |
|---|---|
| 3.834 | 100.00% |
| 4.282 | 90.10% |
| 7.593 | 90.00% |
| 21.198 | 42.30% |
| 16.946 | 41.60% |
| 25.302 | 19.40% |
| 8.451 | 18.80% |
| 11.334 | 18.40% |
| 17.526 | 13.60% |
| 19.847 | 11.70% |
| 15.123 | 11.60% |
| 23.412 | 11.10% |
| 24.234 | 7.60% |
| 12.73 | 7.30% |
| 19.367 | 5.60% |
| 27.174 | 5.40% |
| 10.128 | 4.90% |
| 13.942 | 4.70% |
| 28.858 | 3.00% |
| 14.369 | 2.50% |

**Table 16**

| 2θ | Intensity |
|---|---|
| 23.438 | 100.00% |
| 14.857 | 62.30% |
| 15.021 | 54.50% |
| 24.138 | 54.00% |
| 20.337 | 45.10% |
| 9.887 | 43.90% |
| 14.008 | 43.90% |
| 18.024 | 43.70% |
| 14.638 | 42.80% |
| 7.013 | 41.10% |
| 26.786 | 40.40% |
| 7.43 | 30.20% |
| 11.851 | 27.20% |
| 9.133 | 24.80% |
| 19.462 | 22.00% |
| 9.364 | 21.70% |
| 22.721 | 21.30% |
| 16.517 | 21.00% |
| 22.23 | 19.70% |
| 6.089 | 19.00% |
| 20.958 | 16.00% |
| 24.636 | 15.90% |
| 17.356 | 11.00% |
| 15.702 | 9.90% |
| 26.053 | 9.00% |
| 18.609 | 8.30% |
| 33.042 | 7.00% |
| 12.943 | 6.00% |
| 13.338 | 5.00% |
| 19.81 | 4.60% |
| 12.564 | 4.20% |

**Table 17**

| 2θ | Intensity |
|---|---|
| 8.752 | 100.00% |
| 19.298 | 76.20% |
| 16.631 | 55.90% |
| 17.601 | 45.40% |
| 25.289 | 41.10% |
| 4.296 | 40.30% |
| 16.981 | 38.80% |
| 21.325 | 35.40% |
| 19.842 | 34.10% |
| 13.995 | 25.00% |
| 8.468 | 23.10% |
| 12.953 | 20.40% |
| 23.516 | 18.60% |
| 26.44 | 15.50% |
| 12.733 | 15.00% |
| 24.186 | 11.20% |
| 21.759 | 9.90% |
| 14.942 | 9.60% |
| 3.343 | 8.80% |
| 27.226 | 8.50% |
| 22.526 | 6.90% |
| 10.151 | 6.60% |
| 28.815 | 4.40% |
| 11.717 | 4.20% |
| 7.66 | 4.10% |
| 6.606 | 4.00% |
| 14.69 | 3.40% |
| 28.114 | 2.40% |
| 10.855 | 2.20% |

### Example 9: Preparation of hydrochloride crystal forms IX-A, IX-B, IX-C, IX-D, IX-E, and IX-F

At 0 °C, approximately 25 mg of API was weighed and added to ethanol (0.5mL), followed by the addition of 1.2 eq of hydrochloric acid. The mixture was reacted at 50 °C for 3 hours under magnetic stirring, and then cooled down to room temperature. After reacting overnight, the mixture was filtered to obtain the hydrochloride crystal form IX-A;
At 0 °C, approximately 25 mg of API was weighed and added to tetrahydrofuran (0.5mL), followed by the addition of 1.2 eq of hydrochloric acid. The mixture was reacted at 50 °C for 3 hours under magnetic stirring, and then cooled down to room temperature. After reacting overnight, the mixture was filtered to obtain the hydrochloride crystal form IX-B;
At 0 °C, approximately 25 mg of API was weighed and added to 0.5 mL of ethyl formate, followed by the addition of 1.2 eq of hydrochloric acid. The mixture was reacted at 50 °C for 3 hours under magnetic stirring, and then cooled down to room temperature. After reacting overnight, the mixture was filtered to obtain the hydrochloride crystal form IX-C;
At 0 °C, approximately 25 mg of API was weighed and added to DCM: MeOH (1:1) (0.5mL), followed by the addition of 1.2 eq of hydrochloric acid. The mixture was reacted at 50 °C for 3 hours under magnetic stirring, and then cooled down to room temperature. After reacting overnight, the mixture was filtered to obtain the hydrochloride crystal form IX-D;
At 0 °C, approximately 25 mg of API was weighed and added to tetrahydrofuran (1:1) (0.5mL), followed by the addition of 2.2 eq of hydrochloric acid. The mixture was reacted at 50 °C for 3 hours under magnetic stirring, and then cooled down to room temperature. After reacting overnight, the mixture was filtered to obtainn the hydrochloride crystal form IX-E;
At 0 °C, approximately 25 mg of API was weighed and added to tetrahydrofuran (1:1) (0.5mL), followed by the addition of 4.0 eq of hydrochloric acid. The mixture was reacted at 50 °C for 3 hours under magnetic stirring, and then cooled down to room temperature. After reacting overnight, the mixture was filtered to obtainn the hydrochloride crystal form IX-F;
The crystal forms were subjected to various characterizations. From the analysis results, it can be seen that there are chemical shift deviations in the nuclear magnetic spectra, indicating that the free bases have undergone salt formation reaction. The XRPD data are shown in Tables 18-23.

From the DSC results, it can be seen that the hydrochloride crystal forms IX-A, IX-B, and IX-D all have two endothermic peaks, which may be mixed crystals. According to the DSC&TGA results of the hydrochloride crystal form IX-C and hydrochloride crystal form IX-E, the melting point of hydrochloride crystal form IX-C is 270.82 °C, and the weight loss before 230 °C is 7.826%. The melting point of the hydrochloride crystal form IX-E is 289.17 °C, and the weight loss before 230 °C is 8.507%. According to DSC results, the XRPD of the hydrochloride crystal form IX-E was tested after heating to 250 °C and the hydrochloride crystal form IX-F was tested after heating to 200 °C. The results showed that the hydrochloride crystal form IX-E and hydrochloride crystal form IX-F both changed after solvent removal treatment, indicating that the hydrochloride crystal form IX-E and hydrochloride crystal form IX-F may be solvates or hydrates.

**Table 18**

| 2θ | Intensity |
|---|---|
| 17.395 | 100.00% |
| 21.697 | 88.80% |
| 14.43 | 60.90% |
| 13.765 | 57.40% |
| 22.166 | 37.80% |
| 18.824 | 37.20% |
| 9.694 | 36.60% |
| 7.013 | 33.80% |
| 27.369 | 29.10% |
| 20.086 | 22.90% |
| 22.964 | 22.30% |
| 16.176 | 19.90% |
| 24.522 | 19.00% |
| 12.558 | 18.20% |
| 21.268 | 17.20% |
| 24.93 | 16.60% |
| 11.528 | 13.40% |
| 18.08 | 13.40% |
| 28.083 | 13.10% |
| 16.532 | 12.00% |
| 28.307 | 11.80% |
| 20.459 | 7.30% |
| 40.496 | 7.00% |
| 25.827 | 6.00% |
| 35.1 | 5.50% |
| 32.649 | 4.80% |

**Table 19**

| 2θ | Intensity |
|---|---|
| 20.835 | 100.00% |
| 13.293 | 72.60% |
| 17.98 | 49.10% |
| 23.877 | 37.90% |
| 21.622 | 37.80% |
| 11.265 | 35.00% |
| 24.592 | 34.00% |
| 14.698 | 31.90% |
| 6.767 | 30.70% |
| 6.283 | 30.20% |
| 24.307 | 29.10% |
| 12.566 | 27.10% |
| 17.252 | 23.20% |
| 21.169 | 21.50% |
| 22.434 | 19.90% |
| 15.562 | 18.50% |
| 8.826 | 17.90% |
| 19.852 | 17.50% |
| 26.598 | 17.40% |
| 25.069 | 17.30% |
| 22.904 | 17.20% |
| 14.075 | 15.90% |
| 14.285 | 15.60% |
| 20.468 | 15.60% |
| 25.677 | 15.50% |
| 27.967 | 13.80% |
| 10.209 | 13.50% |
| 16.935 | 13.40% |
| 27.314 | 13.40% |
| 13.651 | 12.60% |
| 19.027 | 11.70% |
| 9.608 | 11.10% |
| 16.506 | 10.80% |
| 31.547 | 8.20% |
| 26.026 | 8.00% |
| 28.35 | 7.00% |
| 29.367 | 5.60% |
| 34.221 | 3.50% |

**Table 20**

| 2θ | Intensity |
|---|---|
| 20.232 | 100.00% |
| 18.948 | 95.20% |
| 23.486 | 81.90% |
| 15.549 | 68.40% |
| 19.362 | 65.70% |
| 10.123 | 59.80% |
| 9.772 | 55.20% |
| 24.632 | 51.90% |
| 15.856 | 43.80% |
| 7.962 | 43.20% |
| 18.366 | 41.90% |
| 6.476 | 39.20% |
| 12.023 | 37.80% |
| 24.033 | 36.60% |
| 13.145 | 31.20% |
| 27.497 | 27.50% |
| 16.832 | 26.70% |
| 26.629 | 23.40% |
| 12.631 | 23.20% |
| 21.536 | 22.30% |
| 6.052 | 21.90% |
| 24.943 | 20.90% |
| 14.943 | 20.00% |
| 25.899 | 19.40% |
| 6.984 | 16.40% |
| 16.419 | 12.50% |
| 14.218 | 9.00% |
| 22.661 | 8.70% |
| 13.743 | 7.60% |
| 11.453 | 6.90% |
| 28.746 | 6.10% |

**Table 21**

| 2θ | Intensity |
|---|---|
| 18.88 | 100.00% |
| 22.356 | 85.20% |
| 15.398 | 26.10% |
| 21.954 | 24.20% |
| 9.411 | 19.50% |
| 19.966 | 13.50% |
| 14.067 | 13.20% |
| 24.809 | 12.90% |
| 25.866 | 12.40% |
| 8.338 | 11.40% |
| 17.595 | 11.00% |
| 28.502 | 10.90% |
| 27.737 | 10.70% |
| 13.596 | 8.70% |
| 11.512 | 8.00% |

**Table 22**

| 2θ | Intensity |
|---|---|
| 22.072 | 100.00% |
| 17.415 | 55.00% |
| 18.438 | 34.60% |
| 18.002 | 23.20% |
| 11.596 | 21.40% |
| 23.237 | 20.90% |
| 25.721 | 20.50% |
| 16.1 | 18.60% |
| 9.191 | 18.40% |
| 8.322 | 15.20% |
| 24.92 | 15.20% |
| 27.119 | 14.50% |
| 13.894 | 13.20% |
| 19.383 | 12.10% |
| 22.862 | 11.80% |
| 24.401 | 11.20% |
| 29.059 | 10.50% |
| 16.686 | 10.30% |
| 9.99 | 9.70% |
| 12.063 | 8.30% |
| 26.392 | 7.90% |
| 28.021 | 5.50% |
| 15.79 | 5.40% |
| 12.411 | 5.30% |
| 32.014 | 4.80% |
| 13.014 | 4.60% |
| 14.366 | 4.50% |
| 14.94 | 4.40% |
| 29.918 | 4.30% |

**Table 23**

| 2θ | Intensity |
|---|---|
| 19.477 | 100.00% |
| 23.233 | 58.50% |
| 16.372 | 57.70% |
| 26.063 | 45.40% |
| 21.02 | 33.00% |
| 22.74 | 27.20% |
| 10.901 | 26.90% |
| 15.633 | 26.60% |
| 17.35 | 21.50% |
| 12.98 | 21.30% |
| 21.762 | 19.50% |
| 23.625 | 19.50% |
| 14.101 | 19.10% |
| 24.702 | 18.20% |
| 24.398 | 16.20% |
| 7.543 | 15.80% |
| 13.187 | 11.40% |
| 14.686 | 11.00% |
| 29.425 | 9.20% |
| 8.95 | 9.10% |
| 16.965 | 7.50% |
| 5.441 | 7.30% |
| 19.89 | 7.10% |
| 9.46 | 7.00% |
| 28.127 | 6.60% |
| 27.504 | 5.60% |
| 29.188 | 5.60% |
| 18.622 | 5.50% |
| 11.528 | 5.30% |
| 28.477 | 4.20% |
| 12.672 | 3.90% |
| 15.141 | 3.80% |
| 39.475 | 3.80% |
| 32.522 | 3.10% |

### Example 10: Repetitive preparation of advantaged crystal forms

Repetitive preparations of salt crystal forms using phosphoric acid, malic acid, and methanesulfonic acid as ligands were performed.

Approximately 30 mg of API was weighed and added to the solvent, then an appropriate amount of ligand was weighed and added. The molar ratio of API to ligand was 1:1.2, and this reaction process was carried out at 0 °C. Th mixture was reacted at 50 °C for 3 hours under magnetic stirring, and then cooled down to room temperature. After reacting overnight and filtration, the obtained solid sample was analyzed.

**Table 24**

| No. | ligand | solvent | Solvent volume (ml) | phenomenon | result |
|---|---|---|---|---|---|
| 1 | phosphoric acid | Ethyl formate | 0.5 | Yellow turbidity | The crystal form is inconsistent with phosphate crystal form IV-A |
| 2 | malic acid | THF | 0.5 | Dark yellow turbidity | The crystal form is consistent with malate crystal form VII-A |
| 3 | Methane sulfonic acid | EtOH | 0.5 | Yellow turbidity | The crystal form is inconsistent with methanesulfonate crystal form III-A |

The malate crystal form VII-A can be stably prepared, and the DSC&TGA characterization results showed that its melting point is high, around 170 °C. Therefore, the scale-up of the malate crystal form VII-A and subsequent solubility and stability evaluation on them were carried out.

### 10.1. Scaled-up Preparation of malate crystal form VII-A

Approximately 400 mg of free base A was weighed and added into a round bottom flask and added with 8 ml of ethyl formate. 1.2 equivalents of malic acid was weighed and added at 0 °C. The mixture was reacted at 50 °C for 3 hours under magnetic stirring, and then cooled down to room temperature. After reacting overnight and filtration, the resulting solid sample was dried overnight at 50 °C and then subjected to various characterizations. According to the characterization results, the malate crystal form VII-A can be stably obtained.

### 10.2. Stability

A certain amount of API and malate crystal form VII-A were weighed and added into a liquid phase vial, and a total of 9 parallels were prepared. The 9 parallels were placed separately in 80 °C (open); 25 °C, 60% RH (open); 40 °C, 75% RH (open); and light stability box closed). The stabilities under conditions of: 80 °C for one day, light exposure for 10 days, as well as 25 °C, 60% RH and 40 °C, 75% RH for one and two weeks were tested, and the XRPD of the solids were measured. Under the condition of light exposure and 40 °C, 75% RH, both free base Form A and malate crystal form VII-A undergo varying degrees of degradation.

**Table 25**

| condition | | Free base Form A | | | Malate crystal form VII-A | | |
|---|---|---|---|---|---|---|---|
| | | Purity (%) | Degradati on (%) | Cryst al form | Purity (%) | Degradati on (%) | Crystal form |
| initial | | 95.53 | N/A | N/A | 95.84 | N/A | N/A |
| 80°C | 1 day | 95.39 | 0.14 | chang ed | 95.22 | 0.62 | Uncha nged |
| Light exposure | 10 day s | 88.14 | 7.89 | chang ed | 89.17 | 6.67 | Uncha nged |
| Light central | 10 day s | 95.51 | 0.02 | Unch anged | 95.79 | 0.05 | Uncha nged |
| 25°C, 60%RH | 1 wee k | 95.50 | 0.03 | Unch anged | 95.81 | 0.03 | Uncha nged |
| | 2 wee ks | 95.49 | 0.04 | Unch anged | 95.52 | 0.32 | Uncha nged |
| 40°C,75%R H | 1 wee k | 95.49 | 0.04 | Unch anged | 95.71 | 0.13 | Uncha nged |
| | 2 wee ks | 92.60 | 2.93 | Unch anged | 94.78 | 1.06 | Uncha nged |

### 10.3. Solubility

Approximately 10 mg of malate crystal form VII-A and free base Form A were weighed separately, and 1 ml of buffer solutions with different pH were added to make the solution supersaturated, and the mixtures were stirred at 37 °C for 2h and 24 hours. The samples were filtered, and the filtrates were measured to determine the concentration and pH value, and the remaining solid was subjected to XRPD determination. The specific results were as follows:

**Table 26**

| | medium | pH | Solubility at 37 °C (mg/ml) | | pH(24h) | Crystal form |
|---|---|---|---|---|---|---|
| | | | 2h | 24h | | |
| | pH1.2 | 1.157 | >10 | >10 | 1.605 | N/A |
| | pH3.0 | 2.997 | >10 | >10 | 4.601 | N/A |
| | pH4.5 | 4.522 | >10 | >10 | 5.468 | N/A |
| | pH6.8 | 6.874 | >10 | >10 | 6.775 | N/A |
| Malate | pH7.4 | 7.471 | >10 | >10 | 7.389 | N/A |
| | FaSSIF | 6.532 | >10 | >10 | 6.524 | N/A |
| | FeSSIF | 5.022 | >10 | >10 | 5.796 | N/A |
| | SGF | 1.190 | >10 | >10 | 1.644 | N/A |
| | H₂O | 6.703 | >10 | >10 | 7.026 | N/A |
| | pH1.2 | 1.157 | >10 | >10 | 1.463 | N/A |
| | pH3.0 | 2.997 | >10 | >10 | 3.683 | N/A |
| | pH4.5 | 4.522 | >10 | >10 | 4.520 | N/A |
| | pH6.8 | 6.874 | 0.294 | 0.271 | 6.180 | Free base Form A |
| Free base | pH7.4 | 7.471 | 0.012 | 0.016 | 6.664 | Free base Form A |
| | FaSSIF | 6.532 | 1.234 | 0.983 | 5.434 | Free base Form A |
| | FeSSIF | 5.022 | >10 | >10 | 4.992 | N/A |
| | SGF | 1.190 | >10 | >10 | 1.494 | N/A |
| | H₂O | 6.703 | 0.0365 | 0.0046 | 4.607 | Free base Form A |

### Example 11: Preparation of free base crystal form Form B

**Suspension crystallization method:** about 25 mg of API was weighed and added into a glass bottle, and 1 mL of EtOH was added to prepare a suspension. The suspensions were placed under 25°C for suspending stirring for 3 days and/or 7 days respectively, and then filtered to obtain the free base crystal form Form B.

Approximately 25 mg of API was weighed and added into a glass bottle, and 1 mL of EtOH was added to prepare a suspension. The suspensions were placed under 50°C for suspending stirring for 3 days and/or 7 days respectively, and then filtered to obtain the free base crystal form Form B.

**Gas liquid diffusion:** A certain amount of API was weighed and added into a liquid phase vial, and a good solvent (NPA) was added to dissolve it, with a concentration of 50mg/mL. The vial was left open and placed in a 30 mL large flask containing anti-solvent (ACN). The flask was sealed with a lid, and left standing at room temperature for one week to obtain the free base crystal form Form B.

**Gas solid diffusion:** An appropriate amount of API solid was taken and added into a liquid phase vial, and the vial was left open and placed in a 30 mL large flask containing ethyl formate. The large flask was sealed and left standing at room temperature for 7 days to obtain the free base crystal form Form B.

**Slow volatilization:** Based on the solubility results, a certain amount of API was weighed and placed into a liquid phase vial, and THF: MIBK (1:1) was added for clear dissolution. The vial was then placed at room temperature for slowly evaporating the solvent (under N₂ protection) to obtain the free base crystal form Form B.

**Dissolution crystallization:** A certain amount of API was weighed and added into a 4 mL glass bottle, and a good solvent NPA (0.3 mL) was added to dissolve it, with a concentration of 66.7%. Then 0.9 mL of anti-solvent ACN (EA) was added quickly, and the mixture was stirred overnight under magnetic force, and then filtered to obtain the free base crystal form B.

A certain amount of API was weighed and added into a 4 mL glass bottle, and a good solvent THF (1 mL) was added to dissolve it, with a concentration of 20%. Then 3 mL of anti-solvent n-heptane (IPAC/MTBE) was added quickly, and the mixture was stirred overnight under magnetic force, and then filtered to obtain the free base crystal form B.

The crystal forms were subjected to various characterizations respectively. The XRPD data were shown in Table 27, and the TGA pattern showed that Form B has two stages of weight loss, with a loss of 4.478% in the first stage and a loss of 0.577% in the second stage; The DSC pattern showed two endothermic peaks, suggesting that Form B should be a hydrate. The initial melting temperatures were 99.38 ° C and 227.13 ° C, respectively, peak temperatures were 118.25 ° C and 228.55 ° C respectively, and the enthalpy values were 63.092 J/g and 76.592 J/g, respectively.

**Table 27**

| 2θ | Intensity |
|---|---|
| 19.31 | 100.00% |
| 23.059 | 41.90% |
| 25.888 | 32.70% |
| 12.805 | 29.00% |
| 17.188 | 18.00% |
| 14.464 | 11.40% |
| 20.6 | 6.70% |
| 18.446 | 5.30% |
| 6.337 | 5.10% |
| 29.3 | 5.00% |
| 16.155 | 4.40% |
| 8.937 | 4.00% |
| 9.29 | 3.90% |
| 23.545 | 2.20% |
| 32.361 | 1.70% |
| 23.932 | 1.50% |
| 10.227 | 1.40% |
| 39.324 | 1.30% |
| 15.042 | 1.00% |
| 26.791 | 1.00% |
| 30.37 | 0.90% |
| 21.132 | 0.80% |
| 24.448 | 0.70% |
| 27.831 | 0.60% |
| 36.599 | 0.60% |

### Example 12: Preparation of free base crystal form Form C

**Suspension crystallization method:** About 25 mg of API was weighed and added into a glass bottle, and 1 mL of THF was added to prepare a suspension. The suspensions were placed under 25°C for suspending stirring for 3 days and/or 7 days respectively, and then filtered to obtain the free base crystal form Form C.

About 25 mg of API was weighed and added into a glass bottle, and 1 mL of IPA was added to prepare a suspension. The suspensions were placed under 50°C for suspending stirring for 3 days and/or 7 days respectively, and then filtered to obtain the free base crystal form Form C.

**Gas solid diffusion:** An appropriate amount of API solid was weighed and placed into a liquid phase vial, and the vial was left open and placed in a 30 mL large flask containing THF. The large flask was sealed and left standing at room temperature for 7 days to obtain the free base crystal form Form C.

**Slow volatilization:** Based on the solubility results, a certain amount of API was weighed and added into a liquid phase vial, and THF was added for clear dissolution. The vial was then placed at room temperature for slowly evaporating the solvent (under N₂ protection) to obtain the free base crystal form Form C.

The crystal forms were subjected to various characterizations. The XRPD data were shown in Table 28, and the TGA pattern showed that Form C had a weight loss of 5.580% before 150 °C; the DSC pattern showed two endothermic peaks, suggesting that Form C should be a hydrate. The initial melting temperatures were 106.30 ° C and 226.16 ° C, respectively, peak temperatures were 120.04 ° C and 228.10 ° C respectively,, and the enthalpy values were 34.713 J/g and 79.591 J/g, respectively.

**Table 28**

| 2θ | Intensity |
|---|---|
| 20.503 | 100.00% |
| 18.54 | 47.00% |
| 17.404 | 30.10% |
| 16.871 | 29.20% |
| 16.214 | 20.70% |
| 11.736 | 18.70% |
| 15.654 | 18.40% |
| 22.017 | 16.10% |
| 19.347 | 15.90% |
| 22.166 | 14.50% |
| 8.954 | 14.30% |
| 9.835 | 13.70% |
| 19.874 | 13.60% |
| 18.853 | 12.70% |
| 23.06 | 11.20% |
| 25.8 | 10.00% |
| 18.129 | 9.40% |
| 14.094 | 8.40% |
| 28.368 | 6.20% |
| 14.724 | 5.90% |
| 8.601 | 5.80% |
| 15.353 | 5.70% |
| 12.754 | 5.60% |
| 24.977 | 5.00% |
| 11.035 | 4.60% |
| 24.57 | 4.50% |
| 5.254 | 3.80% |
| 8.395 | 3.60% |
| 14.996 | 3.40% |
| 12.273 | 2.50% |
| 27.18 | 2.40% |
| 40.473 | 1.90% |
| 23.782 | 1.80% |
| 32.539 | 1.60% |
| 41.542 | 1.60% |
| 31.819 | 1.30% |

### Example 13: Preparation of free base crystal form Form D

**Suspension crystallization method:** About 25 mg of API was weighed and added into a glass bottle, and 1 mL of ACN was added to prepare a suspension. The suspensions were placed under 25°C for suspending stirring for 3 days and/or 7 days respectively, and then filtered to obtain the free base crystal form Form D.

**Slow volatilization:** Based on the solubility results, a certain amount of API was weighed and placed into a liquid phase vial, and THF: MTBE (1:1) was added for clear dissolution. The vial was then placed at room temperature for slowly evaporating the solvent (under N₂ protection) to obtain the free base crystal form Form D.

**Dissolution crystallization:** A certain amount of API was weighed and added into a 4 mL glass bottle, and a good solvent DCM: IPA (1:1) (0.6mL) was added to dissolve it, with a concentration of 33.3%. Then 1.8mL of anti-solvent n-heptane was added quickly, and the mixture was stirred overnight under magnetic force, and then filtered to obtain the free base crystal form D.

The crystal forms were subjected to various characterizations. The XRPD data were shown in Table 29, and the TGA pattern showed that Form D had a weight loss of 5.817% before 150 °C; the DSC pattern showed three endothermic peaks and one exothermic peak, suggesting that Form D underwent transformation process during heating.

**Table 29**

| 2θ | Intensity |
|---|---|
| 20.722 | 100.00% |
| 14.963 | 96.30% |
| 6.586 | 73.90% |
| 12.967 | 54.60% |
| 17.286 | 32.70% |
| 10.025 | 28.90% |
| 25.554 | 20.50% |
| 19.364 | 17.90% |
| 8.401 | 16.90% |
| 11.974 | 16.90% |
| 19.86 | 16.20% |
| 8.191 | 16.00% |
| 13.312 | 15.20% |
| 14.52 | 13.70% |
| 25.035 | 13.00% |
| 24.033 | 8.60% |
| 26.213 | 7.10% |
| 16.529 | 6.50% |
| 23.323 | 6.30% |
| 26.933 | 6.00% |
| 15.982 | 5.30% |
| 18.898 | 4.70% |
| 17.711 | 4.30% |
| 7.882 | 3.50% |
| 21.949 | 3.50% |
| 32.042 | 3.20% |
| 22.526 | 2.90% |
| 33.893 | 2.40% |
| 27.981 | 2.10% |
| 32.821 | 2.10% |
| 41.062 | 2.10% |
| 11.008 | 2.00% |
| 27.218 | 1.80% |
| 33.206 | 1.50% |
| 39.812 | 1.20% |

### Example 14: Preparation of free base crystal form Form E

**Suspension crystallization method:** About 25 mg of API was weighed and added into a glass bottle, and 1 mL of anisole was added to prepare a suspension. The suspensions were placed under 25°C for suspending stirring for 3 days and/or 7 days, and then filtered to obtain the free base crystal form Form E.

About 25 mg of API was weighed and added into a glass bottle, and 1 mL of DMSO was added to prepare a suspension. The suspensions were placed under 50°C for suspending stirring for 3 days and/or 7 days, and then filtered to obtain the free base crystal form Form E.

The crystal forms were subjected to various characterizations. The XRPD data were shown in Table 30, and the TGA pattern showed that Form E had a weight loss of 18.075% before 150 °C; the DSC pattern showed three endothermic peaks, indicating the presence of solvent or water residues in Form E.

**Table 30**

| 2θ | Intensity |
|---|---|
| 8.559 | 100.00% |
| 13.753 | 46.30% |
| 20.533 | 40.40% |
| 12.913 | 28.00% |
| 6.776 | 19.80% |
| 18.511 | 13.50% |
| 13.521 | 12.60% |
| 15.128 | 12.50% |
| 11.724 | 9.60% |
| 4.23 | 9.20% |
| 24.687 | 9.20% |
| 21.235 | 8.50% |
| 16.27 | 8.10% |
| 17.677 | 7.10% |
| 23.693 | 6.80% |
| 20.014 | 6.20% |
| 15.483 | 6.00% |
| 16.887 | 5.80% |
| 7.482 | 5.60% |
| 22.342 | 5.30% |
| 24.031 | 5.30% |
| 9.838 | 4.50% |
| 18.137 | 4.20% |
| 22.826 | 3.20% |
| 19.427 | 2.90% |
| 35.733 | 2.80% |
| 41.301 | 2.70% |
| 39.689 | 2.40% |
| 30.603 | 2.10% |

### Example 15: Preparation of free base crystal form Form F

**Gas liquid diffusion:** A certain amount of API was weighed and added into a liquid phase vial, and a good solvent (DMF) was added to dissolve it, with a concentration of 10 mg/mL. The vial was left open and placed in a 30 mL large flask containing anti-solvent (water). The flask was sealed with a lid, and left standing at room temperature for one week to obtain the free base crystal form Form F.

**Gas solid diffusion:** An appropriate amount of API solid was taken and added into a liquid phase vial, and the vial was left open and placed in a 30 mL large flask containing ethanol. The flask was sealed and left standing at room temperature for 7 days to obtain the free base crystal form Form F.

The crystal forms were subjected to various characterizations. The XRPD data were shown in Table 31, and the TGA pattern showed that Form F had a weight loss of 4.796% before 220 °C; the DSC pattern showed one endothermic peak, suggesting that Form F should be a hydrate. The initial melting temperature is 228.76 ° C, the peak temperature is 229.65 ° C, and the enthalpy value is 55.214 J/g.

**Table 31**

| 2θ | Intensity |
|---|---|
| 19.324 | 100.00% |
| 25.949 | 42.00% |
| 12.82 | 25.50% |
| 8.952 | 3.20% |
| 6.36 | 3.00% |
| 39.352 | 2.80% |
| 23.083 | 2.30% |
| 17.224 | 2.10% |
| 16.746 | 2.00% |
| 20.632 | 1.90% |
| 9.31 | 1.30% |
| 18.407 | 1.10% |
| 17.04 | 0.60% |
| 14.49 | 0.50% |
| 16.186 | 0.50% |
| 32.354 | 0.30% |
| 33.337 | 0.30% |
| 35.404 | 0.30% |
| 10.229 | 0.20% |
| 18.753 | 0.20% |
| 30.373 | 0.20% |
| 36.679 | 0.20% |
| 8.413 | 0.10% |
| 15.142 | 0.10% |
| 17.446 | 0.10% |
| 17.87 | 0.10% |
| 18.054 | 0.10% |
| 23.348 | 0.10% |
| 23.658 | 0.10% |
| 24.109 | 0.10% |
| 25.216 | 0.10% |
| 26.831 | 0.10% |
| 28.946 | 0.10% |
| 29.351 | 0.10% |
| 32.555 | 0.10% |
| 33.903 | 0.10% |
| 38.715 | 0.10% |
| 38.9 | 0.10% |

### Example 16: Preparation of free base crystal form Form G

**Gas liquid diffusion:** A certain amount of API was weighed and added into a liquid phase vial, and a good solvent (NPA) was added to dissolve it, with a concentration of 50mg/mL. The vial was left open and placed in a 30 mL large flask containing anti-solvent (MeOH). The flask was sealed with a lid, and left standing at room temperature for one week to obtain the free base crystal form Form G.

**Slow volatilization:** Based on the solubility results, a certain amount of API was weighed and placed into a liquid phase vial, and MeOH was added for clear dissolution. The vial was then placed at room temperature for slowly evaporating the solvent (under N₂ protection) to obtain the free base crystal form Form G.

**Dissolution crystallization:** A certain amount of API was weighed and added into a 4 mL glass bottle, and a good solvent DCM: MeOH(1:1) (0.2mL) was added to dissolve it, with a concentration of 100%. Then 0.6mL of anti-solvent MTBE was added quickly, and the mixture was stirred overnight under magnetic force, and then filtered to obtain the free base crystal form G.

The crystal forms were subjected to various characterizations. The XRPD data were shown in Table 32, and the TGA pattern showed that Form G hsa two stages of weight loss, with a loss of 1.645% in the first stage and a loss of 3.225% in the second stage; the DSC pattern showed two endothermic peaks, suggesting that Form G is a hydrate. The initial melting temperatures are 103.40 ° C and 226.81 ° C, respectively, the peak temperatures are 124.31 ° C and 228.18 ° C, and the enthalpy values are 69.262 J/g and 80.651 J/g, respectively.

**Table 32**

| 2θ | Intensity |
|---|---|
| 8.228 | 100.00% |
| 19.529 | 76.40% |
| 16.987 | 60.80% |
| 5.253 | 59.10% |
| 24.365 | 52.90% |
| 21.414 | 51.00% |
| 22.713 | 48.00% |
| 15.917 | 42.40% |
| 8.418 | 25.60% |
| 18.689 | 21.20% |
| 19.376 | 19.70% |
| 23.137 | 18.70% |
| 23.553 | 18.20% |
| 9.86 | 17.50% |
| 15.116 | 16.10% |
| 20.615 | 15.60% |
| 22.229 | 15.10% |
| 15.462 | 12.70% |
| 11.505 | 9.90% |
| 28.21 | 8.10% |
| 12.367 | 7.80% |
| 25.112 | 7.80% |
| 24.068 | 7.50% |
| 13.687 | 7.20% |
| 25.59 | 7.20% |
| 10.954 | 6.10% |
| 29.506 | 5.60% |
| 27.964 | 3.50% |
| 13.981 | 3.40% |
| 26.905 | 3.30% |
| 33.657 | 2.90% |
| 34.776 | 2.70% |
| 26.667 | 2.60% |
| 14.35 | 2.10% |
| 36.659 | 2.00% |
| 17.671 | 1.90% |
| 30.615 | 1.90% |

### Example 17: Preparation of free base crystal form Form H

**Gas liquid diffusion:** A certain amount of API was weighed and added into a liquid phase vial, and a good solvent (NPA) was added to dissolve it, with a concentration of 50mg/mL. The vial was left open and placed in a 30 mL large flask containing anti-solvent (water). The flask was sealed with a lid, and left standing at room temperature for one week to obtain the free base crystal form Form H.

The crystal form was subjected to various characterizations. The XRPD data were shown in Table 33, and the TGA pattern showed that Form H has a weight loss of 6.987% before 200 °C; the DSC pattern showed two endothermic peaks, suggesting that Form H should be a hydrate.

**Table 33**

| 2θ | Intensity |
|---|---|
| 19.327 | 100.00% |
| 23.084 | 41.50% |
| 25.903 | 39.40% |
| 12.823 | 24.10% |
| 19.18 | 20.90% |
| 20.628 | 20.40% |
| 17.222 | 18.40% |
| 14.497 | 14.90% |
| 16.169 | 13.10% |
| 8.961 | 11.70% |
| 17.01 | 11.20% |
| 18.425 | 9.50% |
| 9.229 | 6.60% |
| 5.278 | 6.20% |
| 16.826 | 4.80% |
| 29.316 | 4.50% |
| 10.67 | 4.00% |
| 23.972 | 3.50% |
| 15.318 | 3.30% |
| 15.698 | 3.30% |
| 23.639 | 3.00% |
| 26.846 | 2.80% |
| 6.353 | 2.70% |
| 14.113 | 2.70% |
| 15.054 | 2.70% |
| 9.854 | 2.60% |
| 11.773 | 2.50% |
| 24.476 | 2.30% |
| 24.963 | 2.30% |
| 10.241 | 2.20% |
| 13.111 | 2.10% |
| 8.699 | 2.00% |
| 39.333 | 2.00% |
| 19.862 | 1.90% |
| 21.147 | 1.90% |
| 25.584 | 1.80% |
| 26.519 | 1.80% |
| 32.396 | 1.80% |
| 26.134 | 1.70% |
| 27.909 | 1.70% |
| 14.765 | 1.30% |
| 18.846 | 1.30% |
| 21.522 | 1.30% |
| 3.68 | 1.20% |
| 24.769 | 1.20% |
| 28.977 | 1.20% |
| 30.354 | 1.20% |
| 25.208 | 1.10% |
| 30.091 | 1.10% |
| 38.922 | 1.10% |
| 27.214 | 1.00% |
| 20.272 | 0.90% |
| 21.902 | 0.90% |
| 35.705 | 0.90% |
| 36.765 | 0.90% |
| 38.715 | 0.90% |
| 37.543 | 0.80% |
| 8.435 | 0.70% |
| 28.386 | 0.70% |
| 7.593 | 0.60% |
| 17.851 | 0.60% |
| 31.755 | 0.60% |
| 30.955 | 0.50% |
| 31.366 | 0.50% |
| 34.492 | 0.50% |
| 34.827 | 0.50% |
| 40.054 | 0.50% |
| 40.438 | 0.40% |

### Example 18: Preparation of free base crystal form Form I

**Gas solid diffusion:** An appropriate amount of API solid was taken and added into a liquid phase vial, and the vial was left open and placed in a 30 mL large flask containing DCM. The large flask was sealed and left standing at room temperature for 7 days to obtain the free base crystal form Form I.

The crystal form was subjected to various characterizations. The XRPD data were shown in Table 34, and the TGA pattern showed that Form I had a weight loss of 12.244% before 200 °C; the DSC pattern showed two endothermic peaks, suggesting that Form I should be a mixed crystal. The initial melting temperatures are 209.17 ° C and 220.81 ° C, respectively, the peak temperatures are 217.16 ° C and 224.35 ° C, and the enthalpy value are 14.499 J/g and 33.294 J/g, respectively.

**Table 34**

| 2θ | Intensity |
|---|---|
| 22.011 | 100.00% |
| 20.226 | 54.30% |
| 20.986 | 51.40% |
| 13.416 | 50.90% |
| 13.033 | 34.80% |
| 17.029 | 29.80% |
| 14.962 | 29.70% |
| 19.141 | 28.00% |
| 22.563 | 27.30% |
| 22.769 | 19.00% |
| 11.299 | 18.20% |
| 16.466 | 15.60% |
| 5.593 | 14.70% |
| 26.548 | 14.60% |
| 8.231 | 14.10% |
| 25.139 | 13.20% |
| 6.617 | 12.80% |
| 15.768 | 12.40% |
| 23.72 | 12.00% |
| 15.292 | 11.40% |
| 23.975 | 10.00% |
| 18.006 | 8.30% |
| 21.324 | 8.10% |
| 24.849 | 7.50% |
| 24.623 | 6.70% |
| 40.591 | 6.60% |
| 26.263 | 6.50% |
| 27.121 | 6.40% |
| 27.369 | 6.30% |
| 18.441 | 5.60% |
| 19.907 | 5.50% |
| 10.011 | 5.10% |
| 14.558 | 4.80% |
| 28.997 | 4.50% |
| 23.286 | 4.20% |
| 9.288 | 4.10% |
| 14.109 | 3.80% |
| 29.31 | 3.40% |
| 32.007 | 3.40% |
| 30.722 | 2.70% |

### Example 19: Preparation of free base crystal form Form J

**Gas solid diffusion:** An appropriate amount of API solid was taken and added into a liquid phase vial, and the vial was left open and placed in a 30 mL large flask containing 1,4-dioxane. The flask was sealed and left standing at room temperature for 7 days to obtain the free base crystal form Form J.

**Polymer induction:** A certain amount of API was weighed and added into 0.5 mL of ethanol, and about 10% (mass ratio) of high polymer (20.88 mg) was added. The mixrure was stirred at room temperature for 24 hours, and then filtered to obtain the free base crystal form Form J.

**Cooling crystallization:** An excess amount of 11.13 mg of API solid was added to 1 mL of 1,4-dioxane, and dissolved clearly at 50 ° C, and then filtered to remove the undissolved substance. The filtrate was slowly cooled from 50 ° C to 0 ° C at a rate of 0.1 ° C/min and kept at 0 ° C for 10 hours. If no solid separated out, the filtrate was continued to cooling to -10 ° C to obtain the free base crystal form Form J.

The crystal forms were subjected to various characterizations. The XRPD data were shown in Table 35, and the TGA pattern showed that Form J has two stages of weight loss, with a weight loss of 3.699% in the first stage, and a weight lose of 3.272% in the second stage; the DSC pattern showed three endothermic peaks and one exothermic peak, and Form J undergoes a crystallization process during heating.

**Table 35**

| 2θ | Intensity |
|---|---|
| 6.403 | 100.00% |
| 21.406 | 99.40% |
| 11.755 | 93.70% |
| 21.045 | 83.10% |
| 17.314 | 79.20% |
| 9.63 | 78.80% |
| 9.118 | 69.70% |
| 5.655 | 51.40% |
| 8.351 | 43.90% |
| 16.869 | 43.40% |
| 15.439 | 37.40% |
| 9.895 | 27.90% |
| 16.151 | 27.90% |
| 19.388 | 25.30% |
| 12.415 | 23.80% |
| 19.789 | 22.80% |
| 25.318 | 20.10% |
| 13.448 | 19.00% |
| 10.481 | 18.50% |
| 18.763 | 17.40% |
| 11.187 | 16.10% |
| 14.2 | 15.50% |

### Example 20: Preparation of free base crystal form Form K

**Suspension crystallization method:** About 25 mg of API was weighed and added into a glass bottle, and 1 mL of IPA:ACN (1:1) was added to prepare a suspension. The suspensions were placed under 50°C for suspending stirring for 7 days, and then filtered to obtain the free base crystal form Form K.

The crystal form was subjected to various characterizations. The XRPD data were shown in Table 36, and the TGA pattern showed that Form K has a weight loss of 4.482% before 150 °C; the DSC pattern showed two endothermic peaks, suggesting that Form K should be a hydrate. The initial melting temperatures are 102.50 ° C and 227.25 ° C, respectively, and the peak temperatures are 115.47 ° C and 228.34 ° C respectively, and the enthalpy values are 39.929 J/g and 80.766 J/g, respectively.

**Table 36**

| 2θ | Intensity |
|---|---|
| 19.314 | 100.00% |
| 23.064 | 82.70% |
| 5.637 | 69.20% |
| 11.396 | 60.90% |
| 17.197 | 48.80% |
| 20.625 | 44.10% |
| 22.089 | 43.50% |
| 14.482 | 36.40% |
| 25.895 | 33.90% |
| 18.418 | 30.20% |
| 12.808 | 29.00% |
| 9.272 | 28.30% |
| 8.948 | 26.80% |
| 16.154 | 24.70% |
| 21.14 | 18.90% |
| 20.21 | 16.90% |
| 16.539 | 15.60% |
| 22.61 | 15.10% |
| 18.109 | 11.70% |
| 29.288 | 11.70% |
| 7.931 | 10.40% |
| 23.92 | 8.90% |
| 19.98 | 8.70% |
| 15.512 | 8.20% |
| 8.35 | 7.50% |
| 26.836 | 7.50% |
| 15.867 | 7.20% |
| 23.606 | 7.20% |
| 26.668 | 6.00% |
| 24.888 | 5.10% |
| 32.296 | 4.80% |
| 29.096 | 4.60% |
| 10.215 | 4.30% |
| 11.757 | 4.30% |
| 24.467 | 4.20% |
| 15.029 | 3.80% |
| 39.238 | 3.00% |
| 28.325 | 2.40% |
| 27.845 | 2.20% |
| 40.494 | 1.80% |

### Example 21: Preparation of free base crystal form Form L

**Slow evaporation:** Based on the solubility results, a certain amount of API was weighed and placed into a liquid phase vial, and DCM was added for clear dissolution. The vial was then placed at room temperature for slowly evaporating the solvent (under N₂ protection) to obtain the free base crystal form Form L.

**Dissolution crystallization:** A certain amount of API was weighed and added into a 4 mL glass bottle, and a good solvent DCM:IPA (1:1) (0.6 mL) was added to dissolve it, with a concentration of 33.3%. Then 1.8 mL of anti-solvent CYH was added quickly, and the mixture was stirred overnight under magnetic force, and then filtered to obtain the free base crystal form Form L.

**Cooling crystallization:** An excess amount of 22.27 mg of API solid was added to 1 mL of THF, and dissolved clearly at 50 ° C, and then filtered to remove the undissolved substance. The filtrate was slowly cooled from 50 ° C to 0 ° C at a rate of 0.1 ° C/min and kept at 0 ° C for 10 hours. If no solids separated out, the filtrate was continued to cooling to -10 ° C to obtain the free base crystal form Form L.

An excess amount of 51.95 mg of API solid was added to 0.5 mL of DCM, and dissolved clearly at 50 ° C, and then filtered to remove the undissolved substance. The filtrate was slowly cooled from 50 ° C to 0 ° C at a rate of 0.1 ° C/min and kept at 0 ° C for 10 hours. If no solids separated out, the filtrate was continued to cooling to -10 ° C to obtain the free base crystal form Form L.

The crystal form were subjected to various characterizations. The XRPD data were shown in Table 37, and the DSC pattern showed two endothermic peaks, suggesting that Form L should be a hydrate. The initial melting temperatures are 109.88 ° C and 225.72 ° C, respectively, the peak temperatures are 123.96 ° C and 227.56 ° C respectively, and the enthalpy values are 63.617J/g and 69.833J/g, respectively.

**Table 37**

| 2θ | Intensity |
|---|---|
| 21.963 | 100.00% |
| 22.518 | 25.90% |
| 20.943 | 21.30% |
| 19.12 | 20.80% |
| 16.382 | 18.40% |
| 16.978 | 16.60% |
| 15.741 | 16.00% |
| 20.074 | 12.50% |
| 11.225 | 12.30% |
| 22.736 | 10.30% |
| 15.252 | 10.20% |
| 17.989 | 9.80% |
| 5.574 | 9.50% |
| 26.461 | 7.70% |
| 8.088 | 6.90% |
| 18.437 | 5.40% |
| 21.277 | 5.20% |
| 18.632 | 4.50% |
| 23.539 | 4.30% |
| 10.961 | 4.10% |
| 24.576 | 4.10% |
| 16.091 | 3.60% |
| 20.459 | 3.40% |
| 25.703 | 3.20% |
| 9.279 | 3.10% |
| 23.91 | 3.10% |
| 14.018 | 2.90% |
| 28.958 | 2.40% |
| 24.86 | 2.00% |
| 32.879 | 1.60% |
| 27.314 | 1.50% |
| 35.773 | 1.50% |
| 33.542 | 1.40% |
| 29.252 | 1.20% |
| 40.574 | 1.10% |

### Example 22: Preparation of free base crystal form Form M

**Dissolution crystallization:** A certain amount of API was weighed and added into a 4 mL glass bottle, and a good solvent NPA (0.3 mL) was added to dissolve it, with a concentration of 66.7%. Then 0.9 mL of anti-solvent water was added quickly, and the mixture was stirred overnight under magnetic force, and then filtered to obtain the free base crystal form M.

The crystal form was subjected to various characterizations. The XRPD data were shown in Table 38, and the TGA pattern showed that Form M has a weight loss of 10.717% before 150 °C, and it was speculated to be a solvate/hydrate.

**Table 38**

| 2θ | Intensity |
|---|---|
| 20.09 | 100.00% |
| 15.616 | 42.20% |
| 20.566 | 29.00% |
| 9.241 | 25.20% |
| 16.989 | 25.10% |
| 21.173 | 18.70% |
| 9.041 | 18.10% |
| 8.828 | 13.70% |
| 15.34 | 13.50% |
| 16.449 | 10.70% |
| 26.113 | 10.60% |
| 16.099 | 10.10% |
| 19.436 | 9.80% |
| 26.406 | 9.10% |
| 9.717 | 6.70% |
| 5.282 | 5.80% |
| 21.946 | 5.50% |
| 14.107 | 4.20% |
| 23.263 | 3.60% |
| 10.669 | 3.20% |
| 24.735 | 3.10% |
| 11.76 | 2.80% |
| 18.278 | 2.60% |

### Example 23: Preparation of free base crystal form Form N

**Dissolution crystallization:** A certain amount of API was weighed and added into a 4 mL glass bottle, and a good solvent NPA (0.3 mL) was added to dissolve it, with a concentration of 66.7%. Then 0.9 mL of anti-solvent DMSO was added quickly, and the mixture was stirred overnight under magnetic force, and then filtered to obtain the free base crystal form N.

The crystal form was subjected to various characterizations. The XRPD data were shown in Table 39, and the TGA pattern showed that Form N has two stages of weight loss, with a weight loss of 7.259% in the first stage and a weight loss of 2.932% in the second stage, and it was speculated to be a solvate/hydrate.

**Table 39**

| 2θ | Relative intensity |
|---|---|
| 20.569 | 100.00% |
| 16.952 | 89.10% |
| 19.507 | 69.80% |
| 16.069 | 40.70% |
| 7.91 | 40.30% |
| 19.353 | 36.80% |
| 15.677 | 31.80% |
| 18.461 | 23.90% |
| 23.017 | 23.20% |
| 18.837 | 22.80% |
| 23.23 | 22.30% |
| 9.851 | 18.00% |
| 10.726 | 17.80% |
| 24.261 | 17.40% |
| 21.615 | 17.20% |
| 25.889 | 16.80% |
| 26.902 | 14.20% |
| 18.255 | 13.30% |
| 23.903 | 12.50% |
| 15.371 | 11.90% |
| 22.713 | 11.80% |
| 19.864 | 11.70% |
| 25.661 | 11.60% |
| 14.112 | 10.80% |
| 14.798 | 9.20% |
| 12.769 | 9.10% |
| 29.013 | 9.10% |
| 12.46 | 8.70% |
| 24.799 | 8.60% |
| 26.464 | 8.30% |
| 11.751 | 8.00% |
| 13.143 | 8.00% |
| 5.108 | 7.60% |
| 30.667 | 6.10% |
| 34.665 | 5.70% |
| 33.332 | 4.80% |
| 28.357 | 4.40% |
| 8.608 | 4.20% |
| 39.721 | 3.70% |

### Example 24: Preparation of free base crystal form Form O

**Dissolution crystallization:** A certain amount of API was weighed and added into a 4 mL glass bottle, and a good solvent NPA (0.3 mL) was added to dissolve it, with a concentration of 66.7%. Then 0.9 mL of anti-solvent acetone was added quickly, and the mixture was stirred overnight under magnetic force, and then filtered to obtain the free base crystal form O.

The crystal form was subjected to various characterizations. The XRPD data were shown in Table 40, and the TGA pattern showed that Form O has a weight loss of 8.095% before 150 °C; The DSC pattern showed two endothermic peaks, suggesting that Form O should be a hydrate. The initial melting temperatures are 100.07 ° C and 226.17 ° C, respectively, the peak temperatures are 113.53 ° C and 227.77 ° C respectively, and the enthalpy values are 41.053J/g and 76.278J/g, respectively.

**Table 40**

| 2θ | Intensity |
|---|---|
| 21.72 | 100.00% |
| 18.877 | 82.40% |
| 17.769 | 64.10% |
| 16.511 | 45.10% |
| 20.049 | 37.50% |
| 9.294 | 34.50% |
| 22.47 | 34.40% |
| 11.139 | 27.90% |
| 22.635 | 26.40% |
| 15.11 | 23.30% |
| 19.334 | 23.00% |
| 20.676 | 22.20% |
| 16.21 | 19.20% |
| 26.024 | 17.70% |
| 18.318 | 17.50% |
| 8.19 | 13.00% |
| 13.891 | 12.60% |
| 22.05 | 12.60% |
| 23.086 | 12.50% |
| 5.499 | 11.10% |
| 15.632 | 10.90% |
| 16.053 | 9.50% |
| 24.463 | 7.40% |
| 8.501 | 6.70% |
| 12.827 | 6.10% |
| 28.113 | 5.60% |
| 28.729 | 5.10% |
| 17.216 | 4.90% |
| 28.978 | 4.90% |
| 20.921 | 4.60% |
| 10.828 | 4.40% |
| 14.485 | 3.80% |
| 30.779 | 3.40% |
| 27.64 | 3.30% |
| 29.609 | 3.10% |
| 23.81 | 2.80% |
| 32.367 | 2.30% |
| 23.654 | 2.20% |
| 33.6 | 2.00% |
| 31.634 | 1.90% |
| 25.085 | 1.80% |
| 37.002 | 1.60% |
| 11.83 | 1.40% |
| 36.126 | 1.40% |
| 40.712 | 1.30% |
| 41.521 | 1.00% |

### Example 25: Preparation of free base crystal form Form P

**Dissolution crystallization:** A certain amount of API was weighed and added into a 4 mL glass bottle, and a good solvent DCM:MeOH(1:1) (0.2 mL) was added to dissolve it, with a concentration of 66.7%. Then 0.6 mL of anti-solvent EA was added quickly, and the mixture was stirred overnight under magnetic force, and then filtered to obtain the free base crystal form P.

The crystal form was subjected to various characterizations. The XRPD data were shown in Table 41, and the TGA pattern showed that Form P has two stages of weight loss, with weight loss of 3.554% in the first stage, and weight loss of 0.751% in the second stage; the DSC pattern showed two endothermic peaks, suggesting that Form P should be a hydrate. The initial melting temperatures are 96.55 ° C and 225.42 ° C, respectively, the peak temperatures are 115.25 ° C and 227.56 ° C, respectively, and the enthalpy values are 49.897 J/g and 79.541 J/g, respectively.

**Table 41**

| 2θ | Intensity |
|---|---|
| 8.249 | 100.00% |
| 16.791 | 51.80% |
| 15.796 | 37.80% |
| 19.798 | 25.80% |
| 22.14 | 22.40% |
| 24.844 | 22.20% |
| 22.643 | 21.40% |
| 23.927 | 15.70% |
| 15.998 | 10.90% |
| 5.413 | 10.80% |
| 11.243 | 10.80% |
| 19.109 | 8.30% |
| 15.204 | 5.50% |
| 26.032 | 4.20% |
| 20.313 | 4.10% |
| 23.474 | 3.90% |
| 18.855 | 3.40% |
| 11.485 | 3.20% |
| 19.5 | 2.80% |
| 27.044 | 2.70% |
| 30.158 | 2.10% |
| 10.949 | 1.80% |
| 25.584 | 1.70% |
| 35.902 | 1.70% |
| 28.805 | 1.40% |
| 26.693 | 1.00% |
| 27.751 | 1.00% |
| 35.669 | 1.00% |
| 9.756 | 0.90% |
| 12.331 | 0.90% |
| 34.452 | 0.90% |
| 14.013 | 0.70% |
| 32.583 | 0.70% |
| 33.313 | 0.70% |
| 39.326 | 0.70% |
| 13.376 | 0.60% |
| 31.555 | 0.60% |
| 17.643 | 0.50% |

### Example 26: Preparation of free base crystal form Form Q

**Cooling crystallization:** An excess amount of 11.88 mg of API solid was added to 0.5 mL of ethanol, and dissolved clearly at 50 ° C, and then filtered to remove the undissolved substance. The filtrate was slowly cooled from 50 ° C to 0 ° C at a rate of 0.1 ° C/min and kept at 0 ° C for 10 hours. If no solid separated out, the filtrate continued to cooling to -10 ° C to obtain the free base crystal form Form Q.

The crystal form was subjected to various characterizations. The XRPD data were shown in Table 42.

**Table 42**

| 2θ | Intensity |
|---|---|
| 8.114 | 100.00% |
| 19.767 | 58.50% |
| 22.837 | 57.30% |
| 12.949 | 41.10% |
| 21.397 | 36.10% |
| 19.354 | 35.30% |
| 18.995 | 34.30% |
| 11.472 | 32.20% |
| 8.367 | 27.50% |
| 18.593 | 22.60% |
| 16.688 | 15.50% |
| 9.591 | 14.60% |
| 8.825 | 12.10% |
| 26.164 | 10.70% |
| 26.574 | 8.10% |
| 17.931 | 7.80% |
| 24.918 | 7.60% |
| 11.864 | 6.30% |
| 17.354 | 6.20% |
| 5.679 | 5.90% |
| 17.66 | 5.90% |
| 20.677 | 5.70% |
| 15.589 | 4.50% |
| 32.45 | 4.20% |
| 27.084 | 4.10% |
| 11.151 | 3.70% |
| 31.897 | 3.60% |
| 23.703 | 3.30% |
| 35.188 | 3.20% |
| 14.914 | 2.80% |
| 15.758 | 2.60% |
| 30.14 | 2.60% |
| 27.238 | 2.40% |
| 6.581 | 2.00% |
| 23.209 | 2.00% |
| 28.785 | 1.80% |
| 34.05 | 1.70% |
| 7.724 | 1.60% |
| 14.273 | 1.40% |
| 28.179 | 1.40% |
| 29.984 | 1.40% |
| 35.767 | 1.30% |
| 24.539 | 1.10% |
| 25.337 | 1.10% |
| 39.77 | 1.10% |
| 29.683 | 0.90% |
| 31.355 | 0.90% |
| 32.727 | 0.90% |
| 38.588 | 0.60% |

### Example 27: Preparation of free base crystal form Form R

The above prepared free base crystal forms B-Q were heated to 200 °C, and all crystal forms transformed into the same crystal form, which was named as the free base crystal form Form R, and its XRPD data spectrum was shown in Figure 41.

**Table 43**

| 2θ | Intensity |
|---|---|
| 22.248 | 100.00% |
| 12.339 | 88.40% |
| 19.597 | 82.10% |
| 22.748 | 79.80% |
| 17.034 | 75.30% |
| 6.579 | 65.10% |
| 17.62 | 58.10% |
| 13.831 | 37.40% |
| 25.901 | 32.10% |
| 24.013 | 23.10% |
| 9.447 | 16.00% |
| 28.991 | 15.20% |
| 8.241 | 11.30% |
| 13.556 | 10.50% |
| 23.343 | 8.40% |
| 20.822 | 8.30% |
| 13.345 | 7.80% |
| 16.664 | 7.40% |
| 20.571 | 7.00% |
| 19.406 | 6.90% |
| 24.358 | 6.90% |
| 24.919 | 5.30% |
| 8.897 | 4.40% |
| 39.905 | 3.90% |
| 15.257 | 3.60% |
| 28.601 | 3.60% |
| 20.063 | 3.40% |
| 33.028 | 3.20% |
| 30.525 | 2.60% |
| 21.408 | 2.50% |
| 25.383 | 2.40% |
| 36.219 | 2.30% |
| 40.32 | 2.20% |
| 30.27 | 2.10% |
| 33.741 | 2.00% |
| 32.489 | 1.70% |
| 31.21 | 1.60% |
| 26.826 | 1.50% |
| 34.552 | 1.30% |
| 12.777 | 1.20% |
| 18.963 | 0.90% |
| 31.607 | 0.80% |

### Example 28: Preparation of Free Base Crystal Form S

To a mixture of 10 mL of ethanol and 2 mL of water was added 1 g of API solid, the mixture was heated to 50°C and stirred while keeing the temperature. The solution was added with 961mg of trifluoroacetic acid and stirred to dissolve the API solid. After filtration, the filtrate was stirred at 45 °C. Sodium carbonate solution was added to the solution to make it slightly cloudy, and the dripping was stopped. The solution was stirred while keep the temperature at 45°C for 2 hours. The solution was continued to added with sodium carbonate solution dropwise and kept at 40 °C for 1 hour. The solution was slowly cooled to 5 °C, and stirred overnight while keeping the temperature, and then the solid was filtered and dried to obtain the free base crystal form S.

The crystal form was subjected to various characterizations. The XRPD data were shown in Table 44.

**Table 44**

| 2θ | Intensity |
|---|---|
| 5.388 | 100.00% |
| 18.299 | 90.30% |
| 10.892 | 56.00% |
| 16.589 | 30.30% |
| 20.774 | 21.50% |
| 18.103 | 20.10% |
| 15.674 | 19.40% |
| 7.917 | 18.60% |
| 17.512 | 13.80% |
| 21.377 | 13.30% |
| 19.485 | 11.40% |
| 16.025 | 9.60% |
| 11.055 | 9.30% |
| 18.622 | 8.70% |
| 20.009 | 8.60% |
| 23.269 | 7.70% |
| 17.015 | 6.70% |
| 12.162 | 6.60% |
| 15.002 | 6.20% |
| 8.735 | 5.90% |
| 27.664 | 5.80% |
| 8.239 | 5.40% |
| 19.758 | 5.40% |
| 24.248 | 4.90% |
| 36.703 | 4.20% |
| 25.547 | 3.30% |
| 12.903 | 2.70% |

### Example 29: Preparation of free base vrystal form Form T

90 g of API solid was added to 4.5L of water and 71.42g of trifluoroacetic acid was added at room temperature; after filtration, the filtrate was placed at 40 °C and stirred while keeping the temperature. The pH of the solution was adjusted to 7-8 using sodium carbonate solution to make the solution slightly cloudy, and the dripping was stopped. The solution was stirred while keeping the temperature at 40 °C for 2 hours. The solution was continued to added with sodium carbonate solution dropwise and kept at 40 °C for 1-2 hours. The solution was slowly cooled to 5 °C, and stirred for 1-2h while keeping the temperature, and then filtered and dried to obtain the free base crystal form T.

The crystal form was subjected to various characterizations, and the XRPD data were shown in Table 45.

**Table 45**

| 2θ | Intensity |
|---|---|
| 20.644 | 100.00% |
| 17.233 | 57.40% |
| 23.479 | 26.70% |
| 15.867 | 24.90% |
| 20.091 | 20.00% |
| 15.505 | 19.90% |
| 26.036 | 19.50% |
| 19.457 | 19.40% |
| 14.223 | 17.20% |
| 25.043 | 16.80% |
| 16.205 | 16.60% |
| 23.143 | 13.50% |
| 11.843 | 12.90% |
| 9.973 | 11.40% |
| 14.944 | 9.30% |
| 18.921 | 9.00% |
| 26.36 | 8.30% |
| 8.858 | 8.10% |
| 17.69 | 7.70% |
| 22.069 | 7.70% |
| 12.867 | 7.60% |
| 18.428 | 6.80% |
| 5.403 | 4.90% |
| 26.675 | 3.80% |
| 10.788 | 3.40% |
| 22.412 | 3.20% |
| 27.533 | 3.20% |
| 24.211 | 3.10% |
| 32.856 | 3.10% |
| 12.351 | 2.90% |
| 27.321 | 2.60% |
| 40.066 | 2.50% |
| 35.91 | 2.40% |
| 29.079 | 2.20% |
| 31.878 | 2.10% |
| 36.087 | 2.10% |
| 37.559 | 1.80% |
| 21.598 | 1.70% |
| 28.468 | 1.70% |
| 11.168 | 1.60% |
| 34.799 | 1.60% |
| 40.437 | 1.50% |
| 30.249 | 1.20% |
| 33.551 | 1.20% |
| 34.06 | 1.10% |
| 7.753 | 1.00% |
| 30.809 | 1.00% |
| 35.509 | 1.00% |
| 38.988 | 0.90% |
| 29.487 | 0.70% |
| 38.282 | 0.40% |

### Example 30: Scale-up of free base crystal form Form B

Approximately 250 mg of API was weighed and added into a 4 ml glass vial, and 2.5 ml of ethyl acetate was added. The mixture was stirred overnight under magnetic stirring at 50 °C, cooled to room temperature, and then filtered, and the obtained solid was dried in vacuum overnight in a 50 °C oven. The resulting solid was free base crystal form Form B. The free base crystal form Form B was subjected to various characterization. The results showed that the scaled-up free base crystal form Form B was consistent with **Example 11.** The TGA pattern showed that Form A has two stages of weight loss, with a weight loss of 4.837% in the first stage and a weight loss of 0.998% in the second stage; The DSC pattern showed two endothermic peaks, and the initial melting temperatures are 105.17 ° C and 223.07 ° C, respectively, and the peak temperatures are 122.82 ° C and 226.67 ° C, respectively. The DVS results showed that Form A has a moisture absorption weight gain of 1.05% at 80% RH humidity, and has slight hygroscopicity, which showed significantly improved compared to API which had a moisture absorption weight gain of 11.12% at 80% RH humidity. The crystal form of Form B did not change before and after DVS testing.

### Example 31: Competitive pulping

A saturated solution of API (free base Form A) in DCM and water was prepared. Approximately 10 mg of API (free base Form A) and free base crytal form Form B was weighed respectively and added into a 4 ml glass bottle, and 1 mL of the saturated solution was added. The mixture was stirred at 50 °C for 3 days and 7 days and filtered. The resulting solid was subjected to XRPD testing. From the results, it can be seen that the solid crystal form obtained by API and Form B being competitively pulped in DCM for 3 days tended to approach Form L, but it became oily after 5 days of beating, so this crystal form was not considered subsequsently. The crystal form obtained by competitive pulping in water for 3 days was a mixture of Form B and API.

**Table 46**

| number | Temper ature (°C) | API (mg ) | Form B (mg ) | solv ent | Volu me (mL) | 3 days | |
|---|---|---|---|---|---|---|---|
| | | | | | | Experimen tal phenomen a | XRPD results |
| 1 | 50°C | 9.35 | 9.24 | DC M | 0.5 | Red turbidity | Form K |
| 2 | | 9.79 | 10.21 | H₂O | 0.5 | Pale yellow turbidity | Form A+Form B |

### Example 32: Evaluation of solubility and stability of free base crystal form Form B 32.1 Solubility

Approximately 10 mg of Form B sample was weighed and added with 1 ml of buffer solution with different pH, and the mixture was stirred at 37 °C for 2 and 24 hours. The sample was filtered, and the filtrate was measured to determine the concentration and pH value, and the remaining solids were subjected to XRPD teating. The results showed that the solubility of Form B was greater than 10 mg/ml in pH 1.2, pH 3.0, pH 4.5, FeSSIF, and SGF buffer. In pH 6.8, pH 7.4, FaSSIF and water, the solubility was improved compared to API, and the crystal form remained unchanged in the buffers.

**Table 47 Solubility of Form B and API in Buffers with Different pH**

| Crystal form | medium | Original pH | Solubility at 37 °C (mg/ml) | | pH(24h ) | Crystal form |
|---|---|---|---|---|---|---|
| | | | 2h | 24h | | |
| Form B | pH1.2 | 1.196 | >10 | >10 | 1.925 | N/A |
| | pH3.0 | 2.998 | >10 | >10 | 4.892 | N/A |
| | pH4.5 | 4.496 | >10 | >10 | 6.062 | N/A |
| | pH6.8 | 6.831 | 0.3096 | 0.2504 | 6.813 | Form B |
| | pH7.4 | 7.408 | 0.0472 | 0.0399 | 7.311 | Form B |
| | FaSSIF | 6.525 | 1.190 | 1.4008 | 6.755 | Form B |
| | FeSSIF | 4.953 | >10 | >10 | 5.724 | N/A |
| | SGF | 1.194 | >10 | >10 | 1.910 | N/A |
| | H₂O | 5.649 | 0.0395 | 0.0179 | 7.197 | Form B |
| | pH1.2 | 1.157 | >10 | >10 | 1.463 | N/A |
| | pH3.0 | 2.997 | >10 | >10 | 3.683 | N/A |
| | pH4.5 | 4.522 | >10 | >10 | 4.520 | N/A |
| | pH6.8 | 6.874 | 0.294 | 0.271 | 6.180 | Form A |
| API (Form A) | pH7.4 | 7.471 | 0.012 | 0.016 | 6.664 | Form A |
| | FaSSIF | 6.532 | 1.234 | 0.983 | 5.434 | Form A |
| | FeSSIF | 5.022 | >10 | >10 | 4.992 | N/A |
| | SGF | 1.190 | >10 | >10 | 1.494 | N/A |
| | H₂O | 6.703 | 0.0365 | 0.0046 | 4.607 | Form A |

### 32.2 Stability

A certain amount of crystal form B was weighed and added into a liquid phase vial, and a total of 9 parallels were prepared. 9 parallels were placed separately in 80 °C; 25 °C, 60% RH, 40 °C, 75% RH; and light stability boxe. The chemical stability under conditions of: 80 °C for one day, light exposure for 10 days, as well as 25 °C, 60% RH, and 40 °C, 75% RH for one and two weeks were tested using liquid chromatography, and the XRPD of the solids were measured. The specific results were shown below. The results showed that Form B has good physical and chemical stability under various conditions.

**Table 48 Stability Results of API and Form B Solid**

| condition | | API | | | Form B | | |
|---|---|---|---|---|---|---|---|
| | | Purity (%) | Degradati on (%) | Cryst al form | Purity (%) | Degradati on (%) | Crystal form |
| initial | | 95.53 | N/A | N/A | 97.67 | N/A | N/A |
| 80°C | 1 day | 95.39 | 0.14 | chang ed | 97.50 | 0.17 | Uncha nged |
| Light exposure | 10 day s | 88.14 | 7.89 | chang ed | 97.63 | 0.04 | Uncha nged |
| Light control | 10 day s | 95.51 | 0.02 | Unch anged | 97.67 | 0.00 | Uncha nged |
| 25°C,60%R H | 1 wee k | 95.50 | 0.03 | Unch anged | 97.69 | 0.00 | Uncha nged |
| | 2 wee ks | 95.49 | 0.04 | Unch anged | 97.67 | 0.00 | Uncha nged |
| 40°C,75%R H | 1 wee k | 95.49 | 0.04 | Unch anged | 97.70 | 0.00 | Uncha nged |
| | 2 wee ks | 92.60 | 2.93 | Unch anged | 97.66 | 0.01 | Uncha nged |

### Example 33. Solubility of Crystal form Form T

Approximately 60 mg of the sample was weighed and added into a 4 mL bottle, and 2 mL of different solvents (water, pH 1.0, 3.0, 5.0, 7.0, 9.0, FaSSIF, FeSSIF, and SGF) were added and the mixtures were stirred at 37°C for 24h after adding magnetic stirring bar. Samples were taken at 2 and 24 h respectively. After filtration, the sample concentration in the filtrate was tested by HPLC, and the pH value of the filtrate and the XRPD of the remaining solid were also measured. The measured solubility of the sample in different solutions was as follows.

From the results, it can be seen that the solubility of the sample at 37 °C for 2 h and 24 h were similar, and the solubility of the sample gradually decreased with the increase of the pH of the solution. The solubility in pH 1.0, pH 3.0, FeSSIF, and SGF are high, and all exceed 18 mg/mL; the solubility is slightly high in pH 5.0, and is about 8 mg/mL; the solubility in water, pH 7.0, pH 9.0, and FaSSIF are relatively low, and are all less than 0.7 mg/mL. Compared to the initial pH value of the buffer solution, the pH values in pH 1.0, pH 3.0, pH 5.0, FeSSIF, and SGF showed significant changes. In other solvents, the pH of the solution did not change significantly, and the crystal form did not change.

**Table 49 Solubility test results of crystal form Form T in different solutions**

| **name** | **2 h** | | **24 h** | |
|---|---|---|---|---|
| | **Solubility (mg/mL)** | **pH** | **Solubility (mg/mL)** | **pH** |
| water | 0.002 | 7.20 | 0.003 | 7.12 |
| pH1.0 | 28.02 | 5.13 | 29.31 | 5.17 |
| pH3.0 | 23.13 | 6.51 | 23.21 | 6.52 |
| pH5.0 | 8.37 | 6.66 | 8.17 | 6.67 |
| pH7.0 | 0.06 | 6.99 | 0.06 | 6.98 |
| pH9.0 | 0.002 | 8.77 | 0.002 | 8.79 |
| FaSSIF | 0.67 | 6.72 | 0.70 | 6.73 |
| FeSSIF | 18.92 | 6.50 | 18.66 | 6.50 |
| SGF | 27.64 | 6.20 | 27.32 | 6.22 |

All documents referred to in the present invention are incorporated by reference herein as if each document is individually incorporated by reference. Further, it should be understood that upon reading the above teachings of the present invention, various modifications or alternations may be made to the present invention by those skilled in the art, and those equivalents also fall within the scope defined by the appended claims of the present application.

## Claims

1. A crystal form of a compound of formula I, 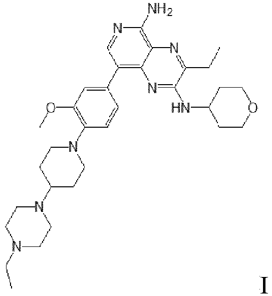 the crystal form is a free base crystal form Form T, and the free base crystal form Form T has the following X-ray powder diffraction characteristic peaks: 20.644 ° ± 0.2 °, 17.233 ° ± 0.2 °, 23.479 ° ± 0.2 °, 15.867 ° ± 0.2 °, and 20.091 ° ± 0.2 °.

2. The crystal form according to claim 1, wherein the crystal form further optionally has one or more (such as 2, 3, 5, 8, or 10) X-ray powder diffraction characteristic peaks selected from the group consisting of: 15.505°±0.2°, 26.036°±0.2°, 19.457°±0.2°, 14.223°±0.2°, 25.043°±0.2°, 16.205°±0.2°, 23.143°±0.2°, 11.843°±0.2°, 9.973°±0.2°, 14.944°±0.2°, 18.921°±0.2°, 26.36°±0.2°, 8.858°±0.2°, 17.69°±0.2°, 22.069°±0.2°, 12.867°±0.2°, 18.428°±0.2°, 5.403°±0.2°, 26.675°±0.2°, 10.788°±0.2°, 22.412°±0.2°, 27.533°±0.2°, 24.211°±0.2°, 32.856°±0.2°, 12.351°±0.2°, 27.321°±0.2°, 40.066°±0.2°, 35.91°±0.2°, 29.079°±0.2°, 31.878°±0.2°, 36.087°±0.2°, 37.559°±0.2°, 21.598°±0.2°, 28.468°±0.2°, 11.168°±0.2°, 34.799°±0.2°, 40.437°±0.2°, 30.249°±0.2°, 33.551°±0.2°, 34.06°±0.2°, 7.753°±0.2°, 30.809°±0.2°, 35.509°±0.2°, 38.988°±0.2°, 29.487°±0.2°, and 38.282°±0.2°.

3. The crystal form according to claim 1, wherein the DSC spectrum of the free base crystal form Form T has three endothermic peaks at 90-95 ° C, 115-120 ° C, and 220-225 ° C.

4. The crystal form according to claim 1, wherein the enthalpy value of the first endothermic peak in the DSC spectrum of the free base crystal form Form T is 50.223 J/g; the enthalpy value of the second endothermic peak in the DSC spectrum of the free base crystal form Form T is 51.492 J/g; and the enthalpy value of the third endothermic peak in the DSC spectrum of the crystal form is 80.538 J/g.

5. A crystal form of a compound of formula I, 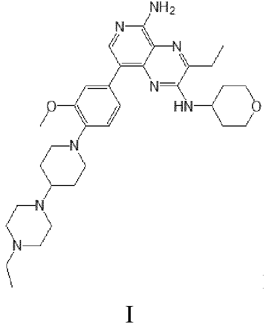 the crystal form is a free base crystal form Form B, and the free base crystal form Form B has the following X-ray powder diffraction characteristic peaks: 19.31 ° ± 0.2 °, 23.059 ° ± 0.2 °, 25.888 ° ± 0.2 °, 12.805 ° ± 0.2 °, and 17.188 ° ± 0.2 °.

6. The crystal form according to claim 5, wherein the crystal form is the free base crystal form Form B, and the free base crystal form Form B further optionally has one or more (such as 2, 3, 5, 8 or 10) X-ray powder diffraction characteristic peaks selected from the group consisting of: 14.464°±0.2°, 20.6°±0.2°, 18.446°±0.2°, 6.337°±0.2°, 29.3°±0.2°, 16.155°±0.2°, 8.937°±0.2°, 9.29°±0.2°, 23.545°±0.2°, 32.361°±0.2°, 23.932°±0.2°, 10.227°±0.2°, 39.324°±0.2°, 15.042°±0.2°, 26.791°±0.2°, 30.37°±0.2°, 21.132°±0.2°, 24.448°±0.2°, 27.831°±0.2°, and 36.599°±0.2°.

7. The crystal form according to claim 5, wherein the DSC spectrum of the crystal form has two endothermic peaks at 115-120 ° C and 225-230 ° C.

8. The crystal form according to claim 5, wherein the enthalpy value of the first endothermic peak in the DSC spectrum of the free base crystal form Form B is 63.092 J/g; and the enthalpy value of the second endothermic peak in the DSC spectrum of the crystal form is 76.592 J/g.

9. A crystal form of a compound of formula I, 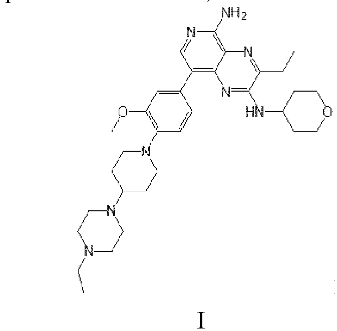 wherein the crystal form is selected from the group consisting of:
salt crystal forms I-A, I-B, and I-C formed by the compound of formula I and p-benzenesulfonic acid;
salt crystal forms II-A, II-B, and II-C formed by the compound of formula I and fumaric acid;
salt crystal forms III-A, III-B, III-C formed by the compound of formula I and methanesulfonic acid;
a salt crystal form IV-A formed by the compound of formula I and phosphoric acid;
salt crystal forms V-A and V-B formed by the compound of formula I and maleic acid;
a salt crystal form VI-A formed by the compound of formula I and citric acid;
a salt crystal form VII-A formed by the compound of formula I and malic acid;
salt crystal forms VIII-A, VIII-B, and VIII-C formed by the compound of formula I and salicylic acid;
salt crystal forms IX-A, IX-B, IX-C, IX-D, IX-E, and IX-F formed by the compound of formula I and hydrochloric acid;
alternatively, the crystal form is a free base crystal form selected from the group consisting of: Form A, Form C, Form D, Form E, Form F, Form G, Form H, Form I, Form J, Form K, Form L, Form M, Form N, Form O, Form P, Form Q, Form R, and Form S.

10. A method for preparing the crystal form according to claim 1, wherein the crystal form is Form T, and the method comprises steps of:
(i) dissolving free base Form A in a fifth solvent, adding with trifluoroacetic acid and filtering;
(ii) keeping the filtrate at 35-45 °C for 1-2h, and then adjusting the filtrate to pH 7-8;
(iii) keeping the filtrate at 35-45 °C for 1-3h, continuously adding dropwise with alkaline solution, and after dropwise addition, maintaining the temperature for 1-2 h;
(v) slowly cooling the filtrate down to 0-10 °C, maintaining the temperature and stirring for 1-3 h to obtain the free base crystal form Form T.

11. A method for preparing the crystal form according to claim 5, wherein the method comprises any one of steps (a), (b), (c), (d) and (e):
**(a) suspension crystallization method:** dissolving free base Form A in a first solvent to form a suspension, then leaving the suspension at 25 ° C-50 °C, optionally conducting suspension stirring, and then subjecting to filtration to obtain the free base crystal form Form B;
**(b) gas-liquid diffusion:** dissolving free base Form A in a liquid-phase vial containing afirst good solvent, and then keeping the liquid-phase vial open and placing it in a large flask containing a first anti-solvent, sealing the large flask, and leaving it at room temperature for one week to obtain the free base crystal form Form B;
**(c) gas solid diffusion:** placing free base Form A in a liquid-phase vial, and then keeping it open and and placing it in a large flask containing a third solvent, sealing the large flask, and leaving it at room temperature for one week to obtain the free base crystal form Form B;
**(d) slow volatilization:** placing free base Form A in a liquid-phase vial, adding a fourth solvent for clear dissolution, and then slowly evaporating the solvent at room temperature (under N₂ protection) to obtain the free base crystal form Form B;
**(e) dissolution crystallization:** placing free base Form A in a vial and dissolving it with a second good solvent; quickly adding a second anti-solvent, stirring overnight, and then subjecting to filtration to obtain the free base crystal form Form B.

12. The method according to claim 11, wherein,
when the method adopts step (a), the first solvent is selected from the group consisting of: EtOH, IPA, MTBE, EA, heptane, IPAC, ethyl formate, anisole, MIBK, a mixed solvent of IPA and ACN, a mixed solvent of DMSO and H₂O, and a mixed solvent of EtOH and H₂O;
when the temperature is 25 °C, the first solvent is selected from the group consisting of: EtOH, IPA, MTBE, EA, IPAC, ethyl formate, MIBK, a mixed solvent of IPA and ACN, and a mixed solvent of DMSO and H₂O;
when the temperature is 50 °C, the first solvent is selected from the group consisting of: EtOH, MTBE, EA, heptane, IPAC, ethyl formate, MIBK, anisole, a mixed solvent of DMSO and H₂O, and a mixed solvent of EtOH and H₂O;
when the method adopts step (b), the first good solvent is NPA, and the first anti-solvent is selected from the group consisting of: ACN, acetone, EA, and a combination thereof;
when the method adopts step (c), the third solvent is ethyl formate;
when the method adopts step (d), the fourth solvent is THF, MIBK, and a combination thereof;
when the method adopts step (e), the second good solvent is selected from the group consisting of: THF, NPA, and a combination thereof; And the second anti-solvent is selected from the group consisting of: ACN, EA, IPAC, MTBE, n-heptane, and a combination thereof.

13. A pharmaceutical composition, containing (a) the crystal form according to any one of claims 1-9 as active ingredient, and (b) pharmaceutically acceptable carriers.
